(19) Europäisches Patentamt / European Patent Office / Office européen des brevets

(11) **EP 4 516 797 A1**

(12) **EUROPEAN PATENT APPLICATION**
published in accordance with Art. 153(4) EPC


(43) Date of publication:
**05.03.2025 Bulletin 2025/10**

(21) Application number: **23796385.5**

(22) Date of filing: **25.04.2023**

(51) International Patent Classification (IPC):
***C07F 9/572*** (2006.01) ***A61K 9/127*** (2025.01)
***A61K 31/403*** (2006.01) ***A61K 41/00*** (2020.01)
***A61K 45/00*** (2006.01) ***A61K 47/14*** (2017.01)
***A61K 47/28*** (2006.01) ***A61K 47/44*** (2017.01)
***A61K 50/00*** (2006.01) ***A61P 35/00*** (2006.01)
***A61P 35/04*** (2006.01) ***A61P 43/00*** (2006.01)
***C07D 209/60*** (2006.01) ***C09B 23/08*** (2006.01)

(52) Cooperative Patent Classification (CPC):
**A61K 9/0009; A61K 9/127; A61K 31/403; A61K 41/00; A61K 45/00; A61K 47/14; A61K 47/28; A61K 47/44; A61P 35/00; A61P 35/04; A61P 43/00; C07D 209/60; C07F 9/572; C09B 23/08**

(86) International application number:
**PCT/JP2023/016320**

(87) International publication number:
**WO 2023/210645 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.04.2022 JP 2022073640**



(71) Applicants:
- **Eisai R&D Management Co., Ltd.**
  **Tokyo 112-8088 (JP)**
- **National University Corporation Hamamatsu University School of Medicine**
  **Hamamatsu-shi, Shizuoka 431-3192 (JP)**

(72) Inventors:
- **KIKUCHI Hiroshi**
  **Tsukuba-shi, Ibaraki 300-2635 (JP)**
- **HYODO Kenji**
  **Tsukuba-shi, Ibaraki 300-2635 (JP)**
- **OKU Naoto**
  **Shizuoka-shi, Shizuoka 422-8526 (JP)**
- **SHIMIZU Kosuke**
  **Hamamatsu-shi, Shizuoka 431-3192 (JP)**
- **NARITA Yudai**
  **Tsukuba-shi, Ibaraki 300-2635 (JP)**

(74) Representative: **Hoffmann Eitle**
**Patent- und Rechtsanwälte PartmbB**
**Arabellastraße 30**
**81925 München (DE)**


(54) **ICG LIPID DERIVATIVE, AND LIPID MICROPARTICLES EACH CONTAINING SAME**


(57) [Problem] To provide: an ICG lipid derivative that can form a lipid-based particle that has an excellent stability and/or can selectively release a drug; and a lipid-based particle containing this ICG lipid derivative.

[Solution] A lipid-based particle comprising a compound represented by formula (A) below or a pharmaceutically acceptable salt thereof,

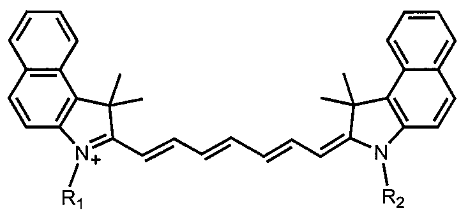

(A)

wherein, $R_1$ and $R_2$ each independently represent $-(CH_2)_k-CONH-R_3$;
$R_3$ represents a group selected from the group consisting


EP 4 516 797 A1

of $-(CH_2)_m-OPO_3^- - CH_2 - CH(CH_2OCOR_4)(OCOR_5)$, branched-chain $C_{14}$ to $C_{40}$ alkyl, and branched-chain $C_{14}$ to $C_{40}$ alkenyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4; and
m represents an integer from 2 to 4.

## Description

Technical Field

The present invention relates to ICG lipid derivatives and lipid-based particles comprising same, and to applications of the preceding.

Background Art

Indocyanine green (ICG), which is a near-infrared-emitting organic dye, is a compound having a structure below.

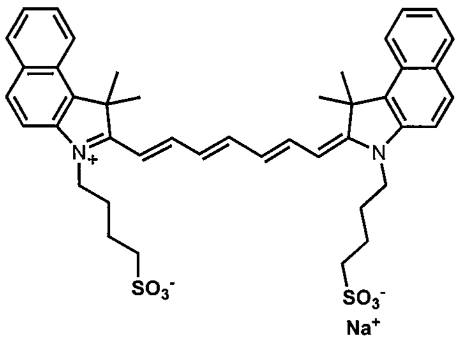

ICG exhibits fluorescence (near-infrared fluorescence capability) upon excitation by light in the near-infrared wavelength region. In addition, ICG exhibits absorption wavelengths in the near-infrared region and, upon irradiation with near-infrared light, exhibits a heat-generating action (heating effect, HT effect) and an active oxygen-generating action (photodynamic effect, PDT effect). ICG has been investigated for applications in various medical fields because ICG is a substance approved for administration into the body and because ICG exhibits the aforementioned actions in the near-infrared wavelength region, to which the living body is highly permeable. In specific terms, by using ICG-incorporating particles or molecular aggregates, research and development has been carried out into applications in molecular imaging technologies, molecular probe technologies, drug delivery systems (DDS), and so forth.

For example, ICG is employed in fluorescence imaging (FI) utilizing near-infrared fluorescence capability thereof. The ICG distribution within a subject can be imaged by administering ICG to the subject and measuring the fluorescence signal generated by ICG within the subject upon irradiation with light from the outside after a certain time; this is used, for example, to identify sentinel lymph nodes. For example, PTL 1 (Japanese Patent Application Laid-open No. 2001-299676) discloses a sentinel lymph node detection method that uses the near-infrared fluorescence capability of ICG. PTL 2 (WO 2011/152046) discloses a fluorescent probe comprising a liposome that contains an ICG lipid derivative yielded by modification of the basic ICG skeleton with an alkyl chain or phospholipid; this is used as a fluorescent probe that can produce a high fluorescence intensity for a long period of time. PTL 3 (Japanese Patent Application Laid-open No. 2010-266295) discloses a fluorescent tissue marker having vesicle clusters generated by the emulsifier-induced formation and aggregation of a plurality of capsules as provided by the encapsulation in hydrophilic solvent of vesicles formed by the combination of phospholipid and a near-infrared fluorescent dye, e.g., ICG.

Photoacoustic imaging (PAI) using ICG has also been studied. PAI is an imaging method that detects the ultrasonic waves (photoacoustic or PA) produced by the thermal expansion of a light absorber that has absorbed pulsed irradiated light. For example, photoacoustic imaging and fluorescence imaging are examined in NPL 1 (Akers WJ et al., ACS Nano. 2011 Jan 25;5(1):173-182, 2011), and using as a near-infrared fluorescent dye an ICG derivative provided by the modification of ICG, a perfluorocarbon (PFC) particle supporting this ICG derivative (ICG-loaded PFC particle) is used as a probe. In this literature, the ICG derivative is loaded on a portion of the particles entirely as a probe for imaging, and the use of the ICG derivative itself for carrying a drug is not considered. In addition, drugs, e.g., anticancer agents and antibiotics, are typically water soluble, but the ultrasonic contrast medium using ICG-loaded PFC particles in this literature is an O/W emulsion of particles in which perfluorocarbon is the core substance, and it is thus difficult from a practical standpoint to incorporate a water-soluble drug in this emulsion.

Photodynamic hyperthermal therapy (PHT) using the HT effect and PDT effect of ICG and photodynamic hyperthermal chemotherapy (PHCT), which combines local chemotherapy with PHT, are known. For example, PTL 4 (Japanese Patent Application Laid-open No. 2010-69001) discloses a photodynamic hyperthermal chemotherapy that uses, as a photosensitizing dye, ICG and utilizes heat-generating action and active oxygen-generating action thereof. In addition, PTL 5 (WO 2000/41726) discloses a transcutaneous photodynamic therapy that uses a photosensitizing agent, e.g., ICG, and discloses, as a photosensitizing agent delivery system, a liposome delivery system comprising a

photosensitizing agent.

The use of ICG-containing compounds in a DDS has also been pursued. PTL 6 (WO 2013/051732) discloses a drug-encapsulating liposome that contains a liposome membrane constituent material that is bonded to ICG; this can be used, for example, for a DDS.

Citation List

Patent Literature

PTL 1: Japanese Patent Application Laid-open No. 2001-299676
PTL 2: WO 2011/152046
PTL 3: Japanese Patent Application Laid-open No. 2010-266295
PTL 4: Japanese Patent Application Laid-open No. 2010-69001
PTL 5: WO 2000/41726
PTL 6: WO 2013/051732

Non Patent Literature

NPL 1: Akers WJ et al., ACS Nano. 2011 Jan 25;5(1):173-182, 2011

Summary of Invention

While a large number of ICG-containing particles have been proposed, ICG lipid derivatives and particles containing same that can be utilized in various medical and diagnostic applications are still required.

The present invention is, for example, as follows.

[1] A compound represented by formula (A) below or a pharmaceutically acceptable salt thereof:

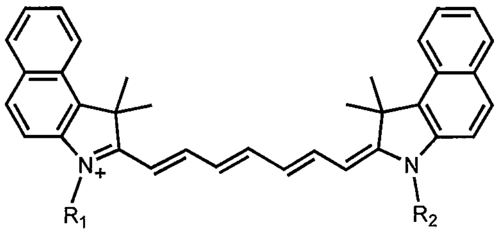

(A)

wherein

$R_1$ and $R_2$ each independently represent - $(CH_2)_k$ - CONH - $R_3$;
$R_3$ is selected from the group consisting of - $(CH_2)_m$ - $OPO_3^-$ - $CH_2$ - $CH(CH_2OCOR_4)(OCOR_5)$, branched-chain $C_{14}$ to $C_{40}$ alkyl, and branched-chain $C_{14}$ to $C_{40}$ alkenyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4; and
m represents an integer from 2 to 4.

[2] The compound according to [1], wherein

$R_1$ and $R_2$ each independently represent - $(CH_2)_2$ - CONH - $R_3$;
$R_3$ represents - $(CH_2)_m$ - $OPO_3^-$ - $CH_2$ - $CH(CH_2OCOR_4)(OCOR_5)$;
$R_4$ and $R_5$ each independently represent straight-chain $C_{13}$ to $C_{21}$ alkyl or straight-chain $C_{13}$ to $C_{21}$ alkenyl; and m represents an integer from 2 to 4.

[3] One compound selected from the group consisting of compounds represented by formula (I) or formula (II) below, or a pharmaceutically acceptable salt thereof:

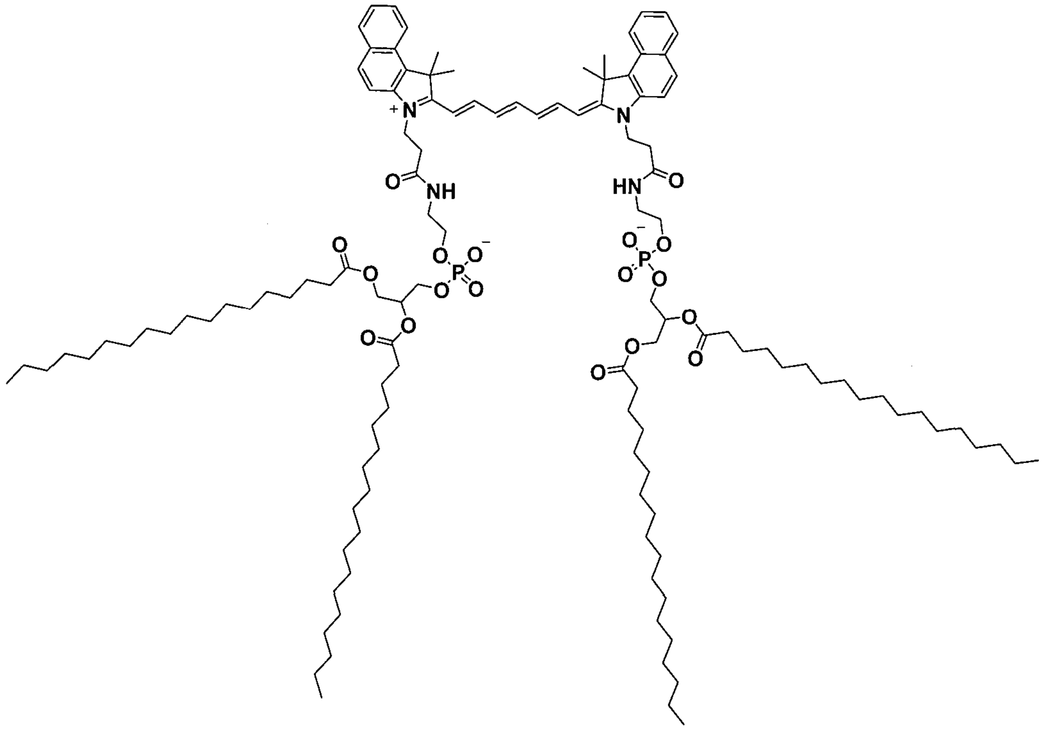

(I)

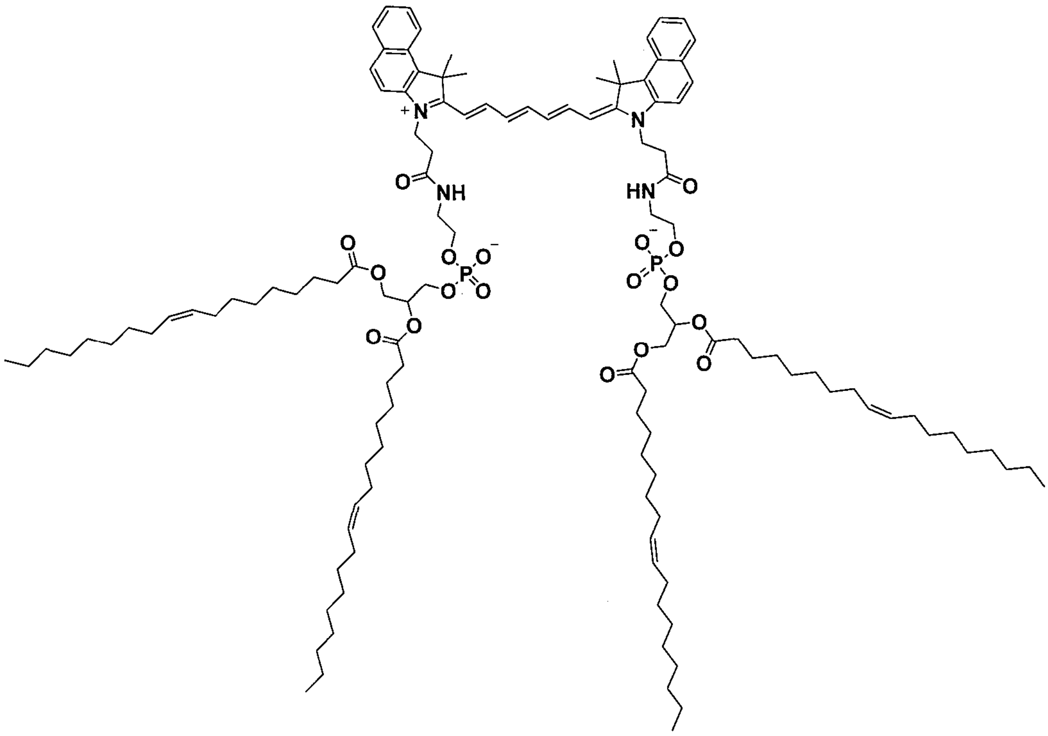

(II)

[3-1] The compound according to [1] wherein

$R_1$ and $R_2$ each independently represent - $(CH_2)_2$ - CONH - $R_3$ and
$R_3$ is selected from the group consisting of branched-chain $C_{14}$ to $C_{40}$ alkyl and branched-chain $C_{14}$ to $C_{40}$ alkenyl.

[3-2] The compound according to [3-1], selected from the group consisting of compounds represented by formula (IV) and formula (V) below, or a pharmaceutically acceptable salt thereof:

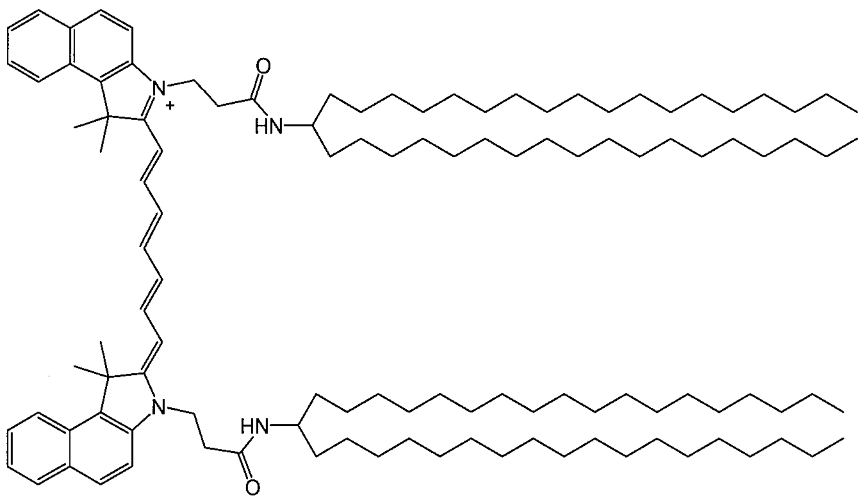

(IV)

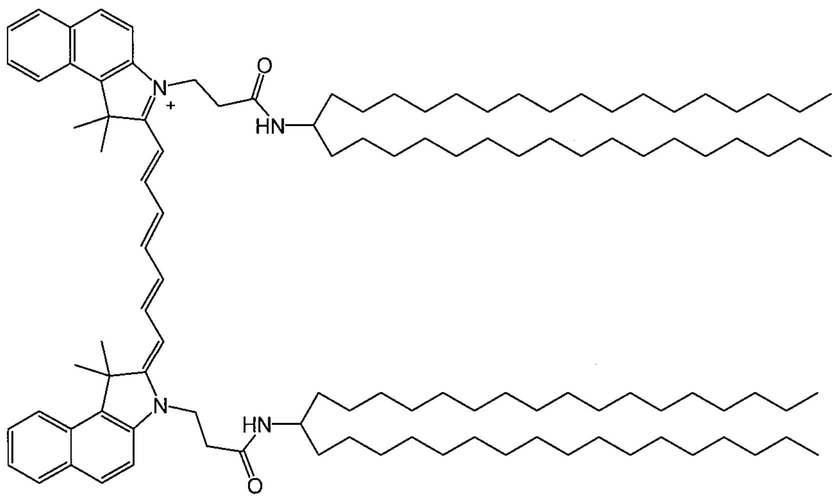

(V)

. ,

[4] A lipid-based particle comprising a compound represented by formula (A) below or a pharmaceutically acceptable salt thereof:

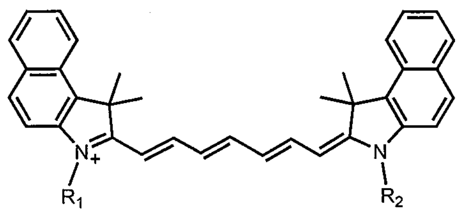

(A)

wherein

$R_1$ and $R_2$ each independently represent $-(CH_2)_k-CONH-R_3$;
$R_3$ is selected from the group consisting of $-(CH_2)_m-OPO_3^{-}-CH_2-CH(CH_2OCOR_4)(OCOR_5)$, straight-chain $C_{14}$ to $C_{22}$ alkyl, straight-chain $C_{14}$ to $C_{22}$ alkenyl, branched-chain $C_{14}$ to $C_{40}$ alkyl, and branched-chain $C_{14}$ to $C_{40}$ alkenyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4; and
m represents an integer from 2 to 4.

[4-1] The lipid-based particle according to [4], wherein

$R_1$ and $R_2$ each independently represent - $(CH_2)_2$ - CONH - $R_3$;
$R_3$ represents - $(CH_2)_m$ - $OPO_3^-$ - $CH_2$ - $CH(CH_2OCOR_4)(OCOR_5)$; and
$R_4$ and $R_5$ each independently represent straight-chain $C_{13}$ to $C_{21}$ alkyl or straight-chain $C_{13}$ to $C_{21}$ alkenyl; and m represents an integer from 2 to 4.

[4-2] The lipid-based particle according to [4], wherein

$R_1$ and $R_2$ each independently represent - $(CH_2)_2$ - CONH - $R_3$, and
$R_3$ is selected from the group consisting of straight-chain $C_{14}$ to $C_{22}$ alkyl, straight-chain $C_{14}$ to $C_{22}$ alkenyl, branched-chain $C_{14}$ to $C_{40}$ alkyl, and branched-chain $C_{14}$ to $C_{40}$ alkenyl.

[4-3] The lipid-based particle according to [4], wherein

$R_1$ and $R_2$ each independently represent - $(CH_2)_2$ - CONH - $R_3$, and
$R_3$ is selected from the group consisting of branched-chain $C_{14}$ to $C_{40}$ alkyl and branched-chain $C_{14}$ to $C_{40}$ alkenyl.

[4-4] The lipid-based particle according to [4], comprising a compound selected from the group consisting of compounds represented by formulas (I) to (V) below, or a pharmaceutically acceptable salt thereof:

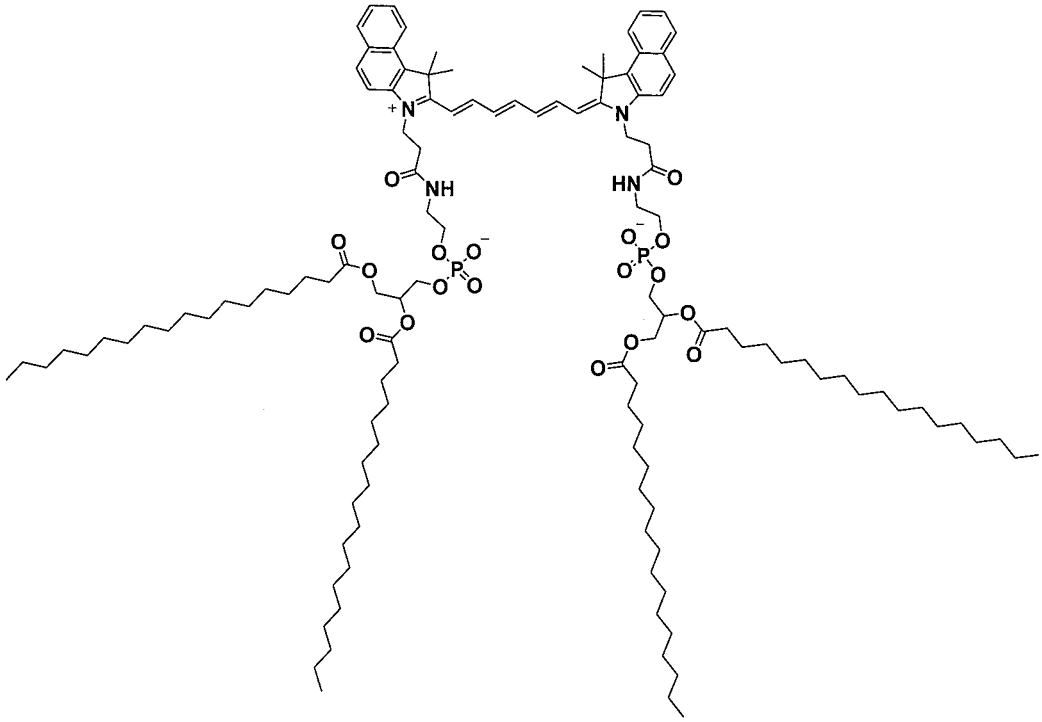

(I)

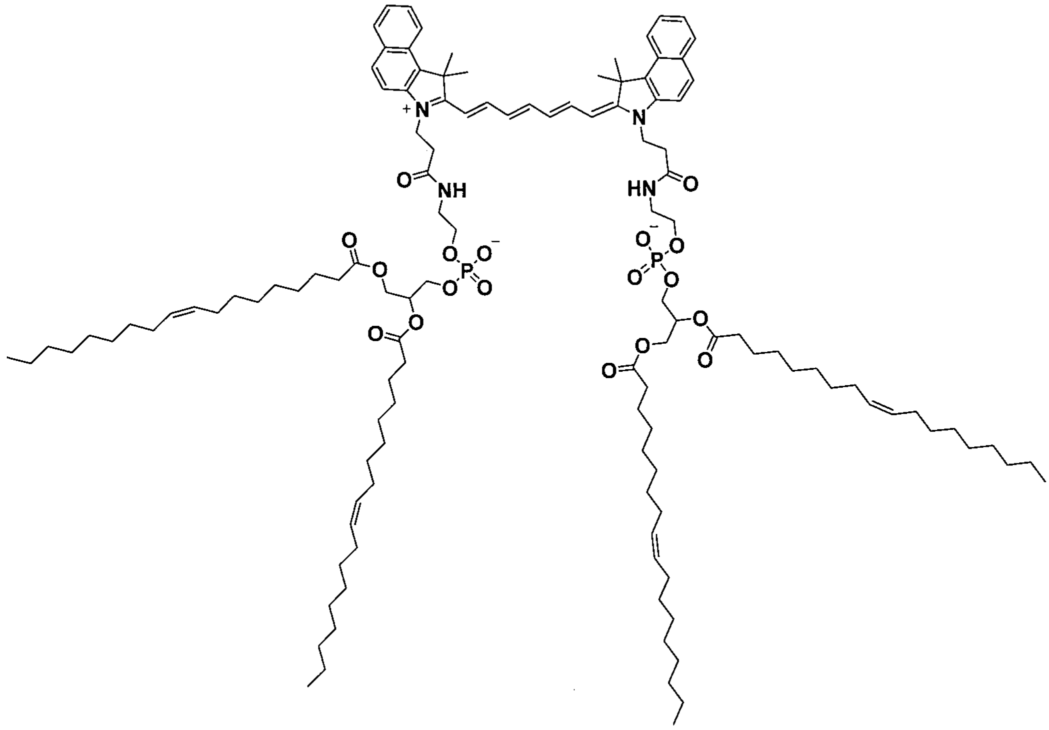

(II)

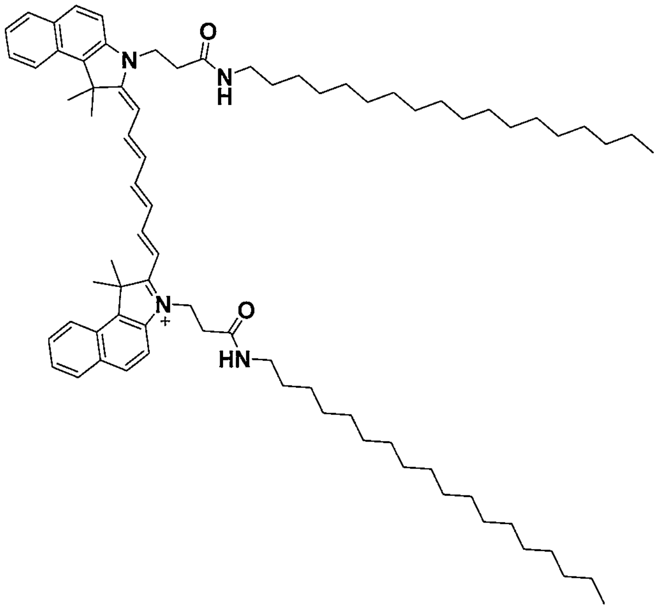

(III)

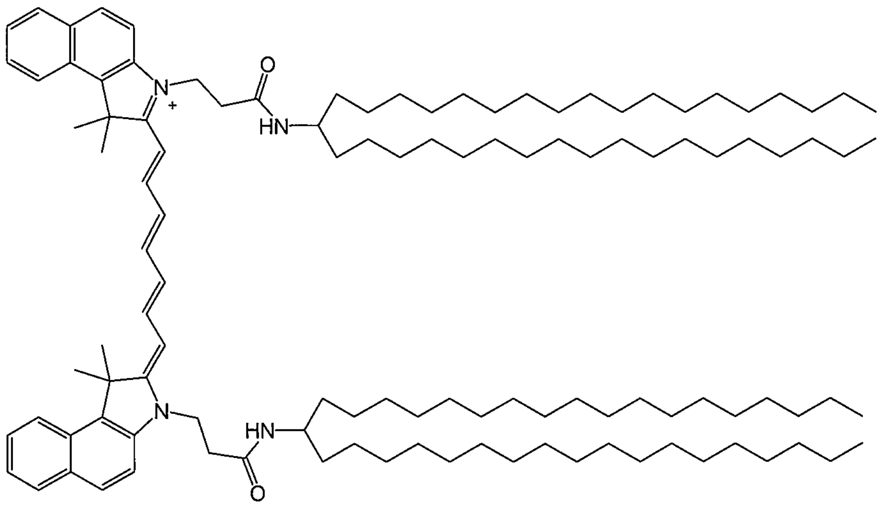

(IV)

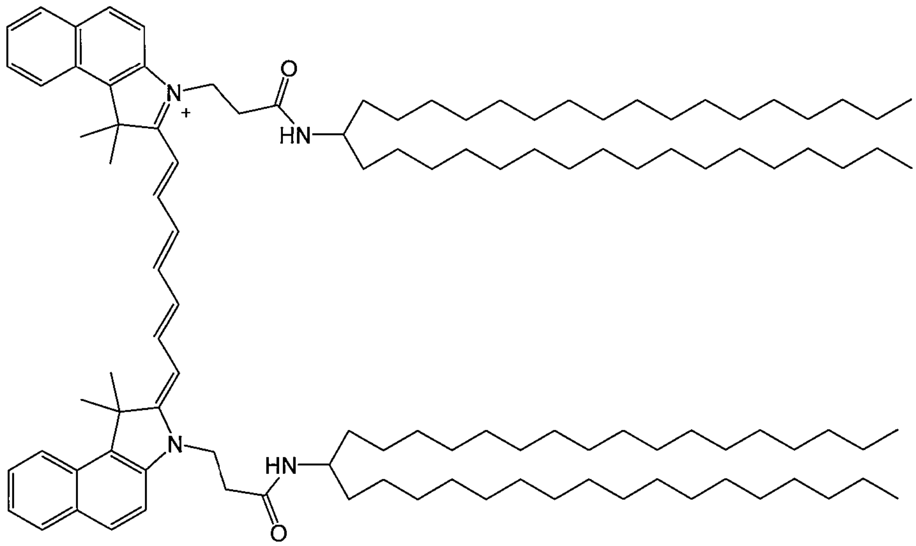

(V)

.

[5] The lipid-based particle according to [4], comprising a compound selected from the group consisting of compounds represented by formulas (I) to (III) below, or a pharmaceutically acceptable salt thereof:

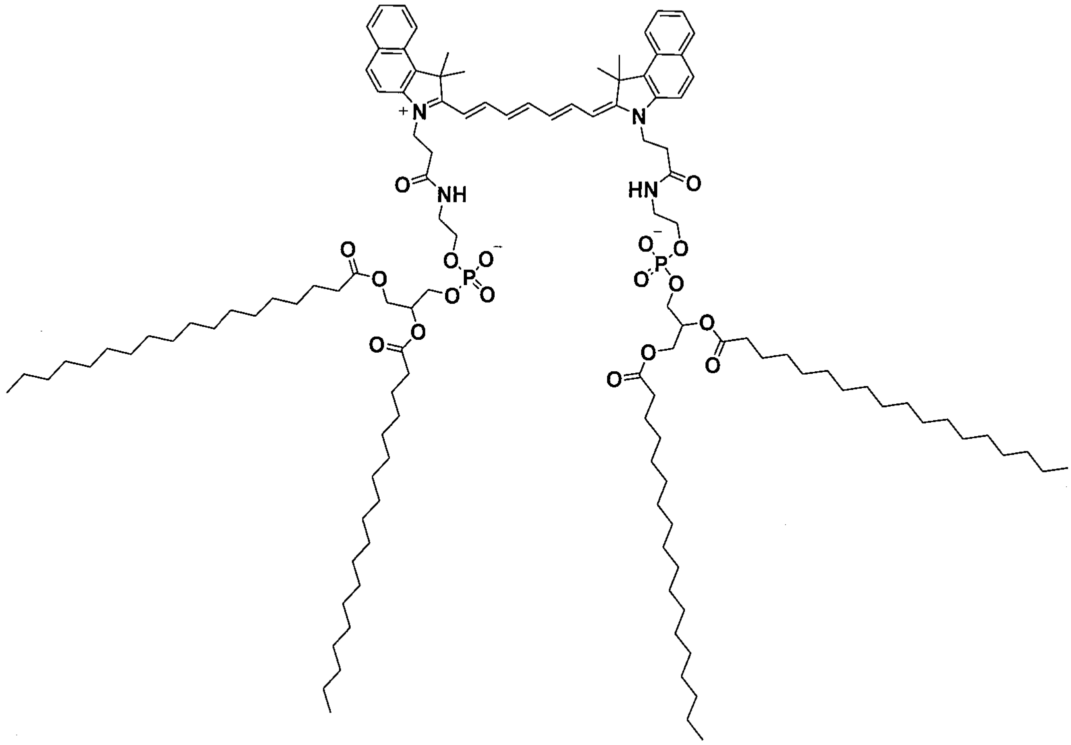

(I)

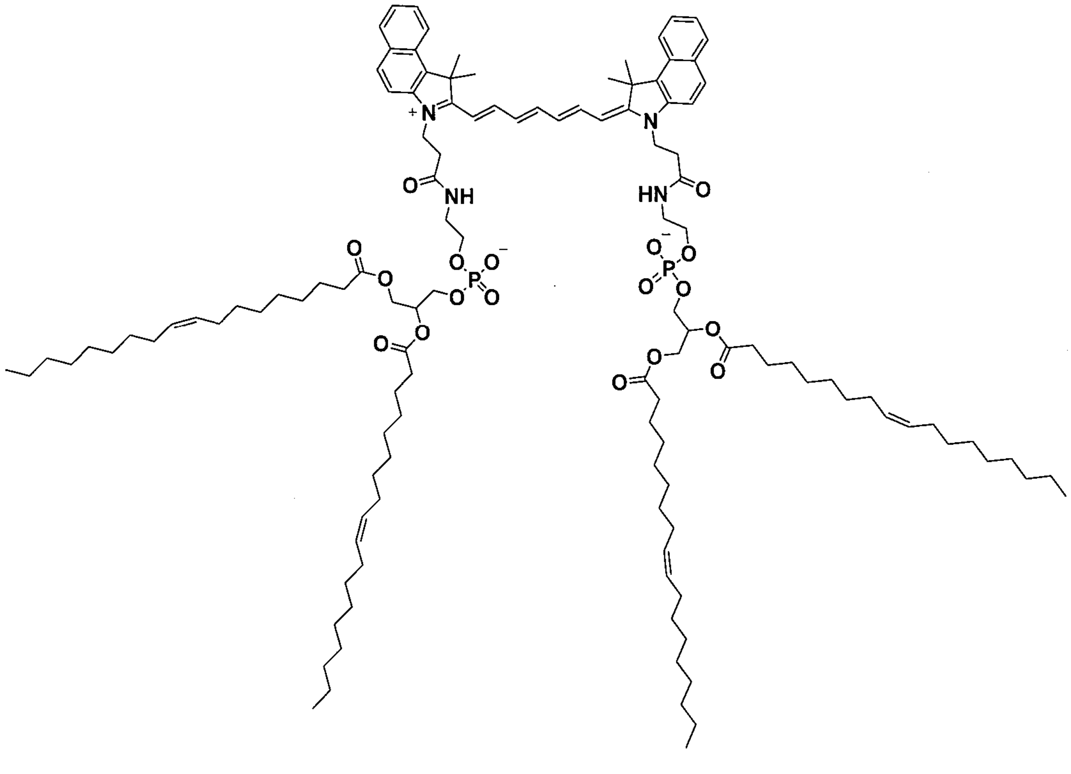

(II)

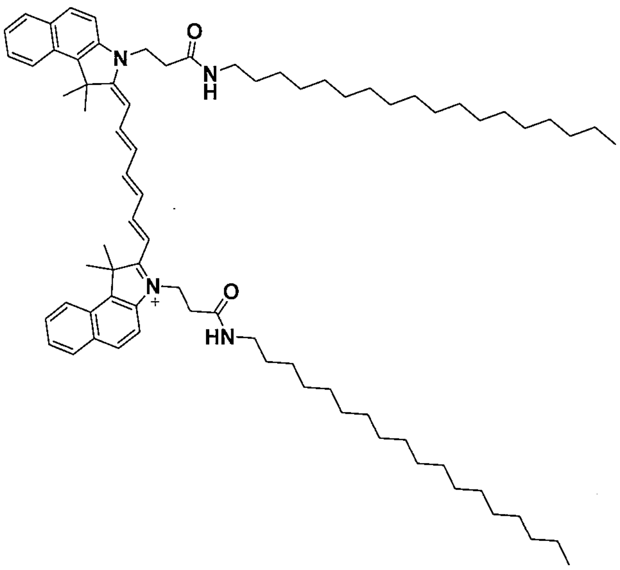

(III)

.

[6] The lipid-based particle according to any of [4], [4-1] to [4-4], and [5], which additionally fixes or supports at least one drug.
[7] The lipid-based particle according to any of [4] to [6] and [4-1] to [4-4], wherein the drug includes an anticancer agent.
[8] The lipid-based particle according to any of [4] to [7] and [4-1] to [4-4], wherein the lipid-based particle further includes at least one lipid selected from the group consisting of neutral lipids, polyethylene glycol-modified lipids, and sterols.
[9] The lipid-based particle according to any of [4] to [8] and [4-1] to [4-4], having an average particle diameter of 30 to 200 nm.
[10] The lipid-based particle according to any of [4] to [9] and [4-1] to [4-4], wherein the lipid-based particle is a polymer micelle or a liposome.
[10-1] The lipid-based particle according to [10], wherein the lipid-based particle is a liposome.
[11] A pharmaceutical composition or a diagnostic composition, comprising the compound according to any of [1] to [3], [3-1], and [3-2] or a pharmaceutically acceptable salt thereof, or the lipid-based particle according to any of [4] to [10], [4-1] to [4-4], and [10-1].
[11-1] The pharmaceutical composition according to [11], for the treatment of cancer.
[12] The pharmaceutical composition or diagnostic composition according to [11], used for at least one selection from photodynamic hyperthermal therapy, photodynamic therapy, and fluorescence imaging.
[13] A method for producing a drug-encapsulating liposome, the method comprising:

a step of producing a liposome from at least one lipid;
a step of replacing the external phase for the liposome to obtain a liposome dispersion containing a liposome having an ammonium sulfate ion gradient and/or a pH gradient between the internal phase and the external phase; and
a step of mixing a drug with the liposome dispersion to encapsulate the drug in the liposome of the liposome dispersion,

wherein the at least one lipid comprises a compound represented by formula (A) below or a pharmaceutically acceptable salt thereof:

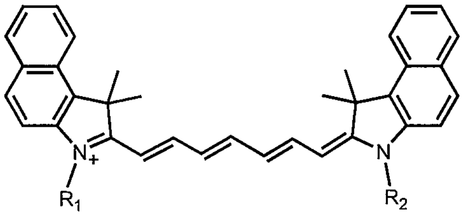

(A)

wherein

$R_1$ and $R_2$ each independently represent - $(CH_2)_k$ - CONH - $R_3$;
$R_3$ is selected from - $(CH_2)_m$ - $OPO_3^-$ - $CH_2$ - $CH(CH_2OCOR_4)(OCOR_5)$, straight-chain $C_{14}$ to $C_{22}$ alkyl, straight-chain $C_{14}$ to $C_{22}$ alkenyl, branched-chain $C_{14}$ to $C_{40}$ alkyl, and branched-chain $C_{14}$ to $C_{40}$ alkenyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4; and
m represents an integer from 2 to 4.

[14] Use of a lipid composite according to any of [4] to [10], [4-1] to [4-4], and [10-1] in the production of a pharmaceutical composition or a diagnostic composition.
[15] A method for treating cancer, the method comprising administering the pharmaceutical composition according to [11] to a subject.
[16] Photodynamic hyperthermal therapy, photodynamic therapy, or fluorescence imaging, comprising administering the pharmaceutical composition or diagnostic composition according to [11] to a subject.
[17] Use of the lipid composite according to [4] to [10], [4-1] to [4-4], and [10-1] or the pharmaceutical composition according to [11] for use in the treatment of cancer.
[18] Use of the lipid composite according to [4] to [10], [4-1] to [4-4], and [10-1] or the pharmaceutical composition according to [11] for use in at least one selection from photodynamic hyperthermal therapy, photodynamic therapy, and fluorescence imaging.

The ICG lipid derivative according to the present invention has one or more of the following effects.

(1) The ICG lipid derivative according to the present invention can form a lipid-based particle (in particular a liposome) that exhibits excellent stability (in particular stability in the blood).
(2) The ICG lipid derivative according to the present invention can form a drug-encapsulating lipid-based particle (in particular a liposome) that exhibits excellent stability (in particular stability in the blood). In some aspects, the drug-encapsulating lipid-based particle can be used as a drug carrier in a DDS.
(3) A lipid-based particle (in particular a liposome) constructed with the ICG lipid derivative according to the present invention exhibits an excellent drug encapsulation efficiency.
(4) Drug encapsulation is easily performed with a lipid-based particle (in particular a liposome) constructed with the ICG lipid derivative according to the present invention. In some aspects, a drug-encapsulating liposome having an excellent stability can be obtained even in the case of use of a method in which the drug is loaded into the liposome from outside the liposome after the empty liposome has been produced from the ICG lipid derivative.
(5) With a lipid-based particle (in particular a liposome) constructed with the ICG lipid derivative according to the present invention, the encapsulated drug can be selectively released to the exterior of the particle by irradiation with near-infrared light.
(6) With a lipid-based particle (in particular a liposome) containing with the ICG lipid derivative according to the present invention, heating and/or active oxygen can be efficiently produced by irradiation with near-infrared light and an increase in the temperature of the corresponding dispersion medium can be brought about. A lipid-based particle containing the ICG lipid derivative according to the present invention can thus be used in photodynamic hyperthermal therapy and/or photodynamic therapy. In some aspects, a lipid-based particle containing the ICG lipid derivative according to the present invention can function as a temperature-sensitive liposome.
(7) A lipid-based particle (in particular a liposome) containing the ICG lipid derivative according to the present invention produces fluorescence upon irradiation with excitation light. In some aspects, a lipid-based particle containing the ICG lipid derivative according to the present invention is thus used for fluorescence imaging. For example, a lipid-based particle containing the ICG lipid derivative according to the present invention can function as a probe for fluorescence imaging.
(8) A pharmaceutical composition or a diagnostic composition containing the ICG lipid derivative or lipid-based particle according to the present invention is provided in some embodiments.

Brief Description of Drawings

[Fig. 1] Fig. 1 provides a $^1$H NMR chart of the ICG-diSA produced in Example A1.
[Fig. 2] Fig. 2 provides a $^1$H NMR chart of the ICG-diDOPE produced in Example A2.
[Fig. 3] Fig. 3 provides a $^1$H NMR chart of the ICG-diDSPE produced in Example A3.
[Fig. 4] Fig. 4 provides a $^1$H NMR chart of the ICG-DOPE produced in Comparative Example A1.

[Fig. 5] Fig. 5 is a diagram that shows the absorption spectra of ICG and the ICG lipid derivatives produced in the examples and a comparative example.

[Fig. 6] Fig. 6 shows the release behavior of doxorubicin (DOX) from liposomes due to exposure to LED light. The diagram on the left gives the doxorubicin release ratio (%) due to exposure to LED light (near-infrared light) from the ICGLip73-DOX of Example B7 and the Lip73-DOX of Comparative Example B3; the diagram on the right gives the doxorubicin release ratio (%) due to exposure to LED light (near-infrared light) from the ICGLip82-DOX of Example B8 and the Lip82-DOX of Comparative Example B4.

[Fig. 7A] Fig. 7A shows the release behavior of cisplatin from liposomes due to exposure to LED light. The diagram on the left gives the cisplatin release ratio (%) due to exposure to LED light (near-infrared light) from the ICGLip73-Cis of Example C1 and the Lip73-Cis of Comparative Example C1; the diagram on the right gives the cisplatin release ratio (%) due to exposure to LED light (near-infrared light) from the ICGLip82-Cis of Example C2 and the Lip82-Cis of Comparative Example C2.

[Fig. 7B] Fig. 7B is a diagram that shows the timewise change in temperature, under LED exposure (near-infrared light), of the liposome dispersions of Examples C1 and C2 and Comparative Examples C1 and C2 (ICGLip73-Cis, Lip73-Cis, ICGLip82-Cis, Lip82-Cis).

[Fig. 8] Fig. 8 shows the temperature, measured using a thermography camera (E5, Flir Systems) before and after exposure to LED light, of the solid tumor site in mice that had received physiological saline (Saline) or a liposome formulation (ICGLip, Lip-DOX, ICGLip-DOX).

[Fig. 9] Fig. 9 shows the cancer therapeutic effect (change in cancer volume of the solid tumor (relative ratio from the day of drug administration)) due to administration of physiological saline (Saline) or a liposome formulation (ICGLip, Lip-DOX, ICGLip-DOX) and/or exposure to LED light (+ LED).

[Fig. 10] Fig. 10 shows the body weight change in mice due to administration of physiological saline (Saline) or a liposome formulation (ICGLip, Lip-DOX, ICGLip-DOX) and/or exposure to LED light (+ LED).

[Fig. 11] Fig. 11 shows the temperature, measured using a thermography camera (E5, Flir Systems) before and after exposure to LED light, of the solid tumor site in mice that had received physiological saline (Saline) or a liposome formulation (ICGLip, Lip-Cis, ICGLip-Cis).

[Fig. 12] Fig. 12 shows the cancer therapeutic effect (change in cancer volume of the solid tumor (relative ratio from the day of drug administration)) due to administration of physiological saline (Saline) or a liposome formulation (ICGLip, Lip-Cis, ICGLip-Cis) and/or exposure to LED light (+ LED).

[Fig. 13] Fig. 13 shows the body weight change in mice due to administration of physiological saline (Saline) or a liposome formulation (ICGLip, Lip-Cis, ICGLip-Cis) and/or exposure to LED light (+ LED).

Description of Embodiments

The present invention is described in detail in the following by providing embodiments, examples, and so forth; however, the present invention is not limited to the embodiments, examples, and so forth provided herebelow and can be freely modified and executed in a range that does not depart from the essential features of the present invention. All of the documents and publications described in this Description are incorporated in this Description in their entirety by reference regardless of their purpose.

The meanings of the terms and so forth used in this Description are described in the following, and a detailed description of the present invention is provided herebelow.

A "particle", referred to as, inter alia, a nanosphere, microsphere, nanocapsule, and microcapsule, is a particle having an average particle diameter from nanosizes to microsizes (1 nm to 1 μm or um). Particles may be the phase dispersed in an emulsion or the internal phase in a suspension.

A "lipid-based particle" denotes a particle that contains a plurality of lipids physically bonded to each other by intermolecular forces. A "lipid-based particle" typically is a particle comprising a membrane-like molecular assembly formed by the association of lipids.

A "particle carrier" or "lipid-based particle carrier" denotes a particle or lipid-based particle that can fix or support a chemical substance, e.g., a drug. In an embodiment, the "particle carrier" or "lipid-based particle carrier" indicates a structure that is biocompatible and that is sufficiently resistant to chemical and/or physical degradation for a sufficient interval of time to enable a desired target to be reached.

"Fixed" denotes an immovable disposition on the outer surface and/or in the interior.

"Support" denotes a movable disposition of, e.g., a drug, on the outer surface and/or in the interior of a particle.

A "liposome" denotes a closed vesicle formed by a bilayer molecular membrane. A liposome is typically constructed of a single lipid bilayer or a plurality of lipid bilayers.

A "polymer micelle" denotes a closed vesicle formed by a single-layer molecular membrane.

"Alkyl" denotes a straight-chain, cyclic, or branched saturated aliphatic hydrocarbon group having a designated number of carbon atoms.

"Alkenyl" designates a straight-chain or branched hydrocarbon group that has a predetermined number of carbon atoms and at least one carbon-carbon double bond. Examples here are monoenes, dienes, trienes, and tetraenes, but there is no limitation to these.

1. The ICG lipid derivative

An embodiment of the present invention relates to a compound represented by formula (A) below or a pharmaceutically acceptable salt thereof.

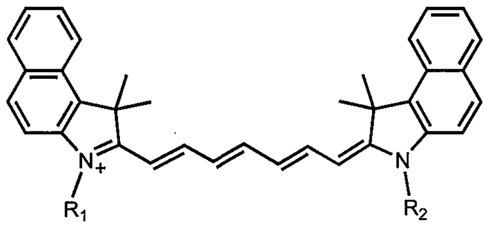

(A)

The compound given by formula (A) has a structure in which the ICG skeleton is modified by hydrocarbon chain-bearing $R_1$ and $R_2$, and can function as a lipid. Because it has the basic skeleton of ICG, the compound given by formula (A) can exhibit a near-infrared fluorescence capability, and, upon irradiation with near-infrared light, can exhibit a heat-generating action (HT effect) and an active oxygen-generating action (PDT effect). The compound with formula (A) or salt thereof is also referred to in the following as an "ICG derivative lipid".

For example, the ICG lipid derivative exhibits absorption and fluorescence in the near-infrared region of 700 to 1300 nm and particularly approximately 700 to 900 nm, and the molar extinction coefficient anywhere in the 700 to 1300 nm (preferably 700 to 900 nm) wavelength region can be at least $1.6 \times 10^5$ [L/mol/cm].

$R_1$ and $R_2$ in formula (A) each independently represent $-(CH_2)_k-CONH-R_3$. $R_1$ and $R_2$ may be the same as each other or may differ from one another.

$R_3$ in formula (A) represents a group selected from the group consisting of $-(CH_2)_m-OPO_3^--CH_2-CH(CH_2OCOR_4)(OCOR_5)$, straight-chain $C_{14}$ to $C_{22}$ alkyl, straight-chain $C_{14}$ to $C_{22}$ alkenyl, branched-chain $C_{14}$ to $C_{40}$ alkyl, and branched-chain $C_{14}$ to $C_{40}$ alkenyl. The "$-OPO_3^--$" represents the following structure (the * represents a connecting point) in this Description.

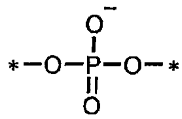

$R_4$ and $R_5$ each independently represent straight-chain or branched-chain $C_{13}$ to $C_{21}$ alkyl or straight-chain or branched-chain $C_{13}$ to $C_{21}$ alkenyl. $R_4$ and $R_5$ may be the same as each other or may differ from one another.

The k in formula (A) represents an integer from 2 to 4. This k is preferably 2 or 3 and is more preferably 2.

The m in formula (A) represents an integer from 2 to 4. This m is preferably 2 or 3 and is more preferably 2.

In some embodiments, the compound represented by formula (A) or a pharmaceutically acceptable salt thereof is a compound, or a pharmaceutically acceptable salt thereof, in which, in formula (A),

$R_1$ and $R_2$ each independently represent $-(CH_2)_k-CONH-R_3$;
$R_3$ represents a group selected from the group consisting of $-(CH_2)_m-OPO_3^--CH_2-CH(CH_2OCOR_4)(OCOR_5)$, branched-chain $C_{14}$ to $C_{40}$ alkyl, and branched-chain $C_{14}$ to $C_{40}$ alkenyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4 (preferably 2 or 3 and more preferably 2); and
m represents an integer from 2 to 4 (preferably 2 or 3 and more preferably 2).

Compounds according to these embodiments are preferred for at least one of the following advantages: biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat generation and drug releasability), and so forth.

In some embodiments, the compound represented by formula (A) or a pharmaceutically acceptable salt thereof is

a compound, or a pharmaceutically acceptable salt thereof, in which, in formula (A),

$R_1$ and $R_2$ each independently represent - $(CH_2)_k$ - CONH - $R_3$;
$R_3$ represents a group selected from the group consisting of - $(CH_2)_m$ - $OPO_3^-$ - $CH_2$ - $CH(CH_2OCOR_4)(OCOR_5)$, straight-chain $C_{14}$ to $C_{22}$ alkyl, and branched-chain $C_{14}$ to $C_{40}$ alkyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4 (preferably 2 or 3 and more preferably 2); and
m represents an integer from 2 to 4 (preferably 2 or 3 and more preferably 2).

Compounds according to these embodiments are preferred for at least one of the following advantages: biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat generation and drug releasability), and so forth.
In some embodiments, the compound represented by formula (A) or a pharmaceutically acceptable salt thereof is a compound, or a pharmaceutically acceptable salt thereof, in which, in formula (A),

$R_1$ and $R_2$ each independently represent - $(CH_2)_k$ - CONH - $R_3$;
$R_3$ represents a group selected from the group consisting of - $(CH_2)_m$ - $OPO_3^-$ - $CH_2$ - $CH(CH_2OCOR_4)(OCOR_5)$ and branched-chain $C_{14}$ to $C_{40}$ alkyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4 (preferably 2 or 3 and more preferably 2); and
m represents an integer from 2 to 4 (preferably 2 or 3 and more preferably 2).

Compounds according to these embodiments are preferred for at least one of the following advantages: biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat generation and drug releasability), and so forth.
In some embodiments, the compound represented by formula (A) or a pharmaceutically acceptable salt thereof is a compound, or a pharmaceutically acceptable salt thereof, in which, in formula (A),

$R_1$ and $R_2$ each independently represent - $(CH_2)_k$ - CONH - $R_3$;
$R_3$ represents - $(CH_2)_m$ - $OPO_3^-$ - $CH_2$ - $CH(CH_2OCOR_4)(OCOR_5)$;
$R_4$ and $R_5$ each independently represent straight-chain or branched-chain $C_{13}$ to $C_{21}$ alkyl or straight-chain or branched-chain $C_{12}$ to $C_{22}$ alkenyl (preferably straight-chain $C_{13}$ to $C_{21}$ alkyl or straight-chain $C_{13}$ to $C_{21}$ alkenyl);
k represents an integer from 2 to 4 (preferably 2 or 3 and more preferably 2); and
m represents an integer from 2 to 4 (preferably 2 or 3 and more preferably 2).

Compounds according to these embodiments are preferred for at least one of the following advantages: biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat generation and drug releasability), and so forth.
In some embodiments, the compound represented by formula (A) or a pharmaceutically acceptable salt thereof is a compound, or a pharmaceutically acceptable salt thereof, in which, in formula (A),

$R_1$ and $R_2$ each independently represent - $(CH_2)_2$ - CONH - $R_3$;
$R_3$ represents - $(CH_2)_m$ - $OPO_3^-$ - $CH_2$ - $CH(CH_2OCOR_4)(OCOR_5)$;
$R_4$ and $R_5$ each independently represent straight-chain $C_{13}$ to $C_{21}$ alkyl or straight-chain $C_{13}$ to $C_{21}$ alkenyl; and
m represents an integer from 2 to 4 (preferably 2 or 3 and more preferably 2).

Compounds according to these embodiments are preferred for at least one of the following advantages: biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat

generation and drug releasability), and so forth.

In an embodiment, the compound represented by formula (A) or a pharmaceutically acceptable salt thereof is a compound represented by formula (B) below or a pharmaceutically acceptable salt thereof.

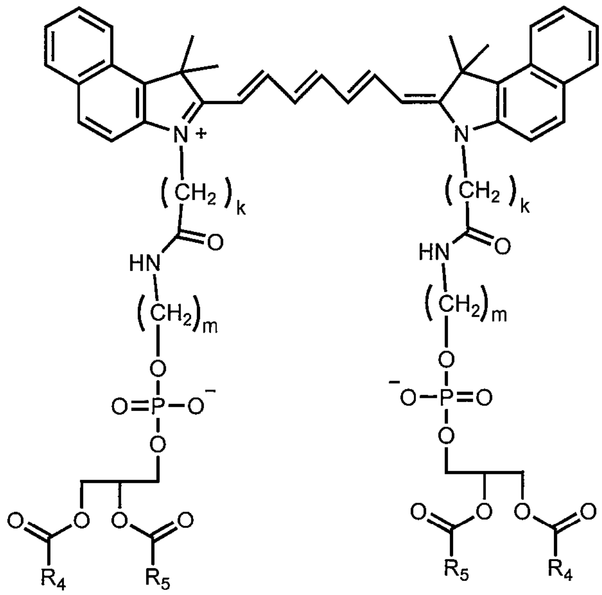

(B)

In formula (B),

$R_4$ and $R_5$ each independently represent straight-chain or branched-chain $C_{13}$ to $C_{21}$ alkyl or straight-chain or branched-chain $C_{12}$ to $C_{22}$ alkenyl;
each k independently represents an integer from 2 to 4 (preferably 2 or 3 and more preferably 2); and
each m independently represents an integer from 2 to 4 (preferably 2 or 3 and more preferably 2).

Compounds according to this embodiment are preferred for at least one of the following advantages: biocompatibility, ease of synthesis, affinity for lipid-based particles, chemical stability, physical stability of the lipid-based particle (inhibition of aggregation), stability and drug encapsulation ratio when a drug is encapsulated in the lipid-based particle, reactivity of the lipid-based particle upon irradiation with near-infrared light (heat generation and drug releasability), and so forth.

In an embodiment, in formula (B),

$R_4$ and $R_5$ each independently represent straight-chain $C_{13}$ to $C_{21}$ alkyl or straight-chain $C_{12}$ to $C_{22}$ alkenyl;
each k independently represents an integer from 2 to 4 (preferably 2 or 3 and more preferably 2); and
each m independently represents an integer from 2 to 4 (preferably 2 or 3 and more preferably 2).

Compounds according to this embodiment are preferred for at least one of the following advantages: biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat generation and drug releasability), and so forth.

In some embodiments, $R_4$ and $R_5$ in the aforementioned formula (B) are the same as each other.

In some embodiments, the compound represented by formula (A) or a pharmaceutically acceptable salt thereof is a compound, or a pharmaceutically acceptable salt thereof, in which, in formula (A),

$R_1$ and $R_2$ each independently represent $-(CH_2)_k-CONH-R_3$;
$R_3$ represents a group selected from the group consisting of straight-chain $C_{14}$ to $C_{22}$ alkyl, straight-chain $C_{14}$ to $C_{22}$ alkenyl, branched-chain $C_{14}$ to $C_{40}$ alkyl, and branched-chain $C_{14}$ to $C_{40}$ alkenyl;
k represents an integer from 2 to 4 (preferably 2 or 3 and more preferably 2); and
m represents an integer from 2 to 4 (preferably 2 or 3 and more preferably 2).

Compounds according to these embodiments are preferred for at least one of the following advantages:

biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat generation and drug releasability), and so forth.

In some embodiments, the compound represented by formula (A) or a pharmaceutically acceptable salt thereof is a compound, or a pharmaceutically acceptable salt thereof, in which, in formula (A),

$R_1$ and $R_2$ each independently represent - $(CH_2)_k$ - CONH - $R_3$;
$R_3$ represents a group selected from the group consisting of branched-chain $C_{14}$ to $C_{40}$ alkyl and branched-chain $C_{14}$ to $C_{40}$ alkenyl; and
k represents an integer from 2 to 4 (preferably 2 or 3 and more preferably 2).

Compounds according to these embodiments are preferred for at least one of the following advantages: biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat generation and drug releasability), and so forth.

In some embodiments, the compound represented by formula (A) or a pharmaceutically acceptable salt thereof is a compound, or a pharmaceutically acceptable salt thereof, in which, in formula (A),

$R_1$ and $R_2$ each independently represent - $(CH_2)_2$ - CONH - $R_3$, and
$R_3$ represents a group selected from the group consisting of branched-chain $C_{14}$ to $C_{40}$ alkyl and branched-chain $C_{14}$ to $C_{40}$ alkenyl.

Compounds according to these embodiments are preferred for at least one of the following advantages: biocompatibility, ease of synthesis, affinity for lipid-based particles, chemical stability, physical stability of the lipid-based particle (inhibition of aggregation), stability and drug encapsulation ratio of the lipid-based particle carrier when a drug is encapsulated in the lipid-based particle, reactivity of the lipid-based particle upon irradiation with near-infrared light (heat generation and drug releasability), and so forth.

In some embodiments, the compound represented by formula (A) or a pharmaceutically acceptable salt thereof is a compound represented by formula (C) below or a pharmaceutically acceptable salt thereof.

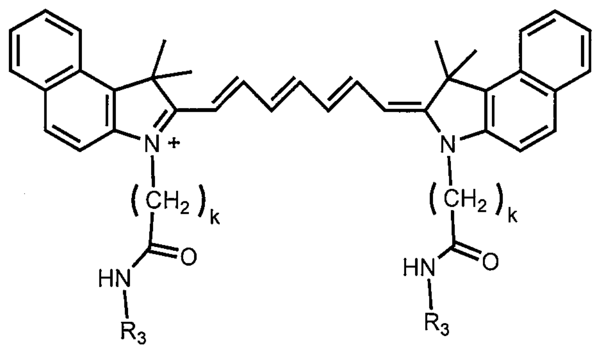

**(C)**

In formula (C),

each $R_3$ independently represents a group selected from the group consisting of straight-chain $C_{14}$ to $C_{22}$ alkyl, straight-chain $C_{14}$ to $C_{22}$ alkenyl, branched-chain $C_{14}$ to $C_{40}$ alkyl, and branched-chain $C_{14}$ to $C_{40}$ alkenyl; and
each k independently represents an integer from 2 to 4 (preferably 2 or 3 and more preferably 2).

In an embodiment, in formula (C),

each $R_3$ independently represents a group selected from the group consisting of branched-chain $C_{14}$ to $C_{40}$ alkyl and branched-chain $C_{14}$ to $C_{40}$ alkenyl, and
each k independently represents an integer from 2 to 4 (preferably 2 or 3 and more preferably 2).

In an embodiment, in formula (C),

each $R_3$ independently represents a branched-chain $C_{14}$ to $C_{40}$ alkyl, and
each k independently represents an integer from 2 to 4 (preferably 2 or 3 and more preferably 2).

In the embodiments described above, the straight-chain $C_{13}$ to $C_{21}$ alkyl cited for $R_4$ or $R_5$ in formula (A) or formula (B) is, for example, straight-chain $C_{13}$ to $C_{19}$ alkyl, and is, for example, straight-chain $C_{15}$ to $C_{17}$ alkyl.

In the embodiments described above, the branched-chain $C_{13}$ to $C_{21}$ alkyl cited for $R_4$ or $R_5$ in formula (A) or formula (B) is, for example, branched-chain $C_{13}$ to $C_{19}$ alkyl, and is, for example, branched-chain $C_{15}$ to $C_{17}$ alkyl, for example, 1,3-dimethylheptadecyl.

In the embodiments described above, the straight-chain $C_{13}$ to $C_{21}$ alkenyl cited for $R_4$ or $R_5$ in formula (A) or formula (B) is, for example, straight-chain $C_{13}$ to $C_{19}$ alkenyl, and is, for example, straight-chain $C_{15}$ to $C_{17}$ alkenyl, for example, 8-heptadecenyl.

In the embodiments described above, the branched-chain $C_{13}$ to $C_{21}$ alkenyl cited for $R_4$ or $R_5$ in formula (A) or formula (B) is, for example, branched-chain $C_{13}$ to $C_{19}$ alkenyl, and is, for example, branched-chain $C_{15}$ to $C_{17}$ alkenyl, for example, 1,3-dimethyl-8-heptadecenyl.

In the embodiments described above, the straight-chain $C_{14}$ to $C_{22}$ alkyl cited for $R_3$ in formula (A) or formula (C) is, for example, straight-chain $C_{14}$ to $C_{20}$ alkyl, and is, for example, straight-chain $C_{16}$ to $C_{18}$ alkyl.

In the embodiments described above, the straight-chain $C_{14}$ to $C_{22}$ alkenyl cited for $R_3$ in formula (A) or formula (C) is, for example, straight-chain $C_{14}$ to $C_{20}$ alkenyl, and is, for example, straight-chain $C_{16}$ to $C_{18}$ alkenyl, for example, 8-heptadecenyl.

In the embodiments described above, the branched-chain $C_{14}$ to $C_{40}$ alkyl cited for $R_3$ in formula (A) or formula (C) is, for example, branched-chain $C_{20}$ to $C_{40}$ alkyl, and is, for example, branched-chain $C_{28}$ to $C_{40}$ alkyl, for example, heptatriacontan-19-yl.

In the embodiments described above, the branched-chain $C_{14}$ to $C_{40}$ alkenyl cited for $R_3$ in formula (A) or formula (C) is, for example, branched-chain $C_{20}$ to $C_{40}$ alkenyl, and is, for example, branched-chain $C_{28}$ to $C_{40}$ alkenyl.

In the embodiments described above, the $R_3$ in formula (A) and formula (C) represents - $CHR_{31}R_{32}$ wherein $R_{31}$ and $R_{32}$ each independently represent straight-chain $C_7$ to $C_{19}$ (preferably $C_{10}$ to $C_{19}$ and more preferably $C_{14}$ to $C_{19}$) alkyl, and
each k independently represents an integer from 2 to 4 (preferably 2 or 3 and more preferably 2).

An example of compounds according to the embodiments is given below.

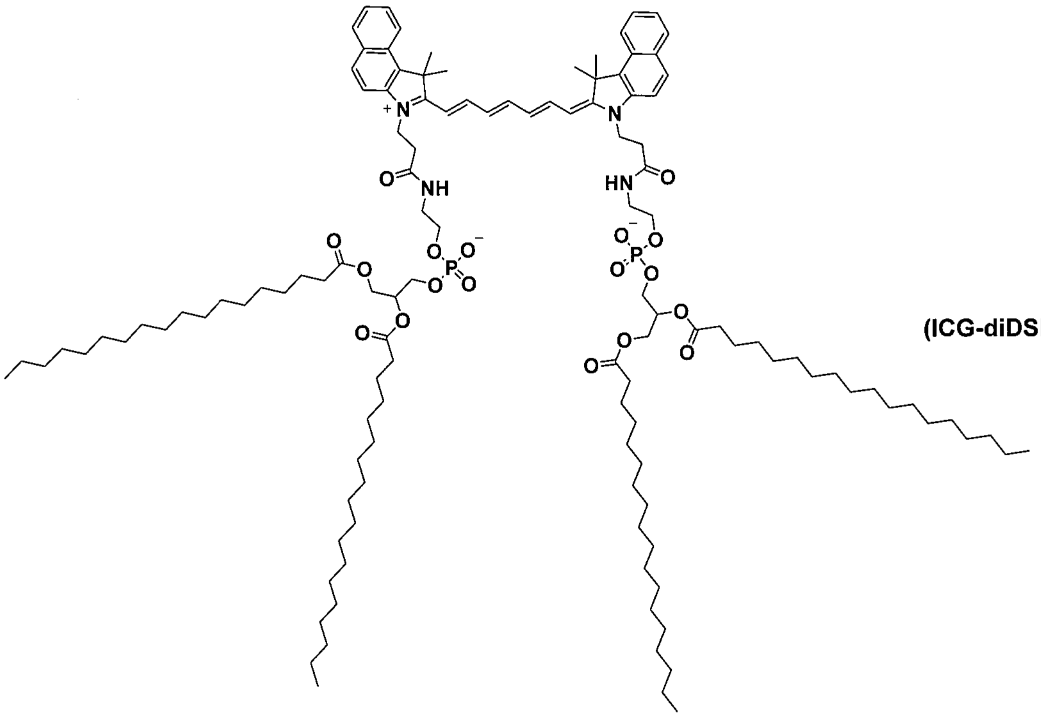

(I)
(ICG-diDSPE)

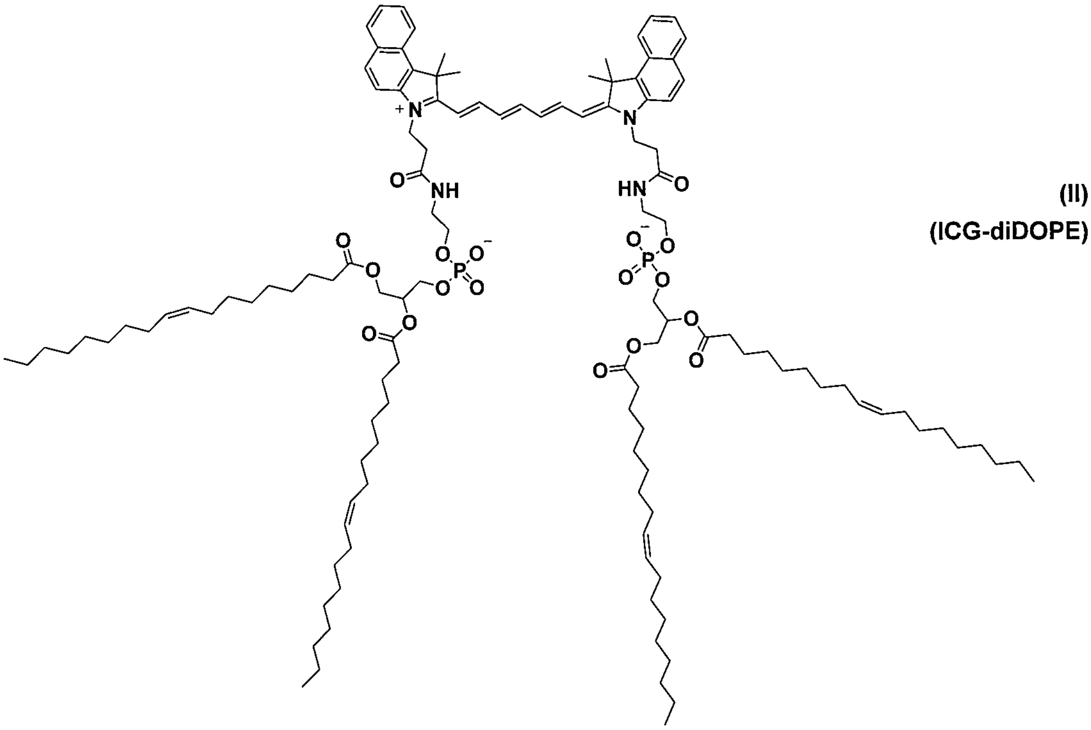

(II)
(ICG-diDOPE)

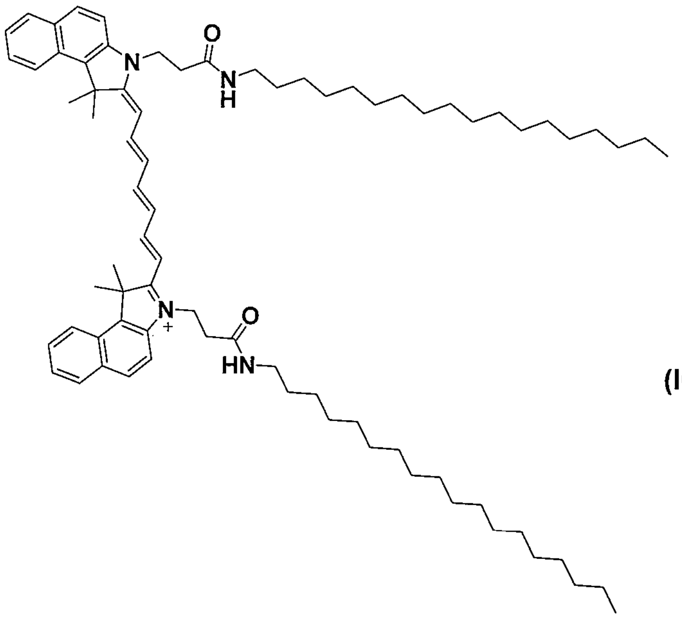

(III)
(ICG-diSA)

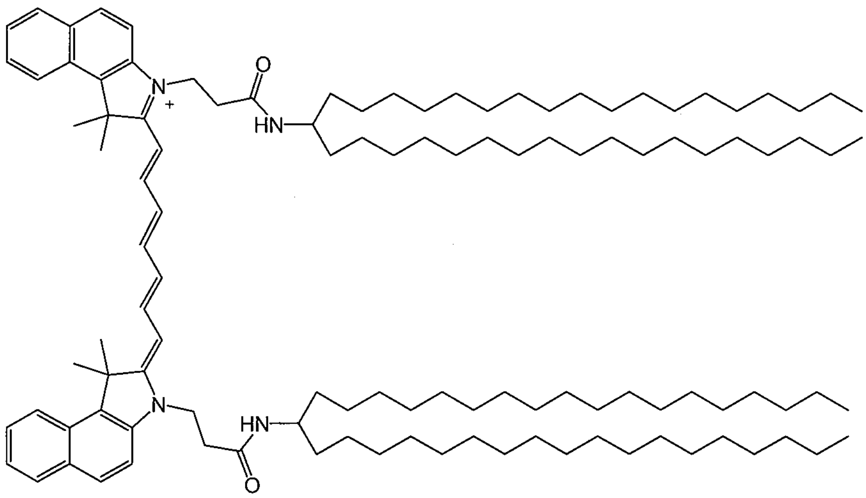

(IV)

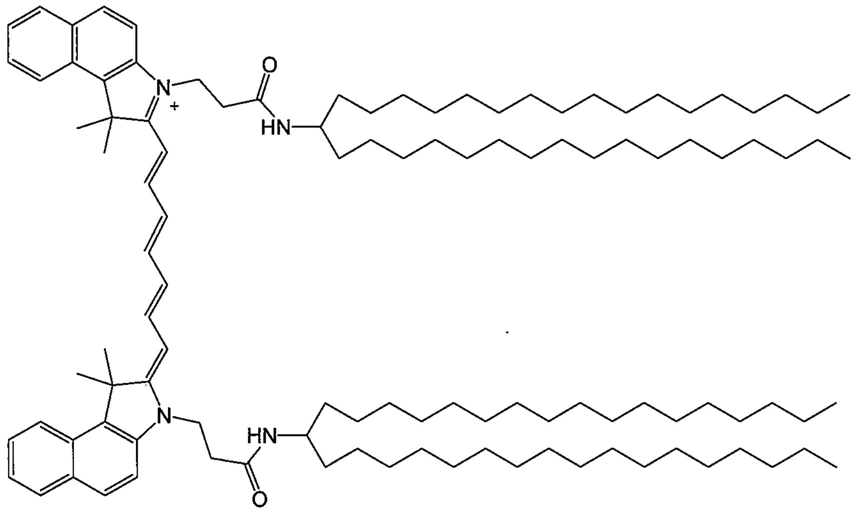

(V)

An embodiment of the present invention is a compound represented by any of the preceding formula (I) (ICG-diDSPE), the preceding formula (II) (ICG-diDOPE), the preceding formula (III) (ICG-diSA), or the preceding formula (IV), or a pharmaceutically acceptable salt thereof. A particular embodiment is a compound represented by any of the preceding formula (I) (ICG-diDSPE) or the preceding formula (II) (ICG-diDOPE), or a pharmaceutically acceptable salt thereof. A particular embodiment is a compound represented by any of the preceding formula (I) (ICG-diDSPE), the preceding formula (II) (ICG-diDOPE), and the preceding formula (III) (ICG-diSA), or a pharmaceutically acceptable salt thereof.

A "pharmaceutically acceptable salt" refers to a salt suitable for use, within the scope of sound medical judgement, in contact with human and animal tissue without causing excessive toxicity or irritation or an allergic response. Pharmaceutically acceptable salts are well known in the instant technical field. For example, pharmaceutically acceptable salts are described in detail by S. M. Berge et al. in J. Pharmaceutical Sciences 66: 1-19, 1977. The salt can be produced by reacting the free base with a suitable organic acid, either in situ during the final isolation and purification of the compound or separately from this.

Representative acid addition salts can be exemplified by the following: the acetic acid salt, adipic acid salt, alginic acid salt, ascorbic acid salt, aspartic acid salt, benzenesulfonic acid salt, benzoic acid salt, bisulfate salt, boric acid salt, butyric acid salt, camphoric acid salt, camphorsulfonic acid salt, citric acid salt, cyclopentanepropionic acid salt, digluconic acid salt, dodecyl sulfate salt, ethanesulfonic acid salt, fumaric acid salt, glucoheptonic acid salt, glycerophosphate salt, hemisulfate salt, heptonic acid salt, hexanoic acid salt, hydrobromic acid salt, hydrochloric acid salt, hydroiodic acid salt, 2-hydroxyethanesulfonic acid salt, lactobionic acid salt, lactic acid salt, lauric acid salt, lauryl sulfate salt, malic acid salt, maleic acid salt, monomaleic acid, malonic acid salt, methanesulfonic acid salt, 2-naphthalenesulfonic acid salt, nicotinic acid salt, nitric acid salt, oleic acid salt, oxalic acid salt, palmitic acid salt, pamoic acid salt, pectic acid salt, persulfate salt, 3-phenylpropionic acid salt, phosphate salt, picric acid salt, pivalic acid salt, propionic acid salt, stearic acid salt, succinic acid salt, sulfuric acid salt, tartaric acid salt, thiocyanic acid salt, toluenesulfonic acid salt, trifluoroacetic acid salt, undecanoic acid salt, and valeric acid salt.

Representative alkali metal salts and alkaline earth metal salts can be exemplified by the sodium salt, lithium salt, potassium salt, calcium salt, and magnesium salt, and nontoxic ammonium, quaternary ammonium, and cations of amines. Examples here are as follows, although there is no limitation to the following: ammonium, tetramethylammonium, tetraethylammonium, and cations of, e.g., methylamine, dimethylamine, trimethylamine, triethylamine, and ethylamine.

Stereoisomers, e.g., geometric isomers and optical isomers, as well as tautomers, etc. may be present in the compounds represented by formula (A) in the present invention, and the compounds according to the present invention encompass all possible isomers and their mixtures. In addition, the compounds represented by formula (A), or their salts, may be in the form of a hydrate or solvate, and the hydrates and solvates are also encompassed by the compounds according to the present invention.

(Methods for producing the ICG derivative lipids)

Methods for producing the ICG derivative lipids according to the present invention will now be described.

The formula (A) compounds can be synthesized with reference to, for example, the methods described in J. Am. Chem. Soc. 2003, 125, 7766, WO 2011/152046, and WO 2013/051732. For example, synthesis can be carried out according to the scheme 1 described in the preceding.

Scheme 1

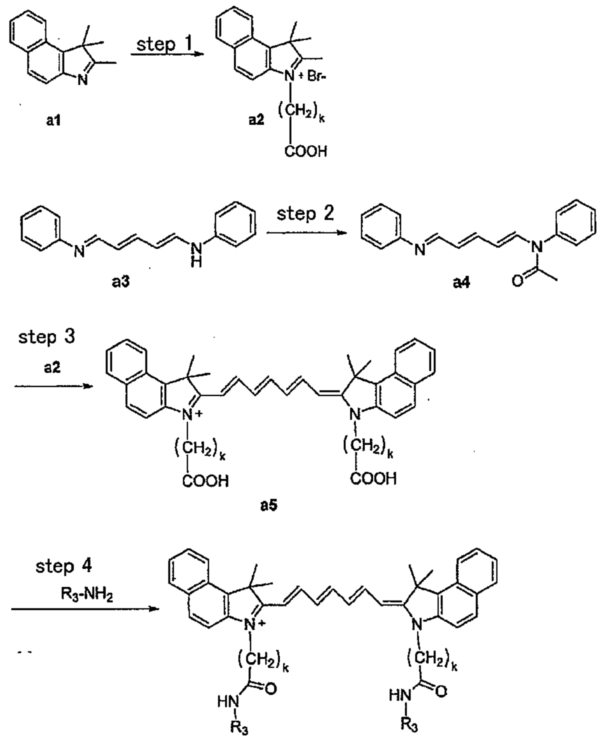


(A)

(In the formulas, k and $R_3$ are each defined as for the preceding formula (A).)

(Step 1)

The carboxyalkyl-substituted indole derivative (a2) is obtained by reacting 1,1,2-trimethyl[1H]benz[e]indole (a1) and a halogen-substituted carboxylic acid X - $(CH_2)_k$ - COOH (X is a halogen atom, preferably bromine (Br)). The solvent is, for example, dichlorobenzene, dichloromethane, toluene, and so forth.

(Step 2)

Compound (a4) is obtained by reacting acetic anhydride and glutaconaldehyde dianilide (a3), preferably in the presence of a base (for example, N,N-diisopropylethylamine (DIPEA)). The solvent is, for example, dichoromethane and so forth.

(Step 3: Dicarboxylic acid synthesis)

The carboxyalkyl-substituted indole derivative (a2) and compound (a4) are then reacted in the presence of a base to obtain the indocyanine green dicarboxylic acid compound (a5), in which the carboxyalkyl-substituted indole derivative is attached at both ends of the hexatriene chain. The base is exemplified by sodium acetate, pyridine, and so forth. The solvent is exemplified by methanol, tetrahydrofuran, and so forth.

A compound in which the alkyl chain $-(CH_2)_k-$ of the carboxyalkyl group substituted on the indole ring on the right is different from that on the left can be obtained by attaching a carboxyalkyl-substituted indole derivative to one end of the hexatriene chain by reacting one equivalent of the carboxyalkyl-substituted indole derivative (a2) with (a4), followed by another addition reaction with a carboxyalkyl-substituted indole derivative (a2) having a different number of carbons (k) in the alkyl chain.

(Step 4: amidation)

The target formula (A) compound is obtained by carrying out an amidation by reacting an amine $R_3-NH_2$ with the dicarboxylic acid compound (a5).

More specifically, a compound with the aforementioned formula (B) can be produced by using a compound given by the following (a6) as the $R_3-NH_2$.

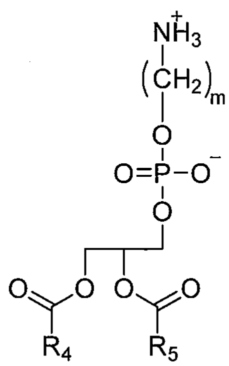

(a6)

(In the formula, m, $R_4$, and $R_5$ are defined as for formula (B).)

In addition, a compound with the previously indicated formula (C) is obtained by using an $R_3-NH_2$ in which $R_3$ is straight-chain $C_{14}$ to $C_{22}$ alkyl, straight-chain $C_{14}$ to $C_{22}$ alkenyl, branched-chain $C_{14}$ to $C_{40}$ alkyl, or branched-chain $C_{14}$ to $C_{40}$ alkenyl.

The $R_3-NH_2$ may be synthesized by a common method or a commercially available $R_3-NH_2$ may be used.

The amidation reaction may be carried out according to the usual means; for example, it is preferably run in the presence of a condensing agent such as, for example, a benzotriazole, e.g., 1-hydroxybenzotriazole (HOBt), N-hydroxysuccinimide (HOSu), 1-hydroxybenzotriazole (HOAt), 1-[bis(dimethylamino)methylene]-1H-benzotriazolium 3-oxide hexafluorophosphate (HBTU), and so forth, or 1-[3-(dimethylamino)propyl]-3-ethylcarbodiimide (EDC) hydrochloride, N,N'-dicyclohexylcarbodiimide (DCC), and so forth. A base may also be added to the reaction as necessary, and the base can be exemplified by N-methylmorpholine (NMM), triethylamine (TEA), N,N-diisopropylethylamine (DIPEA), 4-(dimethylamino)pyridine (DMAP), and so forth. The solvent can be exemplified by dimethylformamide, tetrahydrofuran (THF), dichloromethane, acetonitrile, and so forth.

To obtain a compound in which the two $R_3$'s in formula (A) are different, one equivalent of the carboxyalkyl-substituted indole derivative (a2) may be reacted with (a4) in (step 3) to attach the carboxyalkyl-substituted indole derivative at one terminal of the hexatriene chain, followed by reaction with $R_3-NH_2$ to synthesize a compound in which $R_3-NH_2$ has been added to only one side of (a4); $R_3-NH_2$ bearing a different $R_3$ may be reacted by carrying out the same process on the other side.

In the synthesis of compounds according to the present invention, when the production of the starting materials is not particularly described, these compounds are known or can be produced by analogous methods known in the concerned technical field or can be produced according to the description provided in the examples herebelow. The person skilled in the art will understand that the scheme provided above is nothing more than simply a representative method for producing the compounds according to the present invention, and other well-known methods can be similarly used.

Compounds according to the present invention may have one or more asymmetric centers, and such compounds may be produced as the individual (R)- or (S)-stereoisomer or as a mixture thereof. Unless specified otherwise, the

description of particular compounds in this Description intends to encompass both the individual enantiomers and their racemic mixtures. Methods for establishing the stereochemistry and separating stereoisomers are well-known to the person skilled in the art.

2. The lipid-based particle

An embodiment of the present invention provides a lipid-based particle that contains an ICG derivative lipid according to a hereinabove-described embodiment (also referred to in this Description simply as a "lipid-based particle").

Since the ICG derivative lipid is provided with the ICG structure for a part of its structure, it may itself form an ICG-supporting lipid-based particle. Accordingly, in some embodiments, the lipid-based particle is constructed from the ICG derivative lipid described in the preceding.

The configuration of the lipid particle is not particularly limited and may be any of, for example, a polymer micelle, liposome, emulsion, microsphere, nanosphere, and so forth.

In some embodiments, the lipid-based particle is a polymer micelle or liposome that is formed from a single or a plurality of lipids that includes the hereinabove-described ICG derivative lipid. In some other embodiments, the lipid-based particle is, for example, at least one selection from liposomes, lipid particles, O/W emulsions that use lipid as an interfacial substance, and so forth. The lipid-based particle is a liposome in an embodiment.

In some embodiments, the lipid-based particle is present in an aqueous solvent in the form of a polymer micelle or liposome that is formed from a single or a plurality of lipids that includes the hereinabove-described ICG derivative lipid. The aqueous solvent can be exemplified by sterilized water; physiological saline; isotonic solutions that contain an auxiliary reagent, e.g., glucose, D-sorbitol, D-mannose, D-mannitol, sodium chloride, and so forth; and buffers such as phosphate buffers, citrate buffers, acetate buffers, and so forth.

In a specific embodiment, the lipid-based particle is, for example, a liposome. The liposome according to an embodiment is constructed of a lipid bilayer that contains the hereinabove-described ICG derivative lipid, and has an aqueous phase (internal phase) in the interior (the space in the closed vesicle formed by the lipid bilayer) of the lipid bilayer. The liposome may have a single lipid bilayer or may have a plurality of lipid bilayers, but a liposome having a monolamellar lipid bilayer structure is preferred from a stability standpoint.

With reference to the total lipid contained in the lipid-based particle, the lipid-based particle of the present embodiment contains the ICG derivative lipid according to the hereinabove-described embodiments at, for example, 0.001 to 100 mol%, for example, 0.01 to 20 mol%, for example, 0.1 to 10 mol%. A single species of the ICG derivative lipid may be used alone or a mixture of two or more species may be used.

The lipid-based particle according to a representative embodiment further contains at least one lipid selected from the group consisting of neutral lipids, polyethylene glycol-modified lipids, and sterols. The ICG derivative lipid according to the present invention exhibits a high affinity with these lipids and can form a very stable particle structure.

The lipid-based particle according to some embodiments contains, as its lipid component, (I) the hereinabove-described ICG derivative lipid and (II) at least one lipid selected from the group consisting of neutral lipids, polyethylene glycol-modified lipids, and sterols, and contains the lipid component at, for example, 50 to 100 weight%, for example, 70 to 100 weight%, and, for example, 90 to 100 weight%, with reference to the total weight of the lipid-based particle. The lipid-based particle is, for example, a polymer micelle or liposome (preferably a liposome) that is formed from the hereinabove-described ICG derivative lipid and at least one lipid selected from the group consisting of neutral lipids, polyethylene glycol-modified lipids, and sterols.

The lipid component combination in the lipid-based particle according to the present embodiments is not particularly limited, and can be exemplified by combinations of the hereinabove-described ICG derivative lipid with a neutral lipid and a sterol and combinations of the hereinabove-described ICG derivative lipid with a neutral lipid, a polyethylene glycol-modified lipid, and a sterol.

Neutral lipid denotes a lipid that is present, at physiological pH, in either an uncharged form or in the form of a neutral amphoteric ion. The neutral lipids can be exemplified by dioleoylphosphatidylethanolamine (DOPE), palmitoyloleoylphosphatidylcholine (POPC), egg phosphatidylcholine (EPC), dimyristoylphosphatidylcholine (DMPC), dipalmitoylphosphatidylcholine (DPPC), distearoylphosphatidylcholine (DSPC), diarachidoylphosphatidylcholine (DAPC), dibehenoylphosphatidylcholine (DBPC), dilignoceroyl phosphatidylcholine (DLPC), dioleoylphosphatidylcholine (DOPC), sphingomyelin, ceramide, dioleoylphosphatidylglycerol (DOPG), dipalmitoylphosphatidylglycerol (DPPG), phosphatidylethanolamine (POPE), dioleoylphosphatidylethanolamine 4-(N-maleimidomethyl)cyclohexane-1-carboxylate (DOPE-mal), and so forth. A single species of neutral lipid may be used alone or a mixture of two or more species may be used.

With reference to the total lipid contained by the lipid-based particle, the lipid-based particle of the present embodiments may contain the neutral lipid at, for example, 0 to 99.999 mol%, for example, 50 to 99.99 mol%, for example, 60 to 99.9 mol%.

The polyethylene glycol-modified lipid can be exemplified by PEG2000-DMG (PEG2000-dimyristylglycerol), PEG2000-DPG (PEG2000-dipalmitoylglycerol), PEG2000-DSG (PEG2000-distearoylglycerol), PEG5000-DMG

(PEG5000-dimyristylglycerol), PEG5000-DPG (PEG5000-dipalmitoylglycerol), PEG5000-DSG (PEG5000-distearoylglycerol), PEG-cDMA (N-[(methoxypoly(ethylene glycol)2000)carbamyl]-1,2-dimyristyloxylpropyl-3-amine), PEG-C-DOMG (R-3-[(ω-methoxypoly(ethylene glycol)2000)carbamoyl)]-1,2-dimyristyloxylpropyl-3-amine), polyethylene glycol(PEG)-diacylglycerol (DAG), PEG-dialkyloxypropyl (DAA), PEG-phospholipid, PEG-ceramide (Cer), and so forth.

PEG-dialkyloxypropyl can be exemplified by PEG-dilauryloxypropyl, PEG-dimyristyloxypropyl, PEG-dipalmityloxypropyl, PEG-distearyloxypropyl, and so forth.

PEG-phospholipids can be exemplified by PEG2000-DSPE ((poly(ethylene glycol)2000)-1,2-distearoyl-sn-glycero-3-phosphoethanolamine), MPEG2000-DSPE ((methoxypoly(ethylene glycol)2000)-1,2-distearoyl-sn-glycero-3-phosphoethanolamine), and so forth.

A single species of the polyethylene glycol-modified lipid may be used alone or a mixture of two or more species may be used. The terminals of the PEG (polyethylene glycol) in these polyethylene glycol-modified lipids may be methoxylated (MPEG, methoxy(polyethylene glycol)).

The lipid-based particle according to the present embodiments may contain a polyethylene glycol-modified lipid at, for example, 0 to 30 mol%, for example, 0 to 20 mol%, for example, 0 to 10 mol%, with reference to the total lipid contained by the lipid-based particle.

The sterol is an alcohol having the steroid skeleton. The sterol can be exemplified by cholesterol, dehydrocholesterol, lanosterol, β-sitosterol, campesterol, stigmasterol, brassicasterol, ergosterol, fucosterol, 3β-[N-(N',N'-dimethylaminoethyl)carbamoyl]cholesterol (DC-Chol), and so forth. A single species of the sterol may be used alone or a mixture of two or more species may be used.

The lipid-based particle according to the present embodiments may contain a sterol at, for example, 0 to 90 mol%, for example, 0 to 80 mol%, for example, 0 to 50 mol% or 0 to 40 mol%, for example, 0 to 30 mol%, for example, 0 to 20 mol%, for example, 0 to 10 mol%, for example, 0 to 5 mol%, with reference to the total lipid contained by the lipid-based particle. The stability in the blood can be improved by the presence of a sterol. On the other hand, the heat sensitivity exhibits a declining trend as the amount of sterol increases. In an embodiment, the lipid-based particle does not contain a sterol.

In an example of a blend of lipid components in the lipid-based particle of the present embodiments, the hereinabove-described ICG derivative lipid is contained at 0.01 to 20 mol%, neutral lipid is contained at 50 to 99.99 mol%, and sterol is contained at 0 to 49.99 mol%, in each case with reference to the total lipid contained by the lipid-based particle.

In another example, the hereinabove-described ICG derivative lipid is contained at 0.1 to 10 mol%, neutral lipid is contained at 60 to 99.9 mol%, polyethylene glycol-modified lipid is contained at 0 to 10 mol%, and sterol is contained at 0 to 39.9 mol%, in each case with reference to the total lipid contained by the lipid-based particle.

In another example, the hereinabove-described ICG derivative lipid is contained at 0.1 to 10 mol%, neutral lipid is contained at 70 to 99.9 mol%, polyethylene glycol-modified lipid is contained at 0 to 10 mol%, and sterol is contained at 0 to 10 mol%, in each case with reference to the total lipid contained by the lipid-based particle.

In another example, the hereinabove-described ICG derivative lipid is contained at 0.1 to 10 mol%, neutral lipid is contained at 80 to 99.9 mol%, and polyethylene glycol-modified lipid is contained at 0 to 10 mol%, in each case with reference to the total lipid contained by the lipid-based particle.

The lipid-based particle according to some embodiments further comprises at least one drug. Embodiments in which the lipid-based particle is formed by a drug and ICG derivative lipid-containing lipid can be exemplified by composites of the drug and a membrane (inverse micelle) composed of a lipid mono(monomolecular)layer, drug/liposome composites, and drug/micelle (true micelle) composites.

In some embodiments, a drug is encapsulated within a lipid-based particle constructed of lipid that includes the ICG derivative lipid. This "encapsulated" denotes the assumption of a condition or state in which the drug is contained in the lipid layer itself or within a closed spaced formed by the lipid layer.

In an embodiment, a drug is contained in a closed spaced formed by a lipid layer of the lipid-based particle. In another embodiment, the drug is present within one lipid layer or a plurality of lipid layers of the lipid-based particle. In another embodiment, the drug is linked to the surface of an outer lipid layer of the lipid-based particle or the surface of an inner lipid layer of the lipid-based particle.

In a particular embodiment, a drug is contained in the internal phase and/or a lipid layer of a liposome constructed of lipid containing the ICG derivative lipid.

The drug may be water soluble or lipid soluble. In addition, the drug may be hydrophilic or hydrophobic.

In some embodiments, the drug is water soluble. In a particular embodiment, the lipid-based particle is present in an aqueous solvent and encapsulates a water-soluble drug within a closed space formed by a lipid layer of the lipid-based particle (for example, the internal aqueous phase of the lipid-based particle, such as the internal phase of a liposome) and/or within one lipid layer or a plurality of lipid layers of the lipid-based particle (within a monolamellar or polylamellar lipid layer). The aqueous solvent can be exemplified by sterilized water; physiological saline; isotonic solutions that contain an auxiliary reagent, e.g., glucose, D-sorbitol, D-mannose, D-mannitol, sodium chloride, and so forth; and buffers such as phosphate buffers, citrate buffers, acetate buffers, and so forth.

The drug may be, for example, any of the following: a synthetic compound, fermentation product, peptide, protein,

steroid compound, gene, virus, saccharide (polysaccharide), fatty acid, vitamin, or coenzyme (or derivative). In addition, it may be a drug as used in any field, e.g., medicines, agrochemicals, fertilizers, and so forth, but a medicine is preferably used. The drug can be, for example, a freely selected molecule or compound (for example, an active drug) capable of exercising a desired effect on a cell, tissue, organ, or subject. The drugs provided here are only examples and a limitation to these is not intended. These drugs may be purified or partially purified and may be naturally occurring or derived from nature, or may be synthesized, or may by the product of genetic technology, or may be chemically modified.

In some embodiments, the drug is a therapeutic drug.

In some embodiments, the drug is a low molecular weight compound.

In some embodiments, the drug comprises at least one selection from anticancer agents or antimicrobials.

In one embodiment, the drug comprises an anticancer agent.

In a particular embodiment, the anticancer agent can be exemplified by the following, although this is not a particular limitation: camptothecin derivatives such as irinotecan hydrochloride, nogitecan hydrochloride, and exatecan; taxane derivatives such as docetaxel hydrate, docetaxel, and paclitaxel; as well as ifosfamide, nimustine hydrochloride, carboquone, cyclophosphamide, dacarbazine, thiotepa, busulfan, melphalan, ranimustine, estramustine phosphate sodium, 6-mercaptopurine riboside, enocitabine, gemcitabine hydrochloride, carmofur, cytarabine, cytarabine ocfosfate, tegafur, doxifluridine, hydroxycarbamide, fluorouracil, methotrexate, mercaptopurine, fludarabine phosphate, actinomycin D, aclarubicin hydrochloride, idarubicin hydrochloride, pirarubicin hydrochloride, epirubicin hydrochloride, daunorubicin hydrochloride, doxorubicin hydrochloride, epirubicin, pirarubicin, daunorubicin, doxorubicin, bleomycin hydrochloride, zinostatin stimalamer, neocarzinostatin, mitomycin C, bleomycin sulfate, puromycin sulfate, etoposide, vinorelbine tartrate, vincristine sulfate, vindesine sulfate, vinblastine sulfate, amrubicin hydrochloride, gefinitib, exemestane, and capecitabin. In an embodiment, the anticancer agent contains doxorubicin. The compound in the anticancer agent may be a freely selected salt.

In a particular embodiment, the antimicrobial can be exemplified by the following, although this is not a particular limitation: antifungal triazole derivatives (fluconazole, itraconazole, ketoconazole, miconazole); antibacterial cephalosporins (cefazolin, cefonicid, cefotaxime, ceftazimide, cefoxime); antibacterial β-lactam derivatives (aztreopam, cefmetazole, cefoxitin); erythromycin group antibacterials (erythromycin, azithromycin, clarithromycin, oleandomycin); penicillins (benzylpenicillin, phenoxymethylpenicillin, cloxacillin, methicillin, nafcillin, oxacillin, carbenicillin); and tetracyclines. The compound in these antimicrobials may be a freely selected salt.

With reference to the total weight of the lipid-based particle carrier, the lipid-based particle carrier according to the present embodiment contains the drug at, for example, 0.01 to 50 weight%, for example, 0.1 to 30 weight%, for example, 1 to 10 weight%.

The "average particle diameter" of the lipid-based particle according to the present embodiment (also referred to hereafter as the "average particle diameter" or the "particle diameter") can be calculated by any method selected from volume average, number average, and Z-average, but the "average particle diameter" of the lipid-based particle according to the present invention refers to the Z-average particle diameter. The average particle diameter (Z-average particle diameter) of the lipid-based particle of the present embodiment may be, for example, 30 to 200 nm, for example, 70 to 150 nm.

The lipid-based particle according to embodiments exhibits an excellent stability by the particle structure (particularly the stability in the organism), an excellent drug encapsulation capability, and/or an excellent ability to control drug release.

The ICG derivative lipid according to the hereinabove-described embodiments has an ICG skeleton that is modified by groups containing a hydrocarbon chain ($R_1$ and $R_2$) and can form a stable particle structure. For example, the lipid-based particle according to embodiments forms a liposome that exhibits an excellent stability within the organism (particularly in the blood).

The ICG derivative lipid according to the hereinabove-described embodiments has an ICG skeleton that is modified by groups containing a hydrocarbon chain ($R_1$ and $R_2$) and can support a drug in a stable manner. Accordingly, the lipid-based particle according to embodiments can form a particle structure in which a drug is encapsulated in a stable manner. The lipid-based particle according to embodiments forms, for example, a drug-encapsulating liposome that exhibits an excellent stability in the organism (particularly in the blood) and/or exhibits an excellent drug encapsulation ratio. In addition, a lipid-based particle (particularly a liposome) constructed of the ICG lipid derivative according to the present invention can, upon exposure to near-infrared light, selectively release the encapsulated drug to the outside of the lipid-based particle. Investigations by the present inventors showed that, when the modifying group contains a polar group such as the sulfo group, stable support of a drug is impeded and control of drug encapsulation and release is problematic.

The lipid-based particle according to the present invention provides an ICG-derived near-infrared fluorescence capability because it contains the ICG derivative lipid according to the hereinabove-described embodiments. The lipid-based particle according to embodiments emits fluorescence upon irradiation with excitation light (for example, near-infrared light).

The lipid-based particle according to the present invention, because it contains the ICG derivative lipid according

to the hereinabove-described embodiments, upon irradiation with near-infrared light provides a heat-generating action (heating effect, HT effect) and/or an active oxygen-generating action (photodynamic effect, PDT effect). In some aspects, a lipid-based particle containing the ICG lipid derivative according to the present invention functions as a temperature-sensitive liposome.

(Methods for producing the lipid-based particles)

The lipid-based particle carrier according to embodiments can be produced using any methods known in the concerned technical field.

Methods for producing the lipid-based particle carrier are described in the following using the example of a liposome for the lipid-based particle. For example, a production method according to an embodiment comprises (1) a step of obtaining a liposome-containing liposome dispersion, and optionally (2) a step of mixing a drug with the liposome dispersion to encapsulate the drug in the liposomes in the liposome dispersion.

(1) The step of obtaining a liposome-containing liposome dispersion

For example, the step of obtaining a liposome-containing liposome dispersion preferably comprises (i) a step of producing a liposome from at least one species of lipid that includes the ICG lipid derivative according to the hereinabove-described embodiments, and a (ii) step of replacing or diluting the liposome external phase.

Step (i)

The liposome preparation method is not particularly limited and can be exemplified by the lipid film method (vortex method), reverse phase evaporation method, ultrasonic method, prevesicle method, ethanol injection method, French Press method, cholate depletion method, Triton X-100 batch method, Ca2+ fusion method, ether injection method, annealing method, and freeze-thaw fusion method. The various conditions in liposome preparation can be established as appropriate in correspondence to, e.g., the liposome preparation method, the target liposome composition, the particle diameter, and so forth.

Liposome preparation by the following procedure is provided as an example.

First, one or more lipids containing the ICG lipid derivative is dissolved in a suitable solvent (for example, chloroform) to prepare a lipid solution.

The organic solvent is then removed from the lipid solution (preferably removal under reduced pressure) to form a lipid thin film.

An aqueous solvent is subsequently added to the lipid thin film to effect hydration, and liposome formation is performed by stirring using, for example, a vortex mixer. The aqueous solution can be exemplified by buffers such as HEPES buffers, phosphate buffers, citrate buffers, phosphate-buffered physiological saline and so forth, and by physiological saline. Hydration is preferably carried out while heating to bring the temperature to at least the phase transition temperature of the lipid bilayer of the liposome. When a monolamellar liposome is to be produced, an ultrasound treatment is preferably carried out as necessary after the stirring with, for example, a vortex mixer.

Step (ii)

A liposome dispersion can be obtained by replacing or diluting the external phase for the obtained liposome. The replacement or dilution of the liposome external phase may be carried out a single time or may be carried out a plurality of times and various types of replacement or dilution methods may be combined. The method for replacing the liposome external phase of the liposome preparation solution can be exemplified by dialysis, centrifugal separation, gel filtration, and so forth.

The solvent (dispersion medium) used in replacement and/or dilution of the liposome external phase can be exemplified by buffers such as HEPES buffers, phosphate buffers, citrate buffers, phosphate-buffered physiological saline and so forth, and by physiological saline. The pH of this solvent is not particularly limited, but is preferably 3 to 11, more preferably 4 to 10, and still more preferably 5 to 10.

The liposome particle diameter can be freely adjusted as necessary. For example, the particle diameter can be adjusted by carrying out extrusion under high pressure (extrusion filtration) using a membrane filter having a uniform pore size. Adjustment of the particle diameter can be carried out at any time duing liposome production; for example, it may be carried out prior to replacement or dilution of the liposome external phase, after replacement or dilution of the liposome external phase, or after introduction of a drug into the liposome internal phase. Particle diameter adjustment is preferably carried out prior to the introduction of a drug into the liposome internal phase and is more preferably carried out prior to replacement or dilution of the liposome external phase.

In an embodiment in which a drug is encapsulated in the liposome, in step (ii) an ion gradient and/or a pH gradient is preferably introduced between the interior phase and external phase of the liposome (remote loading method). Accordingly, in some embodiments, step (ii) is a step in which the liposome external phase is replaced to obtain a liposome dispersion containing a liposome that has an ion gradient and/or a pH gradient between the internal phase and the external phase. The production method of the present embodiments yields a drug-encapsulating liposome that exhibits an excellent drug encapsulation ratio and an excellent stability.

The pH gradient method is a technology in which a compound is imported into a liposome utilizing a pH-mediated shift in the molecular form/ionic form dissociation equilibrium of the drug.

When a pH gradient is to be introduced, the difference in the pH of the liposome internal phase and liposome external phase in the liposome dispersion is preferably 1 to 5 and more preferably 2 to 3. Depending on the type of drug, the pH of either the liposome internal phase or external phase may also be increased. On the other hand, the pH of the liposome internal phase and the liposome external phase may be substantially the same. The pH gradient can be adjusted using compounds heretofore known for use in the pH gradient method, for example, amino acids such as arginine, histidine, and glycine; acids such as ascorbic acid, benzoic acid, citric acid, glutamic acid, phosphoric acid, acetic acid, propionic acid, tartaric acid, carbonic acid, lactic acid, boric acid, maleic acid, fumaric acid, malic acid, adipic acid, hydrochloric acid, and sulfuric acid; salts of these acids, such as the sodium salts, potassium salts, and ammonium salts; and alkaline compounds such as trishydroxymethylaminomethane, aqueous ammonia, sodium hydroxide, potassium hydroxide, and sodium bicarbonate.

Doxorubicin (DOX, pKa: 8.2) is an example of a compound that can be encapsulated in a liposome using the pH gradient method. For example, a liposome dispersion can be prepared using a buffer with a pH of approximately 4 (for example, a citrate buffer), and the liposome external phase can then be replaced with a buffer having a pH of approximately 7 (for example, a HEPES buffer) to obtain a liposome dispersion that contains liposomes having a pH gradient between the internal phase and the external phase. When DOX is added to this liposome dispersion in the step (2) described in the following, the molecular-form DOX in the pH 7 external phase, due to its being lipid soluble, transfers into the liposome membrane rather than being in the aqueous phase. In addition, when the DOX that has transferred into the liposome membrane comes into contact with the pH 4 liposome internal phase, the DOX assumes its ionic form and dissolves into the liposome internal phase. The DOX can thus be transported from the liposome external phase to the internal phase through a shift in the dissociation equilibrium.

The ion gradient method is a technology that brings a drug into the liposome internal phase utilizing an ion gradient that is formed between the internal phase/external phase of the liposome.

The ion used in the ion gradient method is not particularly limited and can be exemplified by ammonium sulfate, ammonium chloride, ammonium borate, ammonium formate, ammonium acetate, ammonium citrate, ammonium tartrate, ammonium succinate, and ammonium phosphate. In addition, the ion concentration of the liposome internal phase in the ion gradient method can be selected as appropriate in correspondence to the drug species; however, higher is better and it is preferably at least 10 mM, more preferably at least 20 mM, and still more preferably at least 50 mM. The ion concentration in either the liposome internal phase or external phase can also be increased depending on the drug species. On the other hand, there may not be a substantial difference in the ion concentrations of the liposome internal phase and the liposome external phase, i.e., the ion concentrations of the liposome external phase and the liposome internal phase may be substantially the same. The ion gradient may also be adjusted by replacing or diluting the liposome external phase.

An ion gradient with ammonium sulfate is preferably used rather than the pH gradient in the pH gradient method. Accordingly, in some embodiments step (ii) is a step in which the liposome external phase is replaced to yield a liposome dispersion that contains a liposome that has, between the internal phase and the external phase, an ammonium sulfate ion gradient and/or a pH gradient. In a particular embodiment, step (ii) is a step in which the liposome external phase is replaced to obtain a liposome dispersion that contains a liposome that has an ammonium sulfate ion gradient and a pH gradient between the internal phase and the external phase. A drug can be efficiently encapsulated in the liposome internal phase using this embodiment.

In specific terms, when an ion gradient of ammonium sulfate is to be introduced, an aqueous ammonium sulfate solution may be used as the aqueous medium used for hydration in the aforementioned step (i) and, for example, physiological saline or a buffer such as a HEPES buffer, phosphate buffer, citrate buffer, or phosphate-buffered physiological saline may be used in step (ii).

The lipid concentration in the liposome dispersion is not particularly limited, but is preferably 1 to 100 mM and is more preferably 1 to 50 mM. Within these ranges, a larger number of liposome particles can be suitably formed without impairing the physical properties of the liposome dispersion.

(2) The step of mixing a drug with the liposome dispersion to encapsulate the drug in the liposomes in the liposome dispersion

A drug is encapsulated in the liposomes (preferably the liposome internal phase) in the liposome dispersion by

mixing the drug with the obtained liposome dispersion. A drug-encapsulating liposome can be obtained as a result.

This preferably comprises a step of mixing the drug with the liposome dispersion and subsequently raising the membrane permeability of the liposomes in the drug + liposome dispersion mixture. This makes it possible to carry out encapsulation of the drug into the liposome in a shorter period of time. Methods for increasing the membrane permeability of the liposome in the mixture can be exemplified by methods in which the mixture is heated, methods in which a membrane fluidizing agent is added to the mixture, and so forth.

When the mixture is to be heated, the drug can generally be introduced into the liposome internal phase more efficiently by raising the temperature to a higher temperature. The target temperature is not particularly limited, and the target temperature is preferably established based on a consideration of the thermal stability of the drug and the constituent components used for the liposome membrane. For example, it is preferably equal to or greater than room temperature and is more preferably equal to or greater than the phase transition temperature of the lipid bilayer of the liposome.

3. The composition

An additional aspect of the present invention provides a composition that contains the hereinabove-described lipid-based particle.

The composition according to some embodiments comprises the hereinabove-described lipid-based particle and, depending on the circumstances, a pharmaceutically acceptable medium and other additives.

The pharmaceutically acceptable medium can be exemplified by sterilized water; physiological saline; isotonic solutions that contain an auxiliary reagent, e.g., glucose, D-sorbitol, D-mannose, D-mannitol, sodium chloride, and so forth; and buffers such as phosphate buffers, citrate buffers, acetate buffers, and so forth.

In some embodiments the pharmaceutically acceptable medium is an aqueous solvent.

The other additives may include, for example, solubilizing agents, e.g., an alcohol such as ethanol, propylene glycol, polyethylene glycol, and so forth; stabilizers; antioxidants; antiseptics; excipients as generally used in drug production; fillers; extenders; binders; wetting agents; disintegrants; lubricants; surfactants; dispersants; preservatives; taste- and/or odor-masking agents; analgesics; and so forth. The other additives may also include, for example, sugars such as sucrose, glucose, sorbitol, lactose, and so forth; amino acids such as glutamine, glutamic acid, sodium glutamate, histidine, and so forth; and the salts of an acid such as citric acid, phosphoric acid, acetic acid, lactic acid, carbonic acid, tartaric acid, and so forth.

The composition includes solid forms and liquid forms. When the composition takes a solid form, it can be made into a liquid form by dissolution or suspension in a predetermined pharmaceutically acceptable medium as described in the following. In addition, in the case of a solid provided by freezing the composition, it can be made into a liquid form by melting, for example, by standing at room temperature. In some embodiments, the composition, for example, may have a powder form provided by removal of a solvent by, for example, freeze drying, or may have a liquid form. The composition according to an embodiment of the present invention is a powder composition that contains the lipid-based particle according to the hereinabove-described embodiments. The powder composition may be produced by removing the solvent from the liquid composition (dispersion) by, for example, filtration, centrifugation separation, and the like, or may be produced by freeze-drying the dispersion.

The composition according to the present invention can be, for example, a pharmaceutical composition or a diagnostic composition.

The composition according to the present invention may be formulated as a pharmaceutical composition. An injectable is an example of a pharmaceutical composition formulation.

In addition, the composition according to the present invention can be used as a diagnostic composition. The term "diagnostic composition" denotes a composition that is administered to a subject in order to carry out diagnosis (in vivo diagnosis or in vitro diagnosis and particularly in vivo diagnosis). The formulation of the diagnostic composition is not particularly limited with injectables being an example.

The pharmaceutical composition or diagnostic composition according to an embodiment of the present invention is a liquid composition that contains lipid-based particles according to a hereinabove-described embodiment as well as a pharmaceutically acceptable medium and other additives. When the composition is in liquid form, it can be used as an injectable as such or suspended or dissolved in a pharmaceutically acceptable medium.

The pharmaceutical composition or diagnostic composition according to an embodiment of the present invention is a powder composition that contains lipid-based particles according to a hereinabove-described embodiment as well as other additives. When the pharmaceutical composition is in powder form, it can be used as an injectable by suspension or dissolution in a pharmaceutically acceptable medium prior to use.

Administration of the composition, pharmaceutical composition, or diagnostic composition to a subject can be carried out, for example, by a parenteral method, e.g., intraarterial injection, intravenous injection, subcutaneous injection, and so forth. The dosage will also vary depending on the target of the administration, the target organ, the symptoms, and

the method of administration. The target of the administration is not limited and application to various animals can be carried out, and in particular application to mammals and preferably humans as well as to clinical testing, screening, and experimental animals in experiments can be carried out.

4. Applications

The ICG lipid derivative, lipid-based particle, and lipid-based particle-containing composition according to the hereinabove-described embodiments can be used in a variety of applications.

For example, the ICG lipid derivative, lipid-based particle, and lipid-based particle-containing composition (pharmaceutical composition, diagnostic composition) according to the hereinabove-described embodiments can be used in at least one selection from photodynamic hyperthermal therapy, photodynamic therapy, and fluorescence imaging. In some embodiments there is provided a photodynamic hyperthermal therapy method, photodynamic therapy method, or fluorescence imaging method comprising the administration to a subject of a lipid composite or lipid-based particle-containing composition (pharmaceutical composition or diagnostic composition) according to the hereinabove-described embodiments. In some embodiments there is provided the use of a lipid composite or lipid-based particle-containing composition (pharmaceutical composition or diagnostic composition) according to the hereinabove-described embodiments, for use in at least one selection from photodynamic hyperthermal therapy, photodynamic therapy, and fluorescence imaging.

(Fluorescence imaging)

In some embodiments, the lipid-based particle-containing composition according to the hereinabove-described embodiments is used in fluorescence imaging. The lipid-based particle according to the hereinabove-described embodiments, because it incorporates the ICG lipid derivative, can emit fluorescence upon the absorption of near-infrared light and can be used as a probe (contrast agent) for fluorescence imaging. The lipid-based particle is a probe for fluorescence imaging according to an embodiment. The fluorescence imaging method according to an embodiment is characterized by the use of the lipid-based particle according to the hereinabove-described embodiments. The imaging method of the present embodiment is useful as a noninvasive identification method that can bring about visualization while reducing or preventing damage to the tissue of the organism.

The fluorescence imaging method according to an embodiment typically comprises a step of administering, to a subject, a lipid-based particle (ICG lipid derivative)-containing composition (probe) according to the hereinabove-described embodiments; and exposing the subject to excitation light (for example, near-infrared light) from an excitation light source and then detecting fluorescence emitted from the lipid-based particle (ICG lipid derivative) due to this excitation light. The excitation wavelength will vary depending on the ICG lipid derivative contained in the lipid-based particle and is not limited as long as it is in a range where the lipid-based particle (ICG lipid derivative) effectively emits fluorescence in the near-infrared region. The excitation light is, for example, near-infrared light. The fluorescence imaging is carried out based on known methods, and the individual parameters, such as the excitation wavelength and the fluorescence wavelength to be detected, can be established as appropriate in correspondence to the target of the administration and the type of ICG lipid derivative contained in the lipid-based particle.

The subject or target for this fluorescence imaging method is not limited, and it can be applied to various animals and in particular can be applied to mammals and preferably humans and to clinical testing, screening, and experimental animals in experiments. This may be in vivo or in vitro.

An embodiment is a fluorescence imaging method in order to identify sentinel lymph nodes.

In some embodiments, the lipid-based particle-containing composition according to the hereinabove-described embodiments is used as a diagnostic composition in the aforementioned fluorescence imaging method. The diagnostic composition can be used for diagnosis, or to assist diagnosis, in animals, or for the detection of predetermined cells or tissue.

(Photodynamic hyperthermal therapy · photodynamic therapy)

In some embodiments, the lipid-based particle-containing pharmaceutical composition of the hereinabove-described embodiments is used in photodynamic hyperthermal therapy and/or photodynamic therapy. The photodynamic hyperthermal therapy and/or photodynamic therapy according to an embodiment is characterized by the use of the lipid-based particle according to the hereinabove-described embodiments. The photodynamic hyperthermal therapy and/or photodynamic therapy typically comprises administering (for example, intravenous administration or intratumoral administration), to a subject, the lipid-based particle-containing pharmaceutical composition according to the hereinabove-described embodiments, and, after a certain period of time, irradiating the subject (diseased portion of the subject) with near-infrared light. The photodynamic hyperthermal therapy and/or photodynamic therapy is carried out based on known

methods. The various parameters, such as the wavelength region of the near-infrared light, the irradiation time, and the lipid-based particle concentration, are established as appropriate depending on the type of ICG lipid derivative contained in the lipid-based particle, the subject or target for the administration, and the desired therapeutic effect.

The photodynamic hyperthermal therapy and/or photodynamic therapy according to an embodiment exhibits therapeutic efficacy against various tumors, for example, brain tumors, insulinomas, nasal cancers, oral cancers, kidney cancers, lung cancers, colorectal cancers, soft tissue sarcomas, metastatic cancers (pleural metastases, peritoneal metastases), and so forth.

In some embodiments, the lipid-based particle-containing pharmaceutical composition according to the hereinabove-described embodiments is used for the treatment of cancer.

(Drug delivery and related uses)

The lipid-based particle of the hereinabove-described embodiments can be used as a means for selectively and efficiently introducing a desired encapsulated drug to a target site. For example, the composition according to the present invention can be used in therapies in which a desired drug is transported to a target site. Thus, an embodiment of the present invention provides a method for treating various diseases using a pharmaceutical composition comprising drug-containing lipid-based particles as described in the preceding. Another embodiment provides the use of the lipid-based particle according to the hereinabove-described embodiments in the production of drugs that treat various diseases. Some embodiments provide a method for transporting a drug to a target, comprising administering to a subject a pharmaceutical composition that contains a drug-containing lipid-based particle according to the hereinabove-described embodiments.

Another embodiment provides a method for releasing, or a method for controlling the release, at a target site of a desired drug that is supported by a lipid-based particle. The lipid-based particle according to the embodiment releases the encapsulated drug to the outside of the lipid-based particle (for example, a liposome) accompanying an increase in the temperature of the lipid-based particle when the ICG lipid derivative contained in the lipid-based particle generates heat due to exposure to near-infrared light. Selective drug release at a target site is thus made possible by exposing the target site to near-infrared light. In an embodiment, the liposome is a temperature-sensitive liposome. A temperature-sensitive liposome can efficiently and rapidly release a drug at a target site.

(Cancer treatment)

In some embodiments, the lipid-based particle-containing pharmaceutical composition of the hereinabove-described embodiments is used to treat cancer. There is provided in some embodiments a method for treating cancer comprising administering, to a subject or target, a lipid composite or lipid-based particle-containing pharmaceutical composition according to the hereinabove-described embodiments. Some embodiments provide use of a lipid composite or lipid-based particle-containing pharmaceutical composition according to the hereinabove-described embodiments for use in the treatment of cancer. In a particular embodiment, the lipid-based particle contains an anticancer agent as a drug.

It has already been confirmed, in cancer-bearing animal experiments and in human patients, that liposomes (particularly liposomes with a particle diameter of not more than 200 nm) readily accumulate in solid tumor tissue due to a so-called enhanced permeability and retention (EPR) effect. The ICG lipid derivative-containing liposome according to the hereinabove-described embodiments is advantageously used for cancer treatment because it provides a combination of the following functions in vivo: a cancer tissue imaging function based on the infrared fluorescence capability of the ICG lipid derivative, a thermal cancer treatment function based on the heat-generating effect of the ICG lipid derivative, and, as a result of the encapsulation of an anticancer drug, the function of an anticancer agent-incorporating temperature-sensitive liposome (a liposome that selectively releases an anticancer agent accompanying an increase in temperature).

5. Kit

An embodiment of the present invention is a kit for drug delivery or imaging, that contains a lipid-based particle as described in the preceding. This kit can be used in various treatments or diagnoses. The storage condition of the lipid-based particles in the kit according to this embodiment is not particularly limited and any condition may be adopted, e.g., solution form or powder form, considering, e.g., the corresponding stability (storability) and ease of use, and so forth. Aside from the lipid-based particles as described in the preceding, the kit according to the embodiment may contain, for example, various media (pharmaceutically acceptable media, buffers), various drugs, and use instructions (use manual). The kit according to the present embodiment is used to produce a composition containing a desired lipid-based particle for introduction to a target site. For example, lipid-based particles according to an embodiment and various media may be made into a kit, which may be used by dispersing the particles in the various media prior to administration into an organism.

Examples

The present invention is described further through the examples, production examples, and test examples provided herebelow, but the present invention is not limited to or by these examples.

In this Description, "room temperature" generally indicates approximately 10°C to approximately 35°C. "%" indicates weight percent unless specifically indicated otherwise.

The starting materials, reagents, acids, bases, drying agents, solvents, and catalysts used for synthesis of the compounds of the present invention in all instances can be purchased or can be produced by organic synthesis methods known to the person skilled in the art. In addition, the compounds of the present invention can be produced by organic synthesis methods known to the person skilled in the art, as shown in the following examples.

Mass spectrometry was carried out using a XevoTM G2-XS from the Waters Corporation.

The names generated by "ChemDraw Professional" Version 15.1.0.144 (PerkinElmer Inc.) were used for the names of the compounds shown below, except for those reagents in general use.

The abbreviations used in the examples are the common abbreviations well known to the person skilled in the art. Some abbreviations are given below.

| | |
|---|---|
| $Ac_2O$ | acetic anhydride |
| AcONa | sodium acetate |
| Br | bromo |
| DCM | dichloromethane |
| DIPEA | N,N-diisopropylethylamine |
| DMF | dimethylformamide |
| DOPE | dioleoylphosphatidylethanolamine |
| DPPC | dipalmitoylphosphatidyl |
| DSPC | distearoylphosphatidylcholine |
| DSPE | 1,2-distearoyl-sn-glycero-3-phosphoethanolamine) |
| HBTU | 1-[bis(dimethylamino)methylene]-1H-benzotriazolium 3-oxide hexafluorophosphate |
| MeOH | methanol |
| MPEG | methoxypolyethylene glycol |
| MPEG2000-DSPE | (methoxypoly(ethylene glycol)2000)-1,2-distearoyl-sn-glycero-3-phosphoethanolamine |
| SA | stearylamine |
| o-DCB | 1,2-dichlorobenzene |
| PEG | polyethylene glycol |
| reflux | reflux |

A. Synthesis of ICG lipid derivatives

[Production Example 1]

Synthesis of ICG dicarboxylic acid (compound 5)

Compound 5: 3-(2-((1E,3E,5E,7E)-7-(3-(2-carboxyethyl)-1,1-dimethyl-1,3-dihydro-2H-benzo[e]indol-2-ylidene)hepta-1,3,5-trien-1-yl)-1,1-dimethyl-1H-benzo[e]indol-3-ium)propanoate

Compound 5 was synthesized according to scheme below.

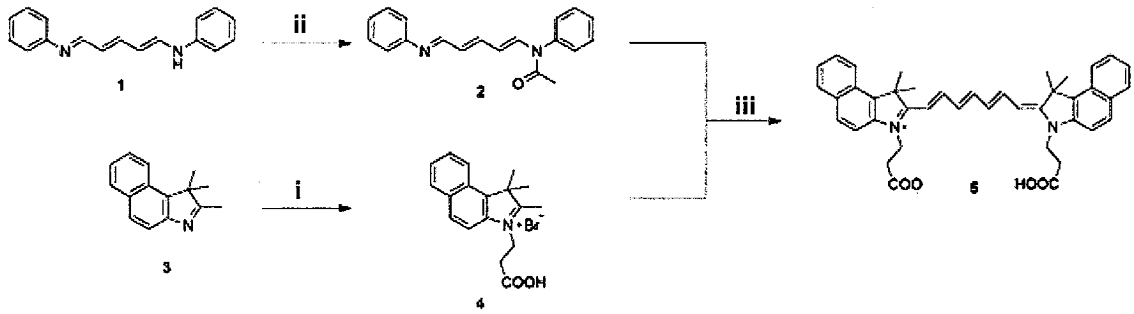


i. $BrCH_2CH_2COOH$/o-DCB/110°C; ii. $Ac_2O$/DIPEA/DCM; iii. MeOH/AcONa/reflux

(Step i)

1,1,2-trimethyl[1H]benz[e]indole (10.0 g, compound 3) and 3-bromopropionic acid (7.3 g) were introduced and dissolved in 50 ml of 1,2-dichlorobenzene and stirring was performed for 18 hours at 110°C. After cooling to room temperature, the precipitated solid was filtered off and washed with dichloromethane. Filtration followed by drying under reduced pressure yielded 13.9 g of 3-(2-carboxyethyl)-1,1,2-trimethyl-1H-benz[e]indol-3-ium bromide (compound 4).

(Step ii)

Glutaconaldehyde dianilide hydrochloride (2.84 g, compound 1), N,N-diisopropylethylamine (2.60 g), and 20 ml of dichloromethane were introduced; a dichloromethane (5 ml) solution of acetic anhydride (1.20 g) was added dropwise with ice cooling; and stirring was carried out for 3 hours and the solvent was distilled off.

(Step iii)

The residue from step ii was dissolved in 5 ml of methanol and was added dropwise while under reflux into a methanol solution (50 ml) of compound 4 (10.0 g) and sodium acetate (3.9 g, 47.54 mmol). After the dropwise addition, a reaction was run for 16 hours under reflux and the solvent was then distilled off. The residue was washed with ethyl acetate, 5% hydrochloric acid, and ethyl acetate in the indicated sequence, and the resulting mixture was crystallized with acetonitrile/water (3/7) to obtain 4.3 g of compound 5.

[Example A1]

ICG-diSA: 2-((1E,3E,5E,7E)-7-(1,1-dimethyl-3-(3-(octadecylamino)-3-oxopropyl)-1,3-dihydro-2H-benzo[e]indol-2-ylidene)hepta-1,3,5-trien-1-yl)-1,1-dimethyl-3-(3-(octadecylamino)-3-oxopropyl)-1H-benzo[e]indol-3-ium

The ICG lipid derivative (ICG-diSA) was synthesized according to scheme below from the compound 5 obtained in Production Example 1.

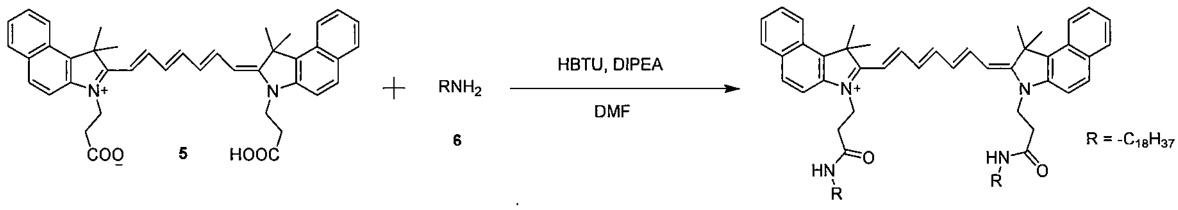


ICG-diSA

A mixture of compound 5 (502 mg), dimethylformamide (85 ml), stearylamine (1.01 g, compound 6), 1-[bis(dimethylamino)methylene]-1H-benzotriazolium 3-oxide hexafluorophosphate (2.71 g), and N,N-diisopropylethylamine (413 mg) was stirred for 3 hours at room temperature. The solvent was distilled off, followed by dissolution in ethyl acetate (130 ml); washing was carried out twice with 50 ml of a saturated aqueous sodium bicarbonate solution and twice with 50 ml of saturated aqueous sodium chloride, and the organic layer was dried over anhydrous magnesium sulfate. The solvent was distilled off, followed by purification with silica gel (Cosmosil 75$C_{18}$OPN (30 g)) using 0.1% trifluoroacetic acid/methanol (100/0 to 0/100) and additionally with silica gel (Cosmosil 75$C_{18}$OPN (40 g) using methanol/water (75/25 to 92/8)) to obtain ICG-diSA (222 mg). The $^1$H NMR chart of ICG-diSA is given in Fig. 1.

[Examples A2 and A3]

ICG-diDOPE:

2-(3-(2-((1E,3E,5E,7E)-7-(3-(3-((2-(((2,3-bis(oleoyloxy)propoxy)(hydroxy)phosphoryl)oxy) ethyl)amino)-3-oxopropyl)-1,1-dimethyl-1,3-dihydro-2H-benzo[e]indol-2-ylidene)hepta-1,3,5-trien-1-yl)-1,1-dimethyl-1H-benzo[e]indol-3-ium-3-yl)propanamide)ethyl(2,3-bis(oleoyloxy)propyl) phosphate

ICG-diDSPE:

2-(3-(2-((1E,3E,5E,7E)-7-(3-(3-((2-(((2,3-bis(stearoyloxy)propoxy)(hydroxy)phosphoryl)oxy)ethyl)amino)-3-oxopropyl)-1,1-dimethyl-1,3-dihydro-2H-benzo[e]indol-2-ylidene)hepta-1,3,5-trien-1-yl)-1,1-dimethyl-1H-benzo[e]indol-3-ium-3-yl)propanamide)ethyl (2,3-bis(stearoyloxy)propyl) phosphate

The ICG lipid derivatives ICG-diDOPE and ICG-diDSPE were synthesized according to scheme below from the compound 5 obtained in Production Example 1.

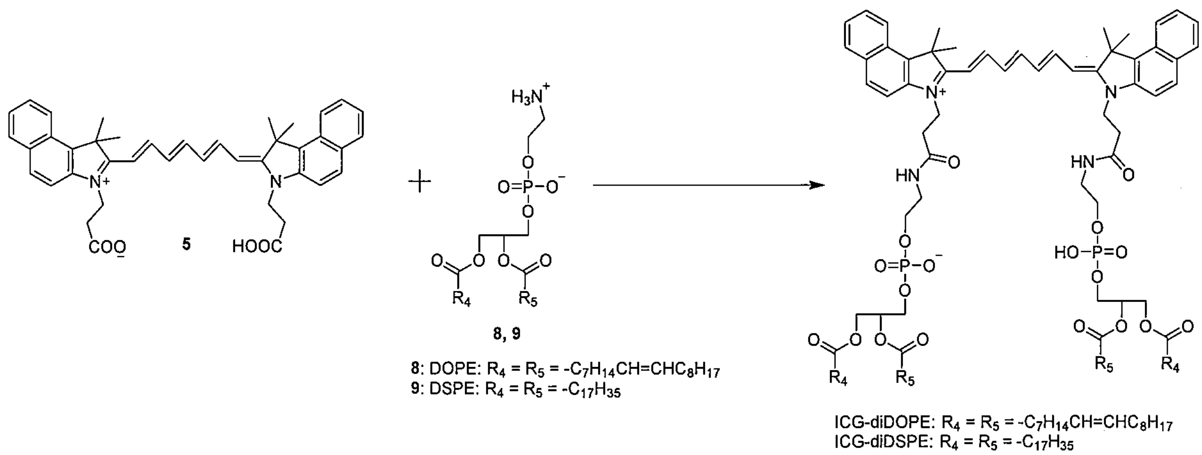

5

8, 9

8: DOPE: $R_4 = R_5 = -C_7H_{14}CH{=}CHC_8H_{17}$
9: DSPE: $R_4 = R_5 = -C_{17}H_{35}$

ICG-diDOPE: $R_4 = R_5 = -C_7H_{14}CH{=}CHC_8H_{17}$
ICG-diDSPE: $R_4 = R_5 = -C_{17}H_{35}$

(1) ICG-diDOPE

A dichloromethane (3 ml) solution of N,N'-dicyclohexylcarbodiimide (511 mg) was added dropwise at room temperature with stirring to a mixture of compound 5 (501 mg), dichloromethane (15 ml), N-hydroxysuccinimide (278 mg), and acetonitrile (3 ml). Filtration was carried out and the solvent was then distilled off and recrystallization was carried out with ethyl acetate (20 ml). The resulting solid was dissolved in dichloromethane (20 ml), 1,2-dioleoyl-sn-glycero-3-phosphoethanolamine (DOPE, 738 mg, compound 8) and triethylamine (0.22 g) were added, and stirring was carried out overnight at room temperature. The solvent was distilled off, followed by purification with silica gel (Cosmosil $75C_{18}$OPN (10 g), methanol/water (50/50 to 100/0), methanol/chloroform (90/10 to 80/20)) and additionally with silica gel (Cosmosil $75C_{18}$OPN (40 g), chloroform/methanol (100/0 to 75/25)) to obtain ICG-diDOPE (97 mg). The $^1$H NMR chart of ICG-diDOPE is given in Fig. 2.

(2) ICG-diDSPE

Compound 5 (1.01 g, 1.60 mmol), 1,2-distearoyl-sn-glycero-3-phosphoethanolamine (DSPE, 2.50 g, compound 9), chloroform (170 ml), 1-[bis(dimethylamino)methylene]-1H-benzotriazolium 3-oxide hexafluorophosphate (2.43 g), and N,N-diisopropylethylamine (435 mg) were combined and stirring was carried out overnight at room temperature. Filtration was carried out and the solvent was subsequently distilled off, followed by purification with silica gel (Cosmosil $75C_{18}$OPN (120 g), chloroform/methanol (100/0 to 90/10)) to obtain ICG-diDSPE (299 mg).
MS (ESI) m/z: 2082.4 $[M - H]^-$
The $^1$H NMR chart of ICG-diDSPE is given in Fig. 3.

[Comparative Example A1]

ICG-DOPE: 4-(2-((1E,3E,5E,7E)-7-(3-(3-((2-(((2,3-bis(oleoyloxy)propoxy)oxidophosphoryl)oxy)ethyl)amino)-3-oxopropyl)-1,1-dimethyl-1,3-dihyro-2H-benzo[e]indol-2-ylidene)hepta-1,3,5-trien-1-yl)-1,1-dimethyl-1H-benzo[e]indol-3-ium-3-yl)butane-1-sulfonate

ICG-DOPE was synthesized based on scheme below with reference to the method described in [0050] to [0053] and [0068] in WO 2011/152046. The $^1$H NMR chart of ICG-DOPE is given in Fig. 4.

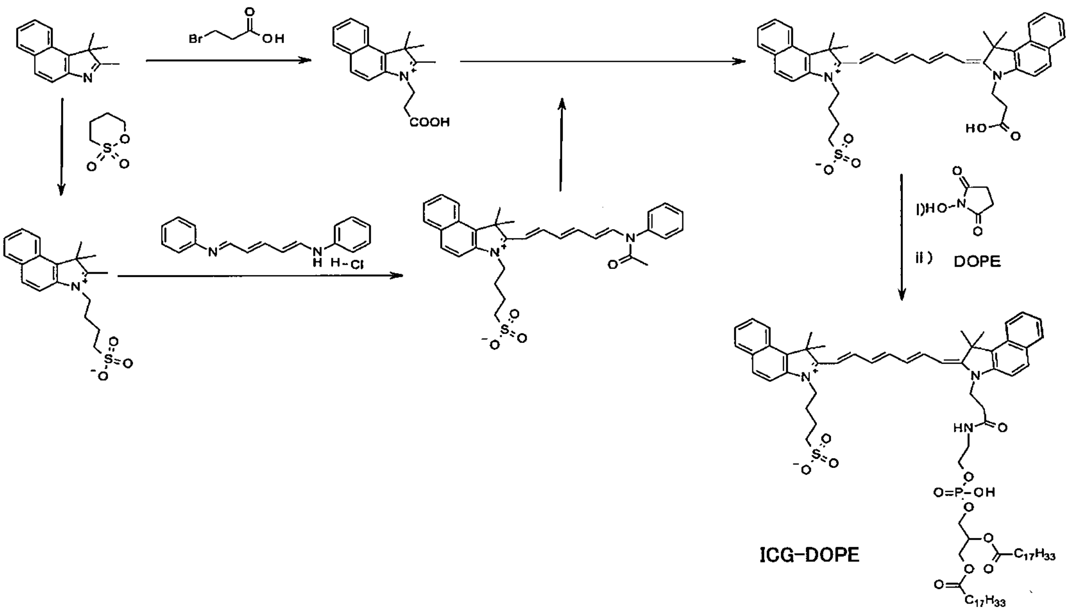


ICG-DOPE

< Calculation of the molar extinction coefficient >

The molar extinction coefficients of ICG and the ICG lipid derivatives synthesized in Examples A1 to A3 and Comparative Example A1 were calculated using the following method.

An ICG lipid solution, prepared by the dissolution of the particular ICG lipid derivative or ICG in ethanol to provide 5 μM in each case, was introduced into a cuvette with an optical path length of 1 cm, and the absorption spectrum at 230 to 1000 nm was measured using an Infinite M200 (Tecan). The absorption spectra of the individual ICG lipid derivatives are given in Fig. 5. The absorption spectra in Fig. 5 were provided by subtracting the absorption spectra of ethanol from the absorbance at each wavelength.

All of the ICG lipids had a maximum absorption wavelength of 790 nm in their absorption spectrum. The molar extinction coefficient ε (L/mol/cm) was calculated from the absorbance (the absorbance of ethanol was subtracted) at 790 nm, which was the maximum absorption wavelength. The molar concentration used in the calculation of the molar extinction coefficient was itself calculated using a purity of 100% for the ICG lipid derivative and using the nonsalt form. The molar extinction coefficients of the individual ICG lipid derivatives are given in Table A.

[Table A]

| Table A. Molar extinction coefficients ε of ICG lipid derivatives | | | | | |
|---|---|---|---|---|---|
| | ICG | Comparative Example A1 | Example A1 | Example A2 | Example A3 |
| | | ICG-DOPE | ICG-diSA | ICG-diDOPE | ICG-diDSPE |
| molar extinction coefficient ε [L/mol/cm] | 208980 | 165860 | 177760 | 161100 | 199840 |

As shown in Fig. 5, all of the ICG lipid derivatives exhibited absorption in the near-infrared region of 700 to 900 nm. In addition, all of the ICG lipid derivatives also had a molar extinction coefficient of at least $1.6 \times 10^5$ [L/mol/cm]. The ICG lipid derivatives according to the present invention were confirmed to have excellent absorption characteristics for near-infrared light.

Because, as shown in the preceding, the ICG lipid derivatives according to the present invention exhibit the same near-infrared absorption characteristics as ICG, a lipid-based particle constructed with the incorporation of such an ICG lipid derivative is thought to be applicable to fluorescence imaging and photoacoustic imaging and to be useful as a diagnostic reagent and/or a contrast agent. That is, ICG lipid derivatives according to the present invention and lipid-based particles containing same can also be used for theranostics (fusion of therapy and diagnosis).

B. Preparation of ICG lipid derivative-modified liposomes and doxorubicin-encapsulating ICG lipid derivative-modified liposomes

1. Preparation of ICG lipid derivative-modified liposomes (iLip-1) and doxorubicin-encapsulating ICG lipid derivative-modified liposomes (DOX-iLip-1)

ICG lipid derivative-modified liposomes (iLip-1 (ICG-diSA), iLip-1 (ICG-diDOPE), iLip-1 (ICG-diDSPE)) and doxorubicin-encapsulating ICG lipid derivative-modified liposomes (DOX-iLip-1 (ICG-diSA), DOX-iLip-1 (ICG-diDOPE), DOX-iLip-1 (ICG-diDSPE)) were prepared according to the following procedure using an ammonium sulfate gradient and a pH gradient and using the ICG lipid derivatives synthesized in Examples A1 to A3 and using doxorubicin as a drug.

In addition, an ICG lipid derivative-modified liposome (iLip-1 (ICG-DOPE)) and a doxorubicin-encapsulating ICG lipid derivative-modified liposome (DOX-iLip-1 (ICG-DOPE)) were prepared according to the following procedure using an ammonium sulfate gradient and a pH gradient and using the ICG lipid derivative synthesized in Comparative Example A1 and using doxorubicin as a drug.

[Example B1]

(1) Preparation of iLip-1 (ICG-diSA)

The ICG-diSA synthesized in Example A1 was used as the ICG lipid derivative.

200 μl of a chloroform solution of 0.1 mol/l dipalmitoylphosphatidylcholine (DPPC, Nippon Fine Chemical Co., Ltd.), 100 μl of a chloroform solution of 0.1 mol/l cholesterol (Nippon Fine Chemical Co., Ltd), 100 μl of a chloroform solution of 0.01 mol/l MPEG2000-DSPE (Nippon Fine Chemical Co., Ltd., average molecular weight of PEG: 2,000), and 200 μl of a chloroform solution of 0.001 mol/l of the ICG lipid derivative (ICG-diSA) were combined and were mixed in the chloroform. This was followed by reduced pressure distillative removal and drying for at least one hour in a vacuum. 2 ml of a 0.3 mol/l aqueous ammonium sulfate solution (pH 5.5) was added to the lipid thin film on the walls of the recovery flask, and the temperature was raised to 50°C and stirring was carried out using a vortex mixer. The resulting crude liposome dispersion was frozen in liquid nitrogen, the frozen material was thawed at 50°C, and this was followed by light stirring with a vortex mixer. This freezing and thawing process was carried out three times, and treatment was performed for 5 minutes using a bath-type ultrasound treatment device (Branson). The liposome particle diameter was adjusted by passing the resulting liposome dispersion five times through a polycarbonate Nuclepore membrane filter having a pore diameter of 100 nm (GE Healthcare) using an extruder (Transferra Nanosciences, Inc.) heated to 50°C. The resulting liposome dispersion was then subjected to a separation process for 15 minutes using an ultracentrifugal separator (CS120GXL, Hitachi Koki Co., Ltd.) set to 453,000×g and 4°C. The supernatant was then removed and resuspension in 1 ml 0.02 mol/l HEPES buffer (pH 7.4) yielded a dispersion of ICG lipid derivative-modified liposomes (iLip-1 (ICG-diSA)).

(2) DOX-iLip-1 (ICG-diSA)

1 ml of a solution of doxorubicin (Kyowa Hakko Kirin Co., Ltd.) dissolved in 0.02 mol/l HEPES buffer (pH 7.4) to give 2 mg/ml, was added to the liposome (iLip-1 (ICG-diSA)) dispersion obtained as described above and stirring was performed for 30 minutes at 50°C to encapsulate the doxorubicin within the liposomes. The liposome dispersion was then subjected to a separation process for 15 minutes using the aforementioned ultracentrifugal separator set to 453,000 × g and 4°C. The supernatant was removed and 2 ml ultrapure water was added to yield a dispersion of doxorubicin-encapsulating ICG lipid derivative-modified liposomes (DOX-iLip-1 (ICG-diSA)).

[Example B2]

Except for the use of the ICG-diDOPE obtained in Example A2 as the ICG lipid derivative rather than ICG-diSA, the same procedure as in Example B1 was carried out to obtain a dispersion of ICG lipid derivative-modified liposomes (iLip-1 (ICG-diDOPE)) and a dispersion of doxorubicin-encapsulating ICG lipid derivative-modified liposomes (DOX-iLip-1 (ICG-diDOPE)).

[Example B3]

Except for the use of the ICG-diDSPE obtained in Example A3 as the ICG lipid derivative rather than ICG-diSA, the same procedure as in Example B1 was carried out to obtain a dispersion of ICG lipid derivative-modified liposomes (iLip-1 (ICG-diDSPE)) and a dispersion of doxorubicin-encapsulating ICG lipid derivative-modified liposomes (DOX-iLip-1 (ICG-diDSPE)).

[Comparative Example B1]

Except for the use of the ICG-DOPE obtained in Comparative Example A1 as the ICG lipid derivative rather than ICG-diSA, the same procedure as in Example B1 was carried out to obtain a dispersion of ICG lipid derivative-modified liposomes (iLip-1 (ICG-DOPE)) and a dispersion of doxorubicin-encapsulating ICG lipid derivative-modified liposomes (DOX-iLip-1 (ICG-DOPE)).

< Liposome analysis 1 >

(1) Measurement of the particle diameter distribution and zeta potential of the liposomes

Using a Zetasizer Nano ZS (produced by Malvern Instruments, Inc., a Spectris plc company; solvent: water), the particle diameter distribution (average particle diameter (Z-average) (d) and polydispersity index) and zeta potential of the liposomes were measured by the usual methods on samples of the liposomes (iLip-1) obtained in Examples B1 to B3 and Comparative Example B1 prior to doxorubicin encapsulation and on samples of the liposomes (DOX-iLip-1) after doxorubicin encapsulation. Table 1 reports the particle diameter distribution and zeta potential of the liposomes (iLip-1) prior to encapsulation of doxorubicin. Table 2 reports the particle diameter distribution and zeta potential of the various doxorubicin-encapsulating liposomes (DOX-iLip-1).

[Table 1]

Table 1. Particle diameter distribution and zeta potential of the liposomes (iLip-1) prior to encapsulation of doxorubicin

| liposome | ICG lipid derivative-modified liposome according to the present invention | | | conventional ICG lipid derivative-modified liposome |
|---|---|---|---|---|
| | iLip-1 (ICG-diSA) | iLip-1 (ICG-diDOPE) | iLip-1 (ICG-diDSPE) | iLip-1 (ICG-DOPE) |
| average particle diameter d (nm) | 126.2 | 126.4 | 135.5 | 103.4 |
| polydispersity index | 0.071 | 0.039 | 0.070 | 0.085 |
| zeta potential (mV) | -0.12 | +1.52 | -1.52 | -1.66 |

[Table 2]

Table 2. Particle diameter distribution and zeta potential of doxorubicin-encapsulating liposomes (DOX-iLip-1)

| liposome | ICG lipid derivative-modified liposome according to the present invention | | | conventional ICG lipid derivative-modified liposome |
|---|---|---|---|---|
| | DOX-iLip-1 (ICG-diSA) | DOX-iLip-1 (ICG-diDOPE) | DOX-iLip-1 (ICG-diDSPE) | DOX-iLip-1 (ICG-DOPE) |
| average particle diameter d (nm) | 129.3 | 143.0 | 140.9 | 367.1 |
| polydispersity index | 0.035 | 0.099 | 0.051 | 1.0 |
| zeta potential (mV) | -0.70 | -0.59 | -1.05 | +0.92 |

(2) Quantitation of doxorubicin encapsulated in liposomes and calculation of encapsulation ratio

The respective liposome solubilized solutions were obtained by adding 35 μl of a 10% reduced Triton X-100 (Sigma-Aldrich) solution and 280 μl ultrapure water to 35 μl of the particular post-doxorubicin-encapsulation liposome (DOX-iLip-1) dispersion obtained in Examples B1 to B3 and Comparative Example B1. In order to construct a calibration curve for doxorubicin, samples were also prepared by the addition of an aqueous doxorubicin solution (solutions prepared by dilution with ultrapure water so as to provide a final doxorubicin concentration of 0, 3.125, 6.25, 12.5, 25, 50, 100, 200, 400, and 800 μg/ml) in place of the liposome dispersion. 100 μl of each particular mixture was dispensed into a well of a 96-well microplate (Corning), and the absorbance at a wavelength of 495 nm was measured using a multiplate reader (Infinite (registered trademark) M200, Tecan). The amount of doxorubicin encapsulated in the DOX-iLip-1 was determined from

the obtained absorbance using the calibration curve method. The percentage for the amount of doxorubicin encapsulated in the DOX-iLip-1 (doxorubicin concentration, DOX concentration) with reference to the amount of doxorubicin (concentration of doxorubicin solution) added to the liposome (iLip-1) dispersion for encapsulation in the liposome, was calculated and is reported as the encapsulation ratio (DOX encapsulation ratio, %) of doxorubicin into the particular liposome. The results are given in Table 3.

[Table 3]

Table 3. Encapsulation ratio of doxorubicin in the individual DOX-iLip-1's

| liposome | ICG lipid derivative-modified liposome according to the present invention | | | conventional ICG lipid derivative-modified liposome |
|---|---|---|---|---|
| | DOX-iLip-1 (ICG-diSA) | DOX-iLip-1 (ICG-diDOPE) | DOX-iLip-1 (ICG-diDSPE) | DOX-iLip-1 (ICG-DOPE) |
| DOX concentration (μg/ml)* | 704.3 | 878.5 | 914.4 | 530.4 |
| DOX encapsulation ratio (%) | 70.4 | 87.9 | 91.4 | 53.0 |

* Gives the doxorubicin concentration in the DOX-iLip-1 dispersion when the liposome concentration (total lipid concentration for the DPPC, cholesterol, MPEG2000-DSPE, and ICG lipid derivative contained in the DOX-iLip-1 dispersion) is 10 mM.

As shown in Table 1 and Fig. 6, a uniform ICG lipid derivative-modified liposome (iLip-1), having prior to doxorubicin encapsulation an average particle diameter of approximately 100 to 140 nm and a polydispersity index not greater than 0.1, could be produced using any of the ICG lipid derivatives. The results in Table 1 show that the ICG lipid derivatives according to the present invention can be used as a constituent material of lipid-based particles.

As shown in Table 2 and Fig. 7, for the iLip-1's prepared using the respective ICG lipid derivatives according to the present invention (ICG-diSA, ICG-diDOPE, ICG-diDSPE), the particle diameters for the DOX-iLip-1's post-doxorubicin encapsulation were substantially unchanged and were 130 to 140 nm. On the other hand, for the DOX-iLip-1 prepared using the heretofore known material ICG-DOPE, a large increase in the polydispersity index and a large increase in the average particle diameter, presumably due to aggregation, were confirmed after doxorubicin encapsulation in the liposome. Based on these results, the ICG lipid derivatives according to the present invention were demonstrated to be useful as a lipid-based particle that encapsulates a drug such as doxorubicin and to be capable of forming a drug-encapsulating liposome that exhibits an excellent stability. While not intending to be limited by the following theory, the reason that a stable drug-encapsulating liposome could not be formed with the use of the heretofore known material ICG-DOPE is hypothesized to be as follows: with the heretofore known material ICG-DOPE, the sulfo group (- $SO_3H$) present in a part of the structure when a liposome is formed is exposed at the surface of the liposome membrane and encapsulation into the liposome interior is impeded by interaction between this sulfo group and the drug (doxorubicin).

With regard to the doxorubicin encapsulation ratios of the individual DOX-iLip-1's, as shown in Table 3 the doxorubicin encapsulation ratios for the liposomes DOX-iLip-1 (ICG-diSA), DOX-iLip-1 (ICG-diDOPE), and DOX-iLip-1 (ICG-diDSPE) prepared using the ICG lipid derivatives according to the present invention (ICG-diSA, ICG-diDOPE, ICG-diDSPE) were higher at 70 to 90% than the approximately 50% of the DOX-iLip-1 (ICG-DOPE) of Comparative Example B1, which was prepared using the heretofore known material ICG-DOPE.

Thus, as has been shown by the preceding, the liposomes prepared using the ICG lipid derivatives according to the present invention (ICG-diSA, ICG-diDOPE, ICG-diDSPE) were demonstrated to exhibit the following in comparison to the liposome prepared using a heretofore known ICG lipid derivative (ICG-DOPE): 1) a better and an excellent stability of the drug-encapsulating liposome formed by the encapsulation of a drug from outside the liposome into the liposome interior; 2) a higher efficiency of encapsulation of a drug into the liposome interior.

2. Preparation of ICG lipid derivative-modified liposomes (iLip-2) and doxorubicin-encapsulating ICG lipid derivative-modified liposomes (DOX-iLip-2)

ICG lipid derivative-modified liposomes (iLip-2 (ICG-diSA), iLip-2 (ICG-diDOPE), iLip-2 (ICG-diDSPE)) and doxorubicin-encapsulating ICG lipid derivative-modified liposomes (DOX-iLip-2 (ICG-diSA), DOX-iLip-2 (ICG-diDOPE), DOX-iLip-2 (ICG-diDSPE)) were prepared by a pH gradient method using citric acid, which is another representative encapsulation method, and using the ICG lipid derivatives synthesized in Examples A1 to A3 and using doxorubicin as a drug.

In addition, an ICG lipid derivative-modified liposome (iLip-2 (ICG-DOPE)) and a doxorubicin-encapsulating ICG

lipid derivative-modified liposome (DOX-iLip-2 (ICG-DOPE)) were prepared according to the following procedure using an ammonium sulfate gradient and a pH gradient and using the ICG lipid derivative synthesized in Comparative Example A1 and using doxorubicin as a drug.

[Example B4]

(1) Preparation of iLip-2 (ICG-diSA)

The ICG-diSA synthesized in Example A1 was used as the ICG lipid derivative.

360 μl of a chloroform solution of 0.1 mol/l dipalmitoylphosphatidylcholine (DPPC, Nippon Fine Chemical Co., Ltd.), 200 μl of a chloroform solution of 0.01 mol/l distearoylphosphatidylcholine (DSPC, Nippon Fine Chemical Co., Ltd), 200 μl of a chloroform solution of 0.01 mol/l MPEG2000-DSPE (Nippon Fine Chemical Co., Ltd., average molecular weight of PEG: 2,000), and 400 μl of a chloroform solution of 0.001 mol/l of the ICG lipid derivative (ICG-diSA) were combined and were mixed in the chloroform. This was followed by reduced pressure distillative removal, drying for at least one hour in a vacuum, and addition of 2 ml of 0.3 mol/l citrate buffer (pH 4.0), and the temperature was raised to 50°C and stirring was carried out using a vortex mixer to obtain a crude liposome dispersion. Using an extruder (Transferra Nanosciences, Inc.) heated to 50°C, passage was effected, five times each in sequence, through polycarbonate Nuclepore membrane filters having pore diameters of 200 nm, 100 nm, and 50 nm (GE Healthcare) to obtain a liposome (iLip-2 (ICG-diSA)) dispersion.

(2) DOX-iLip-2 (ICG-diSA)

To 2 ml of the obtained liposome dispersion were added 1.56 ml of a 0.5 mol/l aqueous sodium bicarbonate solution and 0.44 ml of 0.02 mol/l HEPES buffer (pH 7.4). This was followed by the addition of 2 ml of a solution of doxorubicin (Kyowa Hakko Kirin Co., Ltd.) dissolved in 0.02 mol/l HEPES buffer (pH 7.4) to give 2 mg/ml, and stirring was performed for 2 minutes at 50°C to encapsulate the doxorubicin within the liposomes. The liposome dispersion was then subjected to a separation process for 30 minutes using an ultracentrifugal separator (CS120GXL, Hitachi Koki Co., Ltd.) set to 511,000 × g and 4°C. The supernatant was removed and 4 ml of a 0.9% aqueous sodium chloride solution was added to yield a dispersion of doxorubicin-encapsulating ICG lipid derivative-modified liposomes (DOX-iLip-2 (ICG-diSA)).

[Example B5]

Except for the use of the ICG-diDOPE obtained in Example A2 as the ICG lipid derivative rather than ICG-diSA, the same procedure as in Example B4 was carried out to obtain a dispersion of ICG lipid derivative-modified liposomes (iLip-2 (ICG-diDOPE)) and a dispersion of doxorubicin-encapsulating ICG lipid derivative-modified liposomes (DOX-iLip-2 (ICG-diDOPE)).

[Example B6]

Except for the use of the ICG-diDSPE obtained in Example A3 as the ICG lipid derivative rather than ICG-diSA, the same procedure as in Example B4 was carried out to obtain a dispersion of ICG lipid derivative-modified liposomes (iLip-2 (ICG-diDSPE)) and a dispersion of doxorubicin-encapsulating ICG lipid derivative-modified liposomes (DOX-iLip-2 (ICG-diDSPE)).

[Comparative Example B2]

Except for the use of the ICG-DOPE obtained in Comparative Example A1 as the ICG lipid derivative rather than ICG-diSA, the same procedure as in Example B4 was carried out to obtain a dispersion of ICG lipid derivative-modified liposomes (iLip-2 (ICG-DOPE)) and a dispersion of doxorubicin-encapsulating ICG lipid derivative-modified liposomes (DOX-iLip-2 (ICG-DOPE)).

< Liposome analysis 2 >

(1) Measurement of the particle diameter distribution and zeta potential of the liposomes

Using a Zetasizer Nano ZS (produced by Malvern Instruments, Inc., a Spectris plc company), the particle diameter distribution (average particle diameter (Z-average) and polydispersity index) and zeta potential of the liposomes were measured as in the preceding (1) of < Liposome analysis 1 > on samples of the liposomes (DOX-iLip-2) after

doxorubicin encapsulation that were obtained in Examples B4 to B6 and Comparative Example B2. Table 4 reports the particle diameter distribution and zeta potential of the various doxorubicin-encapsulating liposomes (DOX-iLip-2).

[Table 4]

Table 4. Particle diameter distribution and zeta potential of doxorubicin-encapsulating liposomes (DOX-iLip-2)

| liposome | ICG lipid derivative-modified liposome according to the present invention | | | conventional ICG lipid derivative-modified liposome |
|---|---|---|---|---|
| | DOX-iLip-2 (ICG-diSA) | DOX-iLip-2 (ICG-diDOPE) | DOX-iLip-2 (ICG-diDSPE) | DOX-iLip-2 (ICG-DOPE) |
| average particle diameter d (nm) | 135.5 | 85.4 | 137.3 | 138.6 |
| polydispersity index | 0.072 | 0.17 | 0.073 | 0.155 |
| zeta potential(mV) | -1.52 | -1.49 | -1.70 | -1.52 |

(2) Quantitation of doxorubicin encapsulated in liposomes and calculation of encapsulation ratio

The doxorubicin encapsulated in the liposomes was quantitated and the doxorubicin encapsulation ratio (DOX encapsulation ratio) for each liposome was calculated using the same method as in the preceding (2) in < Liposome analysis 1 > for the post-doxorubicin-encapsulation liposome (DOX-iLip-2) dispersions obtained in Examples B4 to B6 and Comparative Example B2. The results are given in Table 5.

[Table 5]

Table 5. Encapsulation ratio of doxorubicin in the individual DOX-iLip-2's

| liposome | ICG lipid derivative-modified liposome according to the present invention | | | conventional ICG lipid derivative-modified liposome |
|---|---|---|---|---|
| | DOX-iLip-2 (ICG-diSA) | DOX-iLip-2 (ICG-diDOPE) | DOX-iLip-2 (ICG-diDSPE) | DOX-iLip-2 (ICG-DOPE) |
| DOX concentration (μg/mL)* | 882.6 | 803.0 | 842.1 | 522.5 |
| DOX encapsulation ratio (%) | 88.3 | 80.3 | 84.2 | 52.3 |
| * Gives the doxorubicin concentration in the DOX-iLip-2 dispersion when the liposome concentration (total lipid concentration for the DPPC, DSPC, MPEG2000-DSPE, and ICG lipid derivative contained in the DOX-iLip-2 dispersion) is 10 mM. | | | | |

A uniform ICG lipid derivative-modified liposome (ICG-iLip-2), having after doxorubicin encapsulation an average particle diameter of approximately 85 to 140 nm and a polydispersity index not greater than 0.16, could be produced in Examples B4 to B6 and Comparative Example B2 using any of the ICG lipid derivatives (refer to Table 4 and Fig. 8).

On the other hand, significant differences were produced between Examples B4 to C3 and Comparative Example B2 with regard to the doxorubicin encapsulation ratio of the DOX-iLip-2's that were produced. That is, the doxorubicin encapsulation ratio for the DOX-iLip-2 (ICG-DOPE) of Comparative Example B2, which was produced using the heretofore known material ICG-DOPE, was about 50%, in contrast to the higher doxorubicin encapsulation ratios of 80 to 90% exhibited by the ICG lipid derivatives according to the present invention (ICG-diSA, ICG-diDOPE, ICG-diDSPE) (refer to Table 5), and it was thus confirmed that the use of the DOX-iLip-2 (ICG-diSA), DOX-iLip-2 (ICG-diDOPE), and DOX-iLip-2 (ICG-diDSPE) liposomes, which were produced using ICG lipid derivatives according to the present invention, could provide a higher doxorubicin encapsulation than the DOX-iLip-2 (ICG-DOPE) produced using the heretofore known material ICG-DOPE. Based on these results, the ICG lipid derivatives according to the present invention (ICG-diSA, ICG-diDOPE, and ICG-diDSPE) were demonstrated to be useful as a lipid-based particle that encapsulates a drug such as doxorubicin and to be capable of forming a drug-encapsulating liposome that exhibits an excellent stability at a higher encapsulation ratio compared to the heretofore known material ICG-DOPE. While not intending to be limited by the following theory, the reason that the drug encapsulation ratio is smaller with the use of the heretofore known material ICG-DOPE is hypothesized to be as follows: with the heretofore known material ICG-DOPE, the sulfo group (- $SO_3H$) present in

a part of the structure when a liposome is formed is exposed at the surface of the liposome membrane and encapsulation into the liposome interior is impeded by interaction between this sulfo group and the drug (doxorubicin).

(3) Investigation of the temperature increase and doxorubicin release of DOX-iLip-2's due to exposure to near-infrared LED light

Observations were carried out into the temperature increase of the dispersion solvent, and the accompanying doxorubicin release behavior from the particular liposome, when the post-doxorubicin-encapsulation liposome (DOX-Lip) dispersions obtained in Examples B4 to B6 and Comparative Example B2 were exposed to an LED lamp. 500 μl of the particular DOX-iLip-2 dispersion diluted with ultrapure water to provide 0.01 mol/l for the total lipid concentration (concentration in the dispersion of DPPC, cholesterol, MPEG2000-DSPE, and the ICG lipid derivative), was introduced into the wells of a 24-well Falcon microplate (Corning) and exposure to near-infrared light from an LED light source (wavelength maximum = 810 nm, full width at half maximum = 40 nm) from beneath the plate was performed in a thermostatted chamber (Taitec Corporation) set to 37°C. During this time, the temperature of the DOX-iLip-2 dispersion exposed to the near-infrared light was simultaneously measured (data acquisition every 10 seconds) using a temperature logger (SK-L400T, Sato Keiryoki Mfg. Co., Ltd.). The DOX-iLip-2 dispersion was recovered after a near-infrared light exposure of 5, 10, and 60 minutes and a 0.9% aqueous sodium chloride solution was added with mixing. This was followed by sedimentation of the DOX-iLip-2 using an ultracentrifugal separator (CS120GXL, Hitachi Koki Co., Ltd.) set to 511,000 × g and 4°C, the supernatant was then removed, and 500 μl of a 0.9% aqueous sodium chloride solution was subsequently added. To 35 μl of the obtained liposome dispersion were added 35 μl of a 10% Triton X-100 solution and 280 μl ultrapure water with mixing to give a solubilized solution of the particular liposome. Each of the resulting mixtures was processed as in (2) of < Liposome analysis 1 > and the amount of doxorubicin remaining within the liposome was determined and the amount (%) of doxorubicin released from the liposome with elapsed time was calculated. A control experiment was performed for each formulation by introducing each liposome dispersion into microplate wells in a thermostatted chamber (Taitec Corporation) set to 37°C and also determining the amount (%) of doxorubicin released with elapsed time for samples held without exposure to the LED lamp.

Tables 6 to 9 report the timewise changes in the doxorubicin release ratio (%) from the various DOX-iLip-2's due to exposure to near-infrared light.

[Table 6]

Table 6. Timewise change in the doxorubicin release ratio (%) from the ICG lipid derivative-modified liposome DOX-iLip-2 (ICG-diSA) according to the present invention due to exposure to near-infrared light

| time | 0 min | 5 min | | 10 min | | 60 min | |
|---|---|---|---|---|---|---|---|
| exposure to near-infrared light (NIRL), yes (+), no (-) | NIRL- | NIRL- | NIRL+ | NIRL- | NIRL+ | NIRL- | NIRL+ |
| DOX release ratio (%) | 0 | 1.92 | 14.3 | 0 | 14.5 | 0 | 47.4 |

[Table 7]

Table 7. Timewise change in the doxorubicin release ratio (%) from the ICG lipid derivative-modified liposome DOX-iLip-2 (ICG-diDOPE) according to the present invention due to exposure to near-infrared light

| time | 0 min | 5 min | | 10 min | | 60 min | |
|---|---|---|---|---|---|---|---|
| exposure to near-infrared light (NIRL), yes (+), no (-) | NIRL- | NIRL- | NIRL+ | NIRL- | NIRL+ | NIRL- | NIRL+ |
| DOX release ratio (%) | 2.88 | 2.63 | 10.8 | 3.33 | 15.8 | 2.81 | 62.7 |

[Table 8]

Table 8. Timewise change in the doxorubicin release ratio (%) from the ICG lipid derivative-modified liposome DOX-iLip-2 (ICG-diDSPE) according to the present invention due to exposure to near-infrared light

| time | 0 min | 5 min | | 10 min | | 60 min | |
|---|---|---|---|---|---|---|---|
| exposure to near-infrared light (NIRL), yes (+), no (-) | NIRL- | NIRL- | NIRL+ | NIRL- | NIRL+ | NIRL- | NIRL+ |
| DOX release ratio (%) | 0.79 | 1.76 | 28.9 | 1.16 | 40.3 | 2.71 | 68.0 |

[Table 9]

| Table 9. Timewise change in the doxorubicin release ratio (%) from the heretofore known ICG lipid derivative-modified liposome DOX-iLip-2 (ICG-DOPE) due to exposure to near-infrared light | | | | | | | |
|---|---|---|---|---|---|---|---|
| time | 0 min | 5 min | | 10 min | | 60 min | |
| exposure to near-infrared light (NIRL), yes (+), no (-) | NIRL- | NIRL- | NIRL+ | NIRL- | NIRL+ | NIRL- | NIRL+ |
| DOX release ratio (%) | 13.4 | 24.4 | 96.6 | 78.2 | 98.2 | 100 | 94.8 |

The results shown in Figs. 9 to 12 for measurement of the temperature of the liposome dispersion with elapsed time confirmed that a temperature increase in the liposome dispersion accompanying exposure to near-infrared light was seen for all of the 4 liposomes (DOX-iLip-2) of Examples B4 to B6 and Comparative Example B2, and confirmed that the achieved temperature was about 50°C. This shows that the ICG lipid derivative (ICG-modified liposome), which is a liposome-modifying material, is heated by exposure to near-infrared light and that the temperature of the dispersion solvent as a whole rises as a result. This suggests that the ICG lipid derivative according to the present invention can be applied to thermal therapeutic methods based on exposure to near-infrared light. It has generally been reported that cancer cells are killed at 42°C and above. Under actual conditions in vivo, liposome particles are distributed into cancer tissue in a state that is more dilute in comparison with experimental systems in current use and the heating temperature of the solvent as a whole is predicted to be lower; however, when the amount of the ICG lipid derivative added to the liposome particle is considered, it can be expected that the liposome particle itself that has come into the vicinity of cancer cells will be heated by exposure to near-infrared light, causing the death of the nearby cancer cells.

When doxorubicin release from the liposome due to exposure to near-infrared light was measured, it could be confirmed for all 4 of the liposomes (DOX-iLip-2) in Examples B4 to B6 and Comparative Example B2 that the drug (doxorubicin) was released with elapsed time after an exposure to near-infrared light for 5 minutes, 10 minutes, and 60 minutes (refer to "yes" for exposure to near-infrared light (NIRL+) in Tables 6 to 9 and Figs. 9 to 12). This shows the following: exposure to near-infrared light causes heating of the ICG lipid derivative and heating of the liposome particle itself; the fluidity of the liposome membrane is increased as a result; and the encapsulated drug is released to the outside of the liposome particle. This suggests that a lipid-based particle constructed from the ICG lipid derivative according to the present invention can exhibit the function of a so-called temperature-sensitive liposome.

In the case of samples held at 37°C without exposure to near-infrared light, a significant difference was produced between Examples B4 to B6 and Comparative Example B2. That is, almost no doxorubicin leakage was observed for the DOX-iLip-2's that used the three ICG lipid derivatives ICG-diSA, ICG-diDOPE, and ICG-diDSPE of Examples B4 to B6 (refer to "no" for exposure to near-infrared light (NIRL-) in Tables 6 to 8 and Figs. 9 to 11). However, with the DOX-iLip-2 of Comparative Example B2 that used the heretofore known ICG lipid derivative ICG-DOPE, doxorubicin leakage beginning immediately after standing and almost no doxorubicin remained in the liposome after standing for 60 minutes (refer to "no" for exposure to near-infrared light (NIRL-) in Table 9 and Fig. 12).

3. Preparation of ICG lipid derivative-modified liposomes (ICGLip73, ICGLip82) and doxorubicin-encapsulating ICG lipid derivative-modified liposomes (ICGLip73-DOX, ICGLip82-DOX)

ICG lipid derivative-modified liposomes (ICGLip73, ICGLip82) and doxorubicin-encapsulating ICG lipid derivative-modified liposomes (ICGLip73-DOX, ICGLip82-DOX) were prepared using the ICG lipid derivative synthesized in Example A3 and doxorubicin for the drug and using an ammonium sulfate gradient and pH gradient according to the procedure described in the following.

Liposomes (Lip73, Lip82) and doxorubicin-encapsulating liposomes (Lip73-DOX, Lip82-DOX) that did not contain an ICG lipid derivative were also prepared using an ammonium sulfate gradient and a pH gradient according to the procedure described in the following.

[Example B7]

Preparation of ICGLip73-DOX

(1) ICGLip73

(i) 280 μl of a chloroform solution of 0.1 mol/l dipalmitoylphosphatidylcholine (DPPC, Nippon Fine Chemical Co., Ltd.), 600 μl of a chloroform solution of 0.01 mol/l distearoylphosphatidylcholine (DSPC, Nippon Fine Chemical Co., Ltd),

200 μl of a chloroform solution of 0.01 mol/l MPEG2000-DSPE (Nippon Fine Chemical Co., Ltd., average molecular weight of PEG: 2,000), and 200 μl of a chloroform solution of 0.01 mol/l ICG-diDSPE (Nippon Fine Chemical Co., Ltd.) were mixed in the chloroform.

(ii) This was followed by reduced pressure distillative removal of the chloroform, drying for at least one hour in a vacuum, the addition of 2 ml of a 0.3 mol/l aqueous ammonium sulfate solution (pH 5.5), raising the temperature to 55°C, and stirring using a vortex mixer. Using an extruder (Northern Lipids Inc.) heated to 55°C, the resulting crude liposome dispersion was passed, five times each in sequence from the largest pore diameter, through polycarbonate Nuclepore membrane filters having pore diameters of 400 nm, 200 nm, and 100 nm (GE Healthcare). The resulting liposome dispersion was dialyzed for at least four hours in ultrapure water using a dialysis membrane (Spectra/Por (registered trademark) 4 Dialysis Membrane, Spectrum) having a molecular weight cutoff of 12-14 kDa. This was followed by a separation process for 15 minutes using an ultracentrifugal separator (Optima™ TLX, Beckman Coulter, Inc.) set to 543,200 × g and 4°C. The supernatant was then removed and suspension in 2 ml of 0.02 mol/l HEPES buffer (pH 7.4) yielded a liposome dispersion (ICGLip73).

(2) ICGLip73-DOX

1 ml of a solution of doxorubicin (Kyowa Hakko Kirin Co., Ltd.) dissolved in 0.02 mol/l HEPES buffer (pH 7.4) to give 2 mg/ml, was added and the temperature was raised to 55°C and stirring was performed for 5 minutes (750 rpm) to encapsulate the doxorubicin within the liposomes. After ice cooling, the resulting liposome dispersion was then subjected to three 30-minute separation processes using the aforementioned ultracentrifugal separator set to 543,200 × g and 4°C. The supernatant was removed and 2 ml of 0.066 mol/l phosphate buffer (pH 7.4) was then added to yield a dispersion of doxorubicin-encapsulating ICG lipid derivative-modified liposomes (ICGLip73-DOX).

[Comparative Example B3]

Preparation of Lip73-DOX

280 μl of a chloroform solution of 0.1 mol/l dipalmitoylphosphatidylcholine (DPPC, Nippon Fine Chemical Co., Ltd.), 1000 μl of a chloroform solution of 0.01 mol/l distearoylphosphatidylcholine (DSPC, Nippon Fine Chemical Co., Ltd), and 200 μl of a chloroform solution of 0.01 mol/l MPEG2000-DSPE (Nippon Fine Chemical Co., Ltd., average molecular weight of PEG: 2,000) were mixed in the chloroform.

In the procedure after this, a liposome (Lip73) dispersion and a doxorubicin-encapsulating liposome (Lip73-DOX) were obtained proceeding as in (1)(ii) and (2) under Preparation of ICGLip73-DOX in Example B7.

[Example B8]

320 μl of a chloroform solution of 0.1 mol/l dipalmitoylphosphatidylcholine (DPPC, Nippon Fine Chemical Co., Ltd.), 200 μl of a chloroform solution of 0.01 mol/l distearoylphosphatidylcholine (DSPC, Nippon Fine Chemical Co., Ltd), 200 μl of a chloroform solution of 0.01 mol/l MPEG2000-DSPE (Nippon Fine Chemical Co., Ltd., average molecular weight of PEG: 2,000), and 200 μl of a chloroform solution of 0.01 mol/l ICG-diDSPE (Nippon Fine Chemical Co., Ltd.) were mixed in the chloroform.

In the procedure after this, an ICG lipid derivative-modified liposome (ICGLip82) dispersion and a doxorubicin-encapsulating ICG lipid derivative-modified liposome (ICGLip82-DOX) were obtained proceeding as in 1(ii) and (2) under Preparation of ICGLip73-DOX in Example B7.

[Comparative Example B4]

Preparation of Lip82-DOX

320 μl of a chloroform solution of 0.1 mol/l dipalmitoylphosphatidylcholine (DPPC, Nippon Fine Chemical Co., Ltd.), 600 μl of a chloroform solution of 0.01 mol/l distearoylphosphatidylcholine (DSPC, Nippon Fine Chemical Co., Ltd), and 200 μl of a chloroform solution of 0.01 mol/l MPEG2000-DSPE (Nippon Fine Chemical Co., Ltd., average molecular weight of PEG: 2,000) were mixed in the chloroform.

In the procedure after this, a liposome (Lip82) dispersion and a doxorubicin-encapsulating liposome (Lip82-DOX) were obtained proceeding as in 1(ii) and (2) under Preparation of ICGLip73-DOX in Example B7.

< Liposome analysis 3 >

(1) Measurement of the particle diameter distribution and zeta potential of the liposomes

Using an ELSZ-1000 zeta potential · particle diameter measurement system (Otsuka Electronics Co., Ltd.), the particle diameter (average particle diameter (Z-average) and polydispersity index) and zeta potential were measured by the usual methods on the post-doxorubicin-encapsulation liposomes obtained in Examples B7 and B8 and Comparative Examples B3 and B4. The results are reported in Table 10.

(2) Quantitation of doxorubicin encapsulated in liposomes

Liposome solubilized solutions were obtained by adding 10 μl of a 10% reduced Triton X-100 (Sigma-Aldrich) solution and 80 μl ultrapure water to 10 μl of the particular post-doxorubicin-encapsulation liposome dispersion obtained in Examples B7 and B8 and Comparative Examples B3 and B4 in a 96-well microplate (Corning) and mixing. In order to construct a calibration curve for doxorubicin, samples were also prepared by the addition of an aqueous doxorubicin solution (solutions prepared by dilution with ultrapure water so as to provide a final doxorubicin concentration of 0, 12.5, 25, 50, 100, 200, 400, and 800 μg/ml) in place of the liposome dispersion. The absorbance at a wavelength of 495 nm was measured using a multiplate reader (Synergy H1, BIOTEC Co., Ltd.). The amount of doxorubicin (doxorubicin (DOX) concentration) encapsulated in the liposome was determined from the obtained absorbance using the calibration curve. The results are given in Table 10.

(3) Quantitation of the indocyanine green (ICG) in the liposome

Liposome solubilized solutions were obtained by adding 10 μl of a 10% reduced Triton X-100 (Sigma-Aldrich) solution and 80 μl ultrapure water to 10 μl of a tenfold dilution of the particular post-doxorubicin-encapsulation liposome dispersion obtained in Examples B7 and B8 and Comparative Examples B3 and B4 in a 96-well microplate (Corning) and mixing. In order to construct a calibration curve for ICG, samples were also prepared by the addition of an aqueous ICG solution (solutions prepared by dilution with physiological saline from Diagnogreen (Daiichi Sankyo Co., Ltd.) so as to provide a final ICG concentration of 0, 5, 10, 25, 50, and 100 μg/ml) in place of the liposome dispersion. The absorbance at a wavelength of 800 nm was measured using a multiplate reader (Synergy H1, BIOTEC Co., Ltd.). The amount of ICG in the liposome was determined from the obtained absorbance using the calibration curve. The results are given in Table 10.

[Table 10]

Table 10. Formulation characteristics of the various doxorubicin-encapsulating liposomes

| liposome | particle diameter (d, nm) | polydispersity index | zeta potential (mV) | DOX concentration (mM) | ICG concentration (mM) |
|---|---|---|---|---|---|
| Lip73-DOX | 108 | 0.068 | -0.72 | 1.02 | - |
| ICGLip73-DOX | 112 | 0.073 | -0.81 | 1.34 | 0.58 |
| Lip82-DOX | 104 | 0.119 | -2.71 | 0.72 | - |
| ICGLip82-DOX | 110 | 0.087 | -4.76 | 1.17 | 0.59 |

As shown in Table 10, uniform doxorubicin-encapsulating ICG lipid derivative-modified liposomes (ICGLip73-DOX, ICGLip82-DOX) and doxorubicin-encapsulating liposomes (Lip73-DOX, Lip82-DOX), having a particle diameter of approximately 100 to 120 nm and a polydispersity index of not more than 0.12, could be obtained.

In addition, according to the results for the quantitation of doxorubicin (DOX) and ICG, these liposomes encapsulated doxorubicin at high concentrations. In particular, ICGLip73-DOX and ICGLip82-DOX, which were prepared using an ICG lipid derivative (ICG-diDSPE) according to the present invention, encapsulated doxorubicin at higher concentrations than Lip73-DOX and Lip82-DOX, which did not contain an ICG lipid derivative. It was also confirmed for ICGLip73-DOX and ICGLip82-DOX according to analysis by HPLC that they contained ICG-diDSPE.

(4) Investigation of doxorubicin release from ICGLip-DOX's due to exposure to near-infrared LED light

Observations were then carried out into the temperature increase of the dispersion solvent, and the accompanying doxorubicin release behavior from the particular liposome, when the post-doxorubicin-encapsulation liposome dispersions (ICGLip73-DOX, Lip73-DOX, ICGLip82-DOX, Lip82-DOX) obtained in Examples B7 and B8 and Comparative Examples B3 and B4 were exposed to LED light. That is, 200 μl of the particular liposome dispersion diluted with 0.066 mol/l phosphate buffer (pH 7.4) to provide 0.1 mg/ml for the ICG concentration (however, for the Lip73-DOX and Lip82-DOX liposome dispersions, the liposome dispersions were diluted so the doxorubicin concentration of each was the same and this was added), was introduced into the wells of a 96-well microplate (Corning) and exposure for 10 minutes to near-infrared light from an LED exposure device (the set contained an L800 irradiator (EL-SPI-800-01, LED: USHIO L800-66-60) and L800 irradiator power source (EL-DGI-10008-01) (Ebisudenshi Co., Ltd.); wavelength maximum = 808 nm, full width at half maximum = 32 nm, average output = 767 $mW/cm^2$) from beneath the plate was performed in a thermostatted chamber (Taitec Corporation) set to 37°C. After the exposure to near-infrared light, each liposome dispersion was recovered and 2.5 ml of 0.066 mol/l phosphate buffer (pH 7.4) was added with mixing; this was followed by sedimentation of the liposomes for 30 minutes using an ultracentrifugal separator (Optima™ TLX, Beckman Coulter, Inc.) set to 543,200 × g and 4°C, removal of the supernatant, and then the addition of 200 μl of 0.066 mol/l phosphate buffer (pH 7.4). Each of these suspensions was processed as in (2) of < Liposome analysis 3 > and the amount of doxorubicin remaining within the liposome was determined and the amount (%) of doxorubicin released from the liposome was calculated. A control experiment was performed for each formulation by introducing each liposome dispersion into microplate wells in a thermostatted chamber (Taitec Corporation) set to 37°C and also determining the amount (%) of doxorubicin released for samples held for 10 minutes without exposure to the LED light.

Fig. 6 reports the results for ICGLip73-DOX, Lip73-DOX, ICGLip82-DOX, and Lip82-DOX.

It could be confirmed from Fig. 6 that approximately 50% of the doxorubicin encapsulated in the liposome was released from the liposome due to the exposure of ICGLip73-DOX to the LED light for 10 minutes, but that doxorubicin was not released from the ICGLip73-DOX that had not been exposed to the LED light. On the other hand, it was confirmed that the release for the LED light exposure of Lip73-DOX was about the same as for the absence of exposure to the LED light (around approximately 10%).

The same could also be confirmed for ICGLip82-DOX: approximately 50% of the doxorubicin was released from the liposome due to exposure to the LED light, while doxorubicin was not released from the ICGLip82-DOX that was not exposed to the LED light. For Lip82-DOX, doxorubicin release was not confirmed for exposure to the LED light and for the absence of exposure to the LED light.

The post-doxorubicin-encapsulation liposome dispersions of Examples B7 and B8 (ICGLip73-DOX, ICGLip82-DOX) had a much better doxorubicin encapsulation stability than the DOX-iLip-2 (ICG-diDSPE) (DOX release ratio of 40.3% at the 10-minute point during exposure to near-infrared light, Fig. 11A) of Example B6, which used ICG-diDSPE.

The results in the preceding examples have shown that liposomes prepared from ICG lipid derivatives according to the present invention (ICG-diSA, ICG-diDOPE, ICG-diDSPE) store doxorubicin within the liposomes in a stable manner in the organism, have themselves a heat-generating action upon exposure to near-infrared light, and can at the same time release the encapsulated doxorubicin to the outside of the liposome.

C. Preparation of cisplatin-encapsulating ICG lipid derivative-modified liposomes

Cisplatin-encapsulating ICG lipid derivative-modified liposomes (ICGLip73-Cis, ICGLip82-Cis) were prepared using the following procedure and using the ICG lipid derivative synthesized in Example A3 and using cisplatin (Cis) as the drug.

Liposomes (Lip73, Lip82) and cisplatin-encapsulating liposomes (Lip73-DOX, Lip82-DOX) that did not contain the ICG lipid derivative were also prepared using the following procedure.

[Examples C1 and C2 and Comparative Examples C1 and C2]

Preparation of Lip73-Cis, ICGLip73-Cis, Lip82-Cis, and ICGLip82-Cis

DPPC (NOF Corporation), DSPC (Nippon Fine Chemical Co., Ltd.), MPEG2000-DSPE (Genzyme), and ICG-diDSPE were weighed into glass vials in accordance with the following table to provide the following molar ratios (DPPC : DSPC : MPEG2000-DSPE : ICG-diDSPE), respectively: 7 : 2.5 : 0.5 : 0 (Lip73-Cis), 7 : 1.5 : 0.5 : 0.5 (ICGLip73-Cis), 8 : 1.5 : 0.5 : 0 (Lip82-Cis), and 8 : 0.5 : 0.5 : 0.5 (ICGLip82-Cis).

[Table 11]

| Table 11. Lipid mixture compositions | | | | | |
|---|---|---|---|---|---|
| | | DPPC (mg) | DSPC (mg) | MPEG2000-DSPE (mg) | ICG-diDSPE (mg) |
| Comparative Example C1 | Lip73-Cis | 128.6 | 49.5 | 34.9 | 0 |
| Example C1 | ICGLip73-Cis | 120.7 | 29.6 | 35.2 | 25.9 |
| Comparative Example C2 | Lip82-Cis | 146.8 | 29.5 | 35.0 | 0 |
| Example C2 | ICGLip82-Cis | 146.7 | 9.8 | 35.0 | 26.0 |

1 mL ethanol was added to each glass vial and heating to 80°C was carried out to prepare lipid solutions. An 8.3 mg/mL aqueous cisplatin solution was prepared by weighing out 150 mg cisplatin, adding 18 mL physiological saline, and heating to 80°C. 4 mL of the aqueous cisplatin solution was added to each lipid solution to prepare aqueous liposome solutions. While heating to 80°C and using an extruder (LIPEX), each aqueous liposome solution was passed five times through a filter (Whatman) having a pore diameter of 100 nm. Cooling at 4°C for 30 minutes was performed, centrifugation was carried out for 10 minutes (approximately 1600g, Kubota 7780II), and the sedimented material and aqueous liposome solution were separated. Using a Float-A-Lyzer G2 Dialysis Device (100 kD), the supernatant was dialyzed for 12 hours with physiological saline at 4°C. After dilution with physiological saline, ultracentrifugal separation (Optima XPN-80, Beckman) was carried out using a condition of approximately 370,000g. The supernatant was removed and suspension with physiological saline was carried out. This was passed through a PES (polyethersulfone) filter (Whatman) having a pore diameter of 0.45 μm to obtain cisplatin-encapsulating liposome (ICGLip73-Cis, ICGLip82-Cis) aqueous solutions and aqueous solutions of cisplatin-encapsulating liposomes (Lip73-DOX, Lip82-DOX) that did not contain the ICG lipid derivative.

< Liposome analysis 4 >

The following analyses were run on the post-cisplatin-encapsulation liposome aqueous solutions obtained in Examples C1 and C2 and Comparative Examples C1 and C2.

(1) Measurement of the particle diameter and zeta potential of the liposomes

Using an ELSZ-1000 zeta potential · particle diameter measurement system (Otsuka Electronics Co., Ltd.), the particle diameter (average particle diameter (Z-average) and polydispersity index) and zeta potential were measured by the usual methods on each liposome. The results are reported in Table 12.

(2) Quantitation of the cisplatin encapsulated in the liposome

The lipid concentrations and cisplatin concentration were measured on each aqueous liposome solution by HPLC (Shimadzu Corporation). The results are reported in Table 12.

(3) Quantitation of indocyanine green (ICG) in the liposomes

Liposome solubilized solutions were obtained by adding 10 μl of a 10% reduced Triton X-100 (Sigma-Aldrich) solution and 80 μl ultrapure water to 10 μl of a 50-fold dilution of each resulting liposome dispersion in a 96-well microplate (Corning) and mixing. The absorbance at a wavelength of 800 nm was measured using a multiplate reader (Synergy H1, BIOTEC Co., Ltd.). The amount of ICG in the liposomes was determined from the obtained absorbance using a calibration curve. The results are given in Table 12.

[Table 12]

| Table 12. Formulation characteristics of the cisplatin-encapsulating liposomes | | | | | |
|---|---|---|---|---|---|
| liposome | particle diameter (d, nm) | polydispersity index | zeta potential (mV) | cisplatin concentration (μg/mL) | ICG concentration (mM) |
| Lip73-Cis | 92 | 0.076 | -4.18 | 800 | |
| ICGLip73-Cis | 87 | 0.101 | -0.46 | 800 | 4.68 |
| Lip82-Cis | 90 | 0.075 | +1.33 | 800 | |
| ICGLip82-Cis | 86 | 0.095 | -5.37 | 800 | 4.44 |

As shown in Table 12, uniform cisplatin-encapsulating ICG lipid derivative-modified liposomes (ICGLip73-Cis, ICGLip82-Cis) and cisplatin-encapsulating liposomes (Lip73-Cis, Lip82-Cis), having a particle diameter of not more than 100 nm and a polydispersity index of not more than 0.12, could be obtained.

In addition, according to the results of the quantitation of cisplatin and ICG, these liposomes encapsulated cisplatin at high concentrations. The incorporation of ICG-diDSPE in ICGLip73-Cis and ICGLip82-Cis could also be confirmed according to the results of the HPLC analysis.

(4) Investigation of cisplatin release from ICGLip-Cis's due to exposure to near-infrared LED light

Observations were then carried out into the temperature increase of the dispersion solvent, and the accompanying cisplatin release behavior from the particular liposome, when the liposome dispersions (ICGLip73-Cis, Lip73-Cis, ICGLip82-Cis, and Lip82-Cis) of Examples C1 and C2 and Comparative Examples C1 and C2 were exposed to LED light. 200 μl of the particular liposome dispersion diluted with 0.066 mol/l phosphate buffer (pH 7.4) to provide 0.1 mg/ml for the ICG concentration (however, for the Lip73-Cis and Lip82-Cis liposome dispersions, the liposome dispersions were diluted so the cisplatin concentration of each was the same and this was added), was introduced into the wells of a 96-well microplate (Corning) and exposure for 10 minutes to near-infrared light from an LED exposure device (the set contained an L800 irradiator (EL-SPI-800-01, LED: USHIO L800-66-60) and L800 irradiator power source (EL-DGI-10008-01) (Ebisudenshi Co., Ltd.); wavelength maximum = 808 nm, full width at half maximum = 32 nm, average output = 767 $mW/cm^2$) from beneath the plate was performed in a thermostatted chamber (Taitec Corporation) set to 37°C. During this time, the temperature of the liposome dispersion exposed to the near-infrared light was measured every 10 seconds using a temperature logger (SK-L400T, Sato Keiryoki Mfg. Co., Ltd.). After the exposure to near-infrared light, each liposome dispersion was recovered and 2.5 ml of 0.066 mol/l phosphate buffer (pH 7.4) was added with mixing; this was followed by sedimentation of the liposomes for 30 minutes using an ultracentrifugal separator (Optima™ TLX, Beckman Coulter, Inc.) set to 543,200 × g and 4°C, removal of the supernatant, and then the addition of 200 μl of 0.066 mol/l phosphate buffer (pH 7.4). Each of these suspensions was processed as in (2) of < Liposome analysis 4 > and the amount of cisplatin remaining within the liposome was determined and the amount (%) of cisplatin released from the liposome was calculated. A control experiment was performed for each formulation by introducing each liposome dispersion into microplate wells in a thermostatted chamber (Taitec Corporation) set to 37°C and also determining the amount (%) of cisplatin released for samples held for 10 minutes without exposure to the LED light.

Fig. 7A and Fig. 7B report the results for ICGLip73-Cis, Lip73-Cis, ICGLip82-Cis, and Lip82-Cis.

It could be confirmed from Fig. 7A that at least 80% of the cisplatin encapsulated in the liposome was released from the liposome due to the exposure of ICGLip73-Cis to the LED light for 10 minutes, but that cisplatin was not released from the ICGLip73-Cis that had not been exposed to the LED light. On the other hand, for Lip73-Cis, cisplatin release was not confirmed for either exposure to LED light or for the absence of exposure to LED light.

For ICGLip82-Cis, some release was seen in the absence of exposure to the LED light, but release from the liposome of at least 90% of the cisplatin due to exposure to the LED could be confirmed. Release to about the same degree was seen for exposure of Lip82-Cis to the LED light as for the absence of exposure to the LED light.

Fig. 7B shows that for the ICG lipid derivative-containing liposomes (ICGLip73-Cis, ICGLip82-Cis) the temperature of the liposome dispersion underwent a significant increase due to exposure to the LED light.

The results of these examples demonstrated that a liposome prepared from an ICG lipid derivative according to the present invention (ICG-diDSPE) held cisplatin within the liposome in a stable manner and could release the encapsulated cisplatin to the outside of the liposome upon irradiation with near-infrared light.

D. Therapeutic effects in HT1080 cancer-bearing mice

1. Preparation of liposome formulations (ICG-Lip, Lip-DOX, ICGLip-DOX)

Using the ICG lipid derivative synthesized in Example A3 and using doxorubicin (DOX) as the drug, the following were prepared using the same procedures as in the preceding examples: a drug-encapsulating ICG lipid derivative-modified liposome (ICGLip-DOX), a drug-encapsulating liposome that did not contain the ICG lipid derivative (Lip-DOX), and an ICG lipid derivative-modified liposome that did not encapsulate a drug (ICG-Lip).

(Preparative procedure)

DPPC (NOF Corporation), DSPC (Nippon Fine Chemical Co., Ltd.), and MPEG2000-DSPE (Genzyme) were dissolved with ethanol to provide 20 mg/mL, and ICG-diDSPE was dissolved with chloroform to provide 5 mg/mL. Mixing was carried out so as to give the weights in the following table for each lipid. The molar ratios for the lipids (DPPC : DSPC : MPEG2000-DSPE : ICG-diDSPE) are, respectively, 70 : 15 : 5 : 5 (Rp. 1) and 70 : 25 : 5 : 0 (Rp. 2).

[Table 13]

| Table 13. Lipid mixture compositions of liposomes prior to encapsulation | | | | |
|---|---|---|---|---|
| | DPPC (mg) | DSPC (mg) | MPEG2000-DSPE (mg) | ICG-diDSPE (mg) |
| Rp.1 | 540.8 | 124.8 | 147.4 | 108.3 |
| Rp.2 | 513.8 | 197.6 | 140.0 | 0 |

The organic solvent was distillatively removed under reduced pressure and drying was performed in a vacuum for 12 hours to prepare a lipid mixture. An aqueous ammonium sulfate solution was prepared by dissolving ammonium sulfate (FUJIFILM Wako Pure Chemical Corporation) to provide 300 mM. Sucrose (FUJIFILM Wako Pure Chemical Corporation) and L-histidine (FUJIFILM Wako Pure Chemical Corporation) were dissolved in water to provide, respectively, 96 mg/mL and 10 mM to prepare a 96 mg/mL sucrose/10 mM/mL histidine aqueous solution. 10 mL of the aqueous ammonium sulfate solution heated to 80°C was added to each of the lipid mixtures (Rp. 1 and Rp. 2), and aqueous liposome solutions were prepared by inversion mixing. While heating to 80°C and using an extruder (LIPEX), the aqueous liposome solutions were each passed five times through a filter (Whatman) having a pore diameter of 50 nm. The resulting particle size-adjusted material was diluted with physiological saline, followed by ultracentrifugal separation (Optima XPN-80, Beckman) using a condition of approximately 370,000g. The supernatant was removed and suspension with physiological saline was carried out, followed by ultracentrifugal separation using a condition of approximately 370,000g. The supernatant was removed and suspension was performed using the aqueous 96 mg/mL sucrose/10 mM/mL histidine solution. The respective lipid concentrations were measured by HPLC (Shimadzu Corporation).

An aqueous doxorubicin solution was obtained by dissolving doxorubicin (Dox) (Sicor Inc.) with a 5% glucose solution (Otsuka Pharmaceutical Co., Ltd.) to provide 10 mg/mL. Doxorubicin encapsulation was performed by mixing the aqueous doxorubicin solution with each of the aqueous liposome solutions (Rp. 1 and Rp. 2) and heating for 5 minutes at 50°C; after cooling, this was passed through a PES (polyethersulfone) filter (Whatman) having a pore diameter of 0.45 μm to prepare doxorubicin-encapsulating liposome aqueous solutions (ICG-Lip-Dox and LipDOX).

The respective lipid concentrations were measured by HPLC (Shimadzu Corporation). The particle diameter was measured using a Zetasizer Nano ZS (Malvern Instruments, Inc.). The encapsulation ratio was measured by diluting each of the obtained doxorubicin-encapsulating liposome aqueous solutions 10-fold with physiological saline, carrying out ultracentrifugal separation (himac CS120FX, Hitachi) using a condition of approximately 600,000g, and measuring the doxorubicin concentration in the supernatant and the doxorubicin concentration prior to the ultracentrifugal separation using HPLC. The result was an encapsulation ratio of 98.0% and 98.6%, respectively.

In addition, ICG-Lip, which did not contain doxorubicin, was prepared by diluting the Rp. 1 aqueous liposome solution, prior to doxorubicin encapsulation, with the aqueous 96 mg/mL sucrose/10 mM/mL histidine solution.

The doxorubicin (DOX) concentration and the respective lipid (ICG, DPPC, DSPC) concentrations were as given in the following table.

[Table 14]

| Table 14. Formulation characteristics of the liposomes | | | | | | |
|---|---|---|---|---|---|---|
| | particle diameter (d, nm) | polydispersity index | DOX concentration (mg/mL) | ICG concentration (mg/mL) | DPPC concentration (mg/mL) | DSPC concentration (mg/mL) |
| ICG-Lip | 82.3 | 0.05 | - | 3.08 | 37.44 | 8.58 |
| Lip-DOX | 80.5 | 0.04 | 1.50 | - | 36.48 | 12.89 |
| ICGLip-DOX | 81.7 | 0.06 | 1.50 | 3.08 | 32.16 | 8.44 |

2. Experimental treatment of HT1080 solid tumor-bearing mice

(1) Preparation of HT1080 solid tumor-bearing mice

A cell suspension of HT1080 cells, a human fibrosarcoma cell line, was prepared by suspension with D-MEM (high glucose) culture medium (FUJIFILM Wako Pure Chemical Corporation) to give $5 \times 10^7$ cells/ml. 0.1 ml of the HT1080 cell suspension was then filled into a 1 mL syringe (Terumo Corporation) fitted with a 27G injection needle (Terumo Corporation), and subcutaneous administration (Day 0) was performed into the right back of 6-week-old BALB/c nude male mice (acquired from Japan SLC, Inc.) to provide $5 \times 10^6$ cells/mouse. Breeding by the usual methods was then carried out until a solid tumor of suitable size had formed.

(2) Administration of drug and exposure to LED light

On the 11th day (Day 11) after cancer cell transplantation, the mice were held in a mouse holder and ICGLip, Lip-DOX, or ICGLip-DOX was administered via the tail vein at a doxorubicin dose of 10 mg/kg (body weight). Physiological saline (Saline) was administered to the nontreatment group, and in the ICGLip administration group administration was performed so as to provide the same ICG-diDSPE dose as in the ICGLip-DOX administration group. 24 hours after administration, the mice were anesthetized by inhalation using isoflurane (Pfizer) using a small anesthesia machine for small animals (MK-A110, Muromachi Kikai Co., Ltd.) and were placed on a heating pad (Heater Mat KN-475-35, Natsume Seisakusho Co., Ltd.) set at 35°C. The fiber tip of the LED exposure device (the set contained an L800 irradiator (EL-SPI-800-01, LED: USHIO L800-66-60) and L800 irradiator power source (EL-DGI-10008-01) (Ebisudenshi Co., Ltd.); wavelength maximum = 808 nm, full width at half maximum = 32 nm, average output = 767 mW/cm$^2$) was fixed as close as possible to the solid tumor, and LED light was irradiated for 10 minutes under isoflurane inhalation anesthesia. A non-LED-exposure group was also prepared as a control.

(3) Temperature measurement at the solid tumor site

Prior to exposure to the LED light and immediately after the end of exposure, the temperature of the solid tumor site was measured using a thermography camera (E5, Flir Systems). The results of the temperature measurements are given in Fig. 8.

(4) Evaluation of therapeutic effect and measurement of body weight

The short diameter and long diameter of the solid tumor formed subcutaneously in the mice was measured using calipers. The volume of the solid tumor was calculated using the following formula.

$$\text{cancer volume (cm}^3\text{)} = 0.4 \times \text{(short diameter (cm))} \times \text{(short diameter (cm))} \times \text{(long diameter (cm))}$$

Fig. 9 provides a graph that shows, using the calculated tumor volume, the proportion (relative tumor volume (RTV)) of the change from the day of drug administration.

The results of the body weight measurements for the mice are shown in Fig. 10.

As shown in Fig. 8, an increase in solid tumor temperature to about 50°C due to exposure of the solid tumor to LED light was confirmed for HT1080 solid tumor-bearing mice that had received ICGLip and ICGLip-DOX, which were liposomes that contained the ICG lipid derivative (ICG-diDSPE). On the other hand, an increase in the solid tumor

temperature due to exposure to LED light was not confirmed in the mice that received physiological saline (Saline) or Lip-DOX.

As shown in Fig. 9, with HT1080 solid tumor-bearing mice that received ICGLip-DOX and then subsequently received irradiation of the solid tumor with LED light (ICGLip-DOX + LED), a very large tumor regression effect was confirmed and mice in which the cancer had disappeared were also seen. Mice receiving ICGLip and LED light irradiation (ICGLip + LED) presented a transitory tumor shrinkage, but this was followed by tumor enlargement. In comparison to Saline-administered (non-LED light-exposure) mice (Saline), mice receiving Saline followed by exposure to LED light (Saline + LED), and ICGLip-administered (non-LED light-exposure) mice (ICGLip), the ICGLip-DOX-administered (non-LED light-exposure) mice (ICGLip-DOX), Lip-DOX-administered (non-LED light-exposure) mice (Lip-DOX), and mice receiving Lip-DOX followed by exposure to LED light (Lip-DOX + LED) presented a greater cancer growth-inhibiting effect which, however, did not reach to cancer shrinkage.

As shown in Fig. 10, an influence due to drug administration or exposure to LED light was not seen in the changes in mouse body weight, and a trend of recovery from the body weight reduction due to the carried cancer was seen in the mice that received ICGLip-DOX followed by exposure of the solid tumor to LED light (ICGLip-DOX + LED).

E. Therapeutic effect in BxPC3 cancer-bearing mice

1. Preparation of liposome formulations (ICG-Lip, Lip-Cis, ICGLip-Cis)

Using the ICG lipid derivative synthesized in Example A3 and using cisplatin (Cis) as the drug, the following were prepared using the same procedures as in the preceding examples: a drug-encapsulating ICG lipid derivative-modified liposome (ICGLip-Cis), a drug-encapsulating liposome that did not contain the ICG lipid derivative (Lip-Cis), and an ICG lipid derivative-modified liposome that did not encapsulate a drug (ICG-Lip).

(Preparative procedure)

DPPC (NOF Corporation), DSPC (Nippon Fine Chemical Co., Ltd.), MPEG2000-DSPE (Genzyme), and ICG-diDSPE were weighed into glass vials in accordance with the table below. The molar ratios for the lipids (DPPC : DSPC : MPEG2000-DSPE : ICG-diDSPE) are, respectively, 70 : 15 : 5 : 5 (ICG-Lip), 70 : 25 : 5 : 0 (Lip-Cis), and 70 : 15 : 5 : 5 (ICG-Lip-Cis).

[Table 15]

Table 15. Lipid mixture compositions of liposomes prior to encapsulation

| | DPPC (mg) | DSPC (mg) | MPEG2000-DSPE (mg) | ICG-diDSPE (mg) |
|---|---|---|---|---|
| ICG-Lip | 385.4 | 89.0 | 105.0 | 77.4 |
| Lip-Cis | 366.2 | 140.9 | 99.9 | 0 |

3 mL ethanol was added to each glass vial and heating to 80°C was carried out to prepare the lipid solutions. An 8.7 mg/mL aqueous cisplatin solution was prepared by weighing out 304 mg cisplatin, adding 35 mL physiological saline, and heating to 80°C. 12 mL of the aqueous cisplatin solution was added to each lipid solution to prepare aqueous liposome solutions. While heating to 80°C and using an extruder (LIPEX), each aqueous liposome solution was passed five times through a filter (Whatman) having a pore diameter of 100 nm. Cooling at 4°C for 30 minutes was performed, centrifugation was carried out for 10 minutes (approximately 1600g, Kubota 7780II), and the sedimented material and aqueous liposome solution were separated. Using a Float-A-Lyzer G2 Dialysis Device (100 kD), the supernatant was dialyzed for 12 hours with physiological saline at 4°C. After dilution with physiological saline, ultracentrifugal separation (Optima XPN-80, Beckman) was carried out using a condition of approximately 370,000g. The supernatant was removed and suspension with physiological saline was carried out. This was passed through a PES (polyethersulfone) filter (Whatman) having a pore diameter of 0.45 μm to obtain cisplatin-encapsulating liposome (ICGLip-Cis, Lip-Cis) aqueous solutions and an aqueous solution of liposome (ICGLip) that did not contain cisplatin.

The concentration of the particular lipid and the cisplatin (Cis) concentration were measured by HPLC (Shimadzu Corporation). The particle diameter was measured using a Zetasizer Nano ZS (Malvern Instruments, Inc.). The results are reported in the following table.

[Table 16]

| Table 16. Formulation characteristics of the liposomes | | | | |
|---|---|---|---|---|
| | particle diameter (d, nm) | polydispersity index | Cis concentration (mg/mL) | ICG-diDSPE concentration (mg/mL) |
| ICG-Lip | 83.9 | 0.05 | - | 9.22 |
| Lip-Cis | 79.7 | 0.02 | 0.94 | - |
| ICGLip-Cis | 81.8 | 0.03 | 0.94 | 9.22 |

2. Experimental treatment of BxPC3 solid tumor-bearing mice

(1) Preparation of BxPC3 solid tumor-bearing mice

A cell suspension of BxPC3 cells, a human pancreatic cancer cell line, was prepared by suspension with RPMI1640 culture medium (FUJIFILM Wako Pure Chemical Corporation) to give 2.5 $\times$ $10^7$ cells/ml. 0.2 ml of the BxPC3 cell suspension was then filled into a 1 ml syringe (Terumo Corporation) fitted with a 27G injection needle (Terumo Corporation), and subcutaneous administration (Day 0) was performed into the right back of 5-week-old BALB/c nude male mice (acquired from Japan SLC, Inc.) to provide 5 $\times$ $10^6$ cells/mouse. Breeding by the usual methods was then carried out until a solid tumor of suitable size had formed.

(2) Administration of drug and exposure to LED light

On the 18th day (Day 18) after cancer cell transplantation, the mice were held in a mouse holder and ICGLip, Lip-Cis, or ICGLip-Cis was administered via the tail vein at a cisplatin dose of 10 mg/kg (body weight). Physiological saline (Saline) was administered to the nontreatment group, and in the ICGLip administration group administration was performed so as to provide the same ICG-diDSPE dose as in the ICGLip-Cis administration group. 24 hours after administration, the mice were anesthetized by inhalation using isoflurane (Pfizer) using a small anesthesia machine for small animals (MK-A110, Muromachi Kikai Co., Ltd.) and were placed on a heating pad (Heater Mat KN-475-35, Natsume Seisakusho Co., Ltd.) set at 35°C. The fiber tip of the LED exposure device (the set contained an L800 irradiator (EL-SPI-800-01, LED: USHIO L800-66-60) and L800 irradiator power source (EL-DGI-10008-01) (Ebisudenshi Co., Ltd.); wavelength maximum = 808 nm, full width at half maximum = 32 nm, average output = 767 mW/$cm^2$) was fixed as close as possible to the solid tumor, and LED light was irradiated for 10 minutes under isoflurane inhalation anesthesia. A non-LED-exposure group was also prepared as a control.

(3) Temperature measurement at the solid tumor site

Prior to exposure to the LED light and immediately after the end of exposure, the temperature of the solid tumor site was measured using a thermography camera (E5, Flir Systems). The results of the temperature measurements are given in Fig. 11.

(4) Evaluation of therapeutic effect and measurement of body weight

The short diameter and long diameter of the solid tumor formed subcutaneously in the mice was measured using calipers. The volume of the solid tumor was calculated using the following formula.

$$\text{cancer volume } (cm^3) = 0.4 \times (\text{short diameter (cm)}) \times (\text{short diameter (cm)}) \times (\text{long diameter (cm)})$$

Fig. 12 provides a graph that shows, using the calculated tumor volume, the proportion (relative tumor volume (RTV)) of the change from the day of drug administration.
The results of the body weight measurements for the mice are shown in Fig. 13.
As shown in Fig. 11, an increase in solid tumor temperature to about 43°C due to exposure of the solid tumor to LED light was confirmed for BxPC3 solid tumor-bearing mice that had received ICGLip and ICGLip-Cis, which were liposomes that contained ICG-diDSPE. On the other hand, an increase in the solid tumor temperature due to exposure to LED light was not confirmed in the mice that received physiological saline (Saline) or Lip-Cis.
As shown in Fig. 12, a tumor regression effect was confirmed for BxPC3 solid tumor-bearing mice that received

ICGLip-Cis and then subsequently received irradiation of the solid tumor with LED light (ICGLip-Cis + LED). Mice receiving ICGLip and LED light irradiation (ICGLip + LED) presented a transitory tumor regression, but this followed by tumor enlargement. Some growth-inhibiting effect was seen in the ICGLip-Cis-administered (non-LED light-exposure) mice (ICGLip-Cis), Lip-Cis-administered (non-LED light-exposure) mice (Lip-Cis), and mice receiving Lip-Cis followed by exposure to LED light (Lip-Cis + LED), but this effect was small.

As shown in Fig. 13, an influence due to drug administration or exposure to LED light was not seen in the changes in mouse body weight.

[Industrial Applicability]

The ICG lipid derivative according to the present invention can be advantageously applied in the fields of fluorescence imaging, photodynamic hyperthermal therapy and/or photodynamic therapy, or DDS.

## Claims

**1.** A compound represented by formula (A) below or a pharmaceutically acceptable salt thereof:

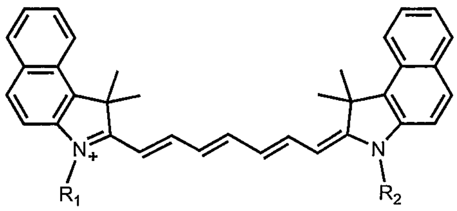

(A)

wherein

$R_1$ and $R_2$ each independently represent - $(CH_2)_k$ - CONH - $R_3$;
$R_3$ represents a group selected from the group consisting of - $(CH_2)_m$ - $OPO_3^-$ - $CH_2$ - $CH(CH_2OCOR_4)(OCOR_5)$, branched-chain $C_{14}$ to $C_{40}$ alkyl, and branched-chain $C_{14}$ to $C_{40}$ alkenyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4; and
m represents an integer from 2 to 4.

**2.** The compound according to claim 1, wherein

$R_1$ and $R_2$ each independently represent - $(CH_2)_2$ - CONH - $R_3$;
$R_3$ represents - $(CH_2)_m$ - $OPO_3^-$ - $CH_2$ - $CH(CH_2OCOR_4)(OCOR_5)$;
$R_4$ and $R_5$ each independently represent straight-chain $C_{13}$ to $C_{21}$ alkyl or straight-chain $C_{13}$ to $C_{21}$ alkenyl; and
m represents an integer from 2 to 4.

**3.** One compound selected from compounds represented by formula (I) or formula (II) below, or a pharmaceutically acceptable salt thereof:

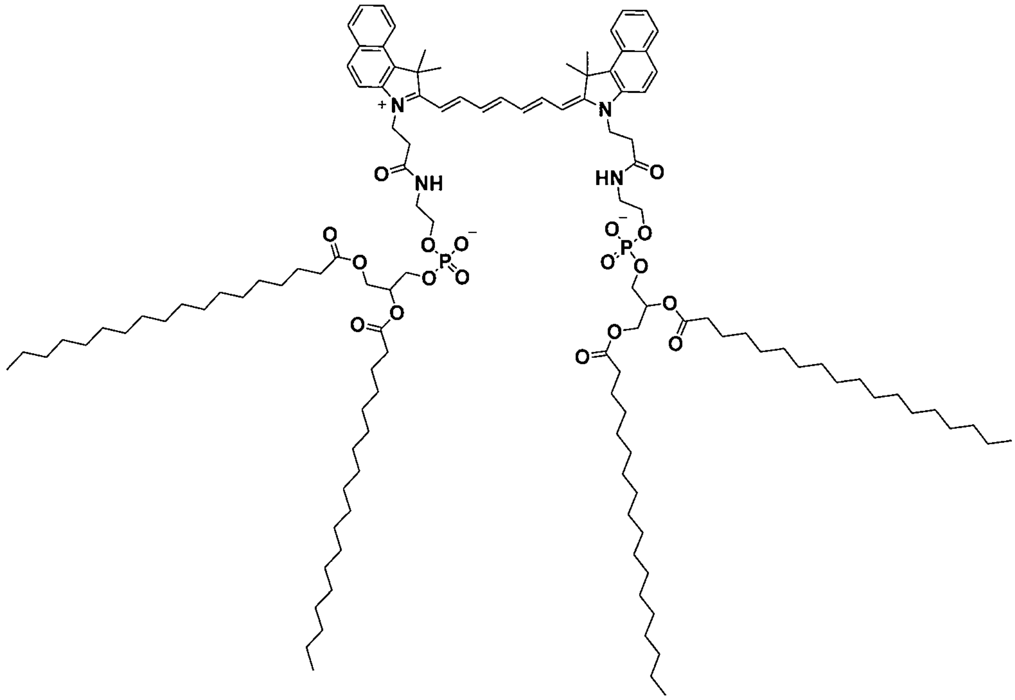

(I)

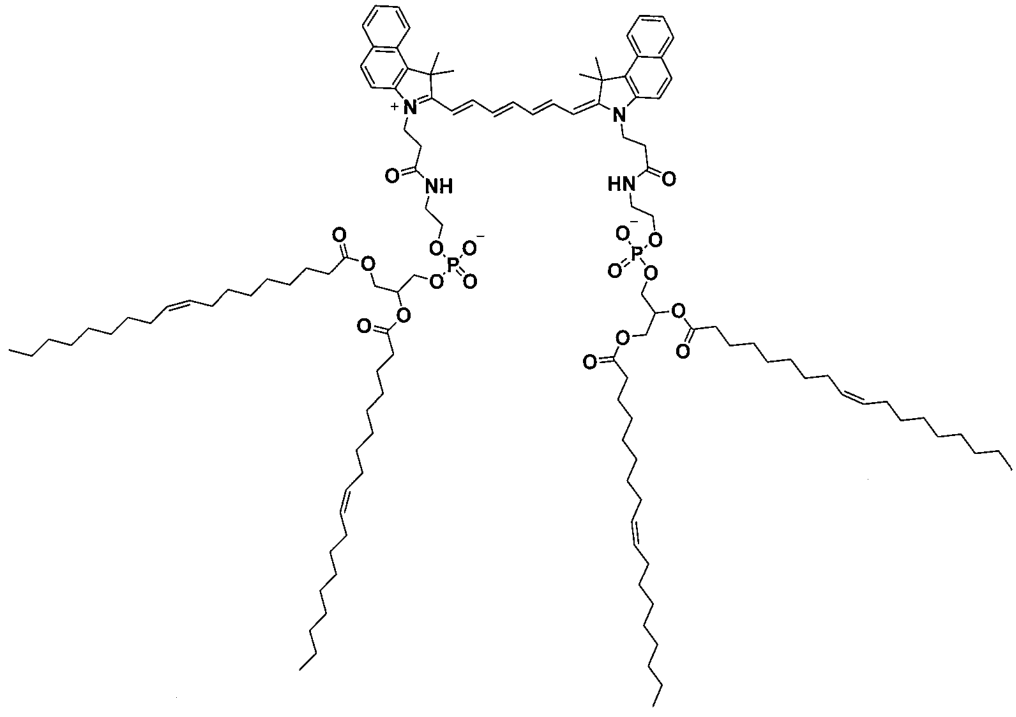

(II)

4. A lipid-based particle comprising a compound represented by formula (A) below or a pharmaceutically acceptable salt thereof:

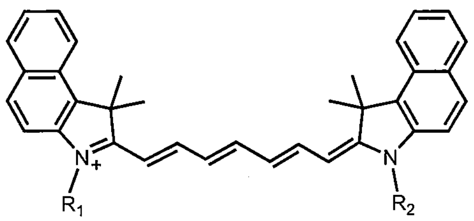

(A)

wherein

$R_1$ and $R_2$ each independently represent - $(CH_2)_k$ - CONH - $R_3$;
$R_3$ represents a group selected from the group consisting of - $(CH_2)_m$ - $OPO_3^-$ - $CH_2$ - $CH(CH_2OCOR_4)(OCOR_5)$, straight-chain $C_{14}$ to $C_{22}$ alkyl, straight-chain $C_{14}$ to $C_{22}$ alkenyl, branched-chain $C_{14}$ to $C_{40}$ alkyl, and branched-chain $C_{14}$ to $C_{40}$ alkenyl;
$R_4$ and $R_5$ each independently represent straight-chain or branched-chain $C_{13}$ to $C_{21}$ alkyl or straight-chain or branched-chain $C_{13}$ to $C_{21}$ alkenyl;
k represents an integer from 2 to 4; and
m represents an integer from 2 to 4.

**5.** The lipid-based particle according to claim 4, comprising a compound selected from the group consisting of compounds represented by formulas (I) to (III) below, or a pharmaceutically acceptable salt thereof:

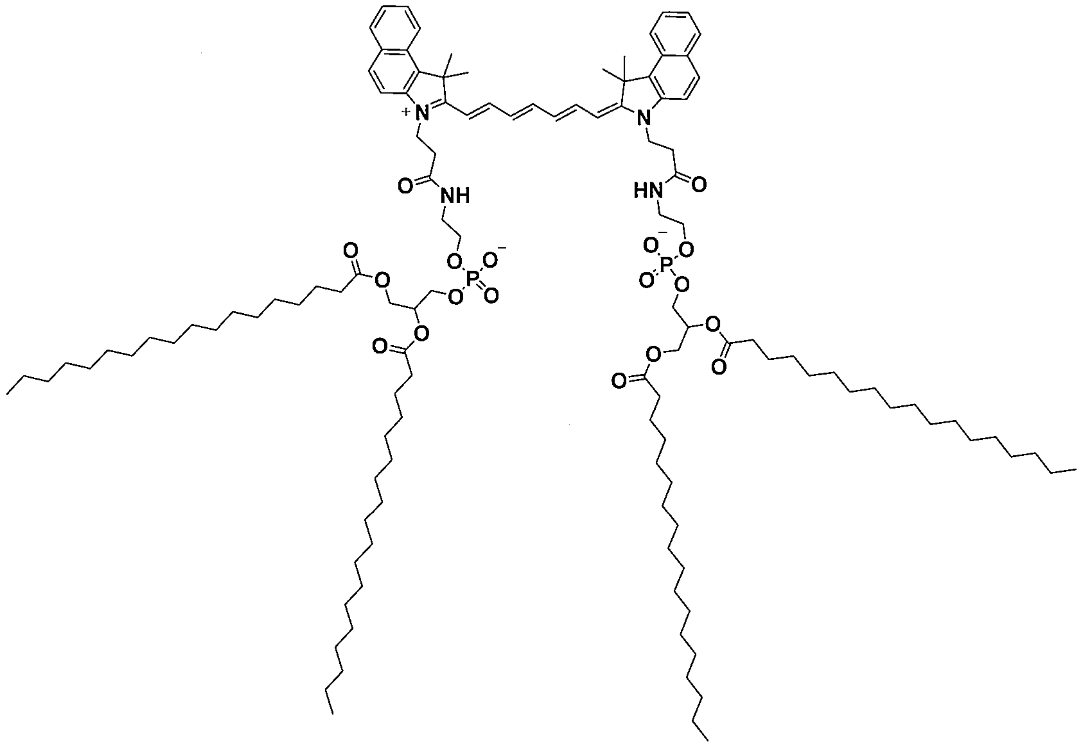

(I)

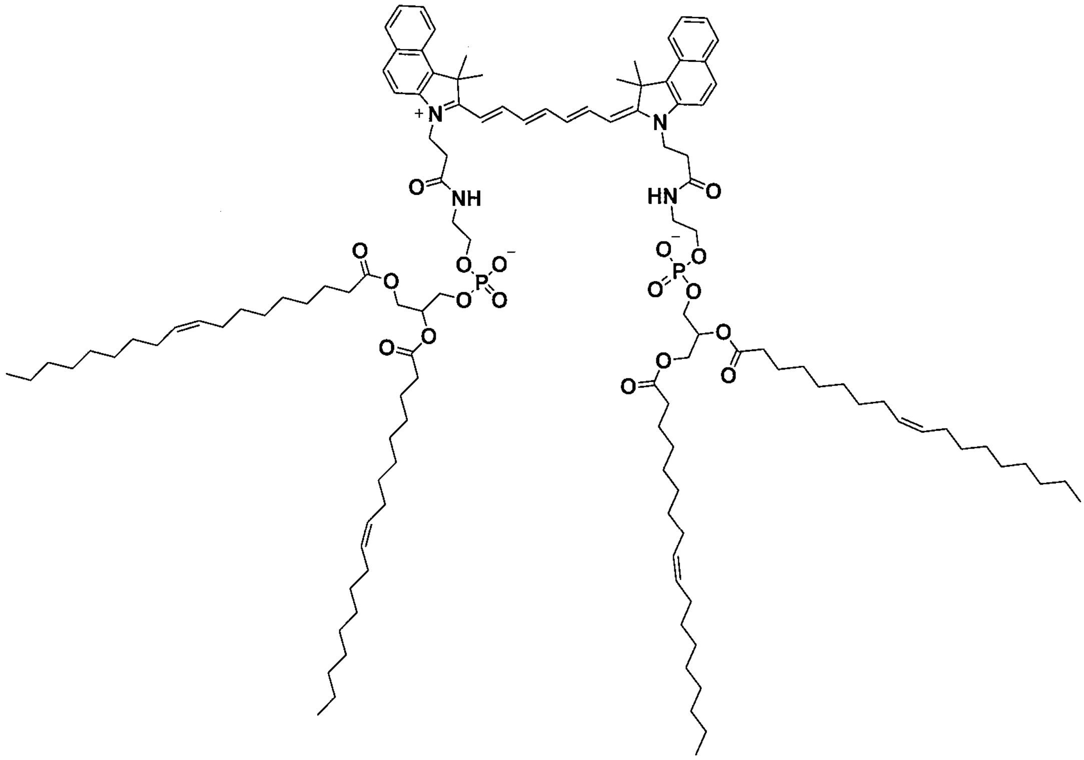

(II)

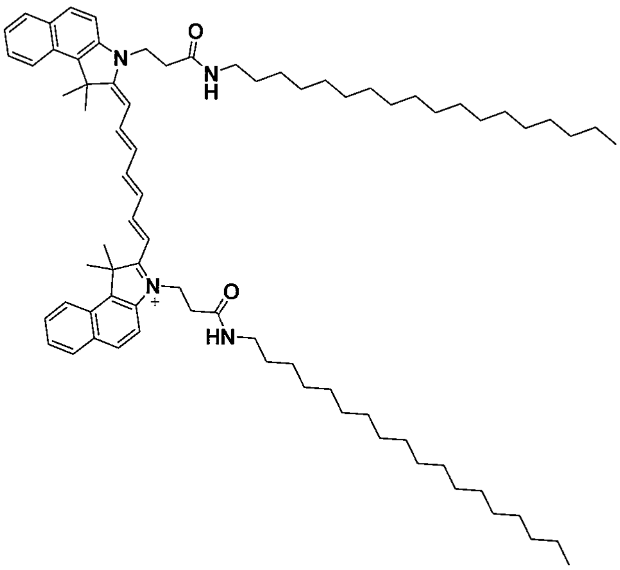

(III)

.

6. The lipid-based particle according to claim 4 or 5, which additionally fixes or supports at least one drug.

7. The lipid-based particle according to any one of claims 4 to 6, wherein the drug comprises an anticancer agent.

8. The lipid-based particle according to any one of claims 4 to 7, wherein the lipid-based particle further comprises at least one lipid selected from the group consisting of neutral lipids, polyethylene glycol-modified lipids, and sterols.

9. The lipid-based particle according to any one of claims 4 to 8, having an average particle diameter of 30 to 200 nm.

10. The lipid-based particle according to any one of claims 4 to 9, wherein the lipid-based particle is a polymer micelle or a liposome.

11. A pharmaceutical composition or a diagnostic composition, comprising the compound according to any one of claims

1 to 3 or a pharmaceutically acceptable salt thereof, or the lipid-based particle according to any one of claims 4 to 10.

**12.** The pharmaceutical composition or diagnostic composition according to claim 11, used for at least one selection from photodynamic hyperthermal therapy, photodynamic therapy, and fluorescence imaging.

[Fig. 1]

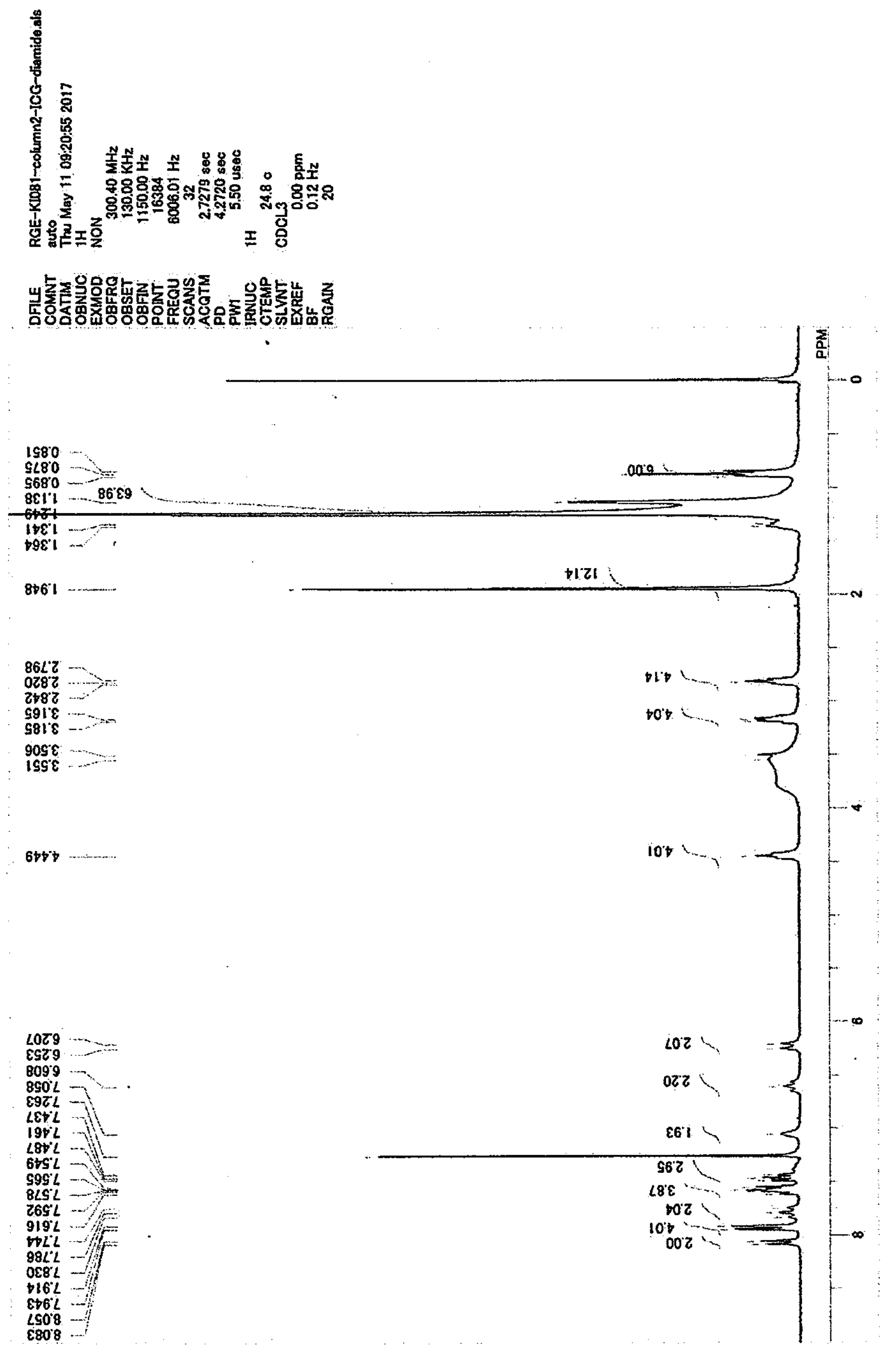

[Fig. 2]

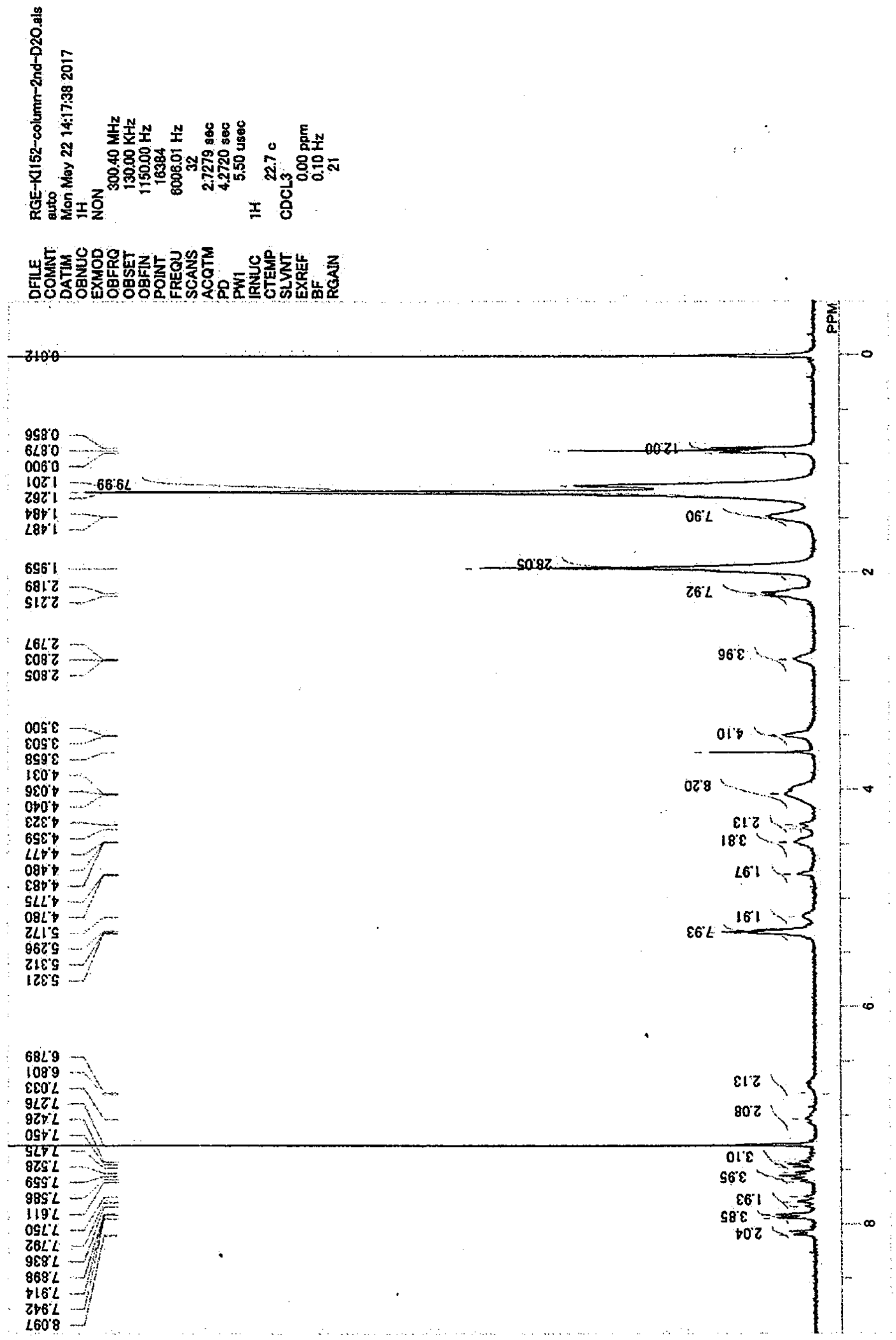
DFILE RGE-KI152-column-2nd-D2O.als
COMNT auto
DATIM Mon May 22 14:17:38 2017
OBNUC 1H
EXMOD NON
OBFRQ 300.40 MHz
OBSET 130.00 KHz
OBFIN 1150.00 Hz
POINT 16384
FREQU 6006.01 Hz
SCANS 32
ACQTM 2.7279 sec
PD 4.2720 sec
PW1 5.50 usec
IRNUC 1H
CTEMP 22.7 c
SLVNT CDCL3
EXREF 0.00 ppm
BF 0.10 Hz
RGAIN 21
PPM

[Fig. 3]

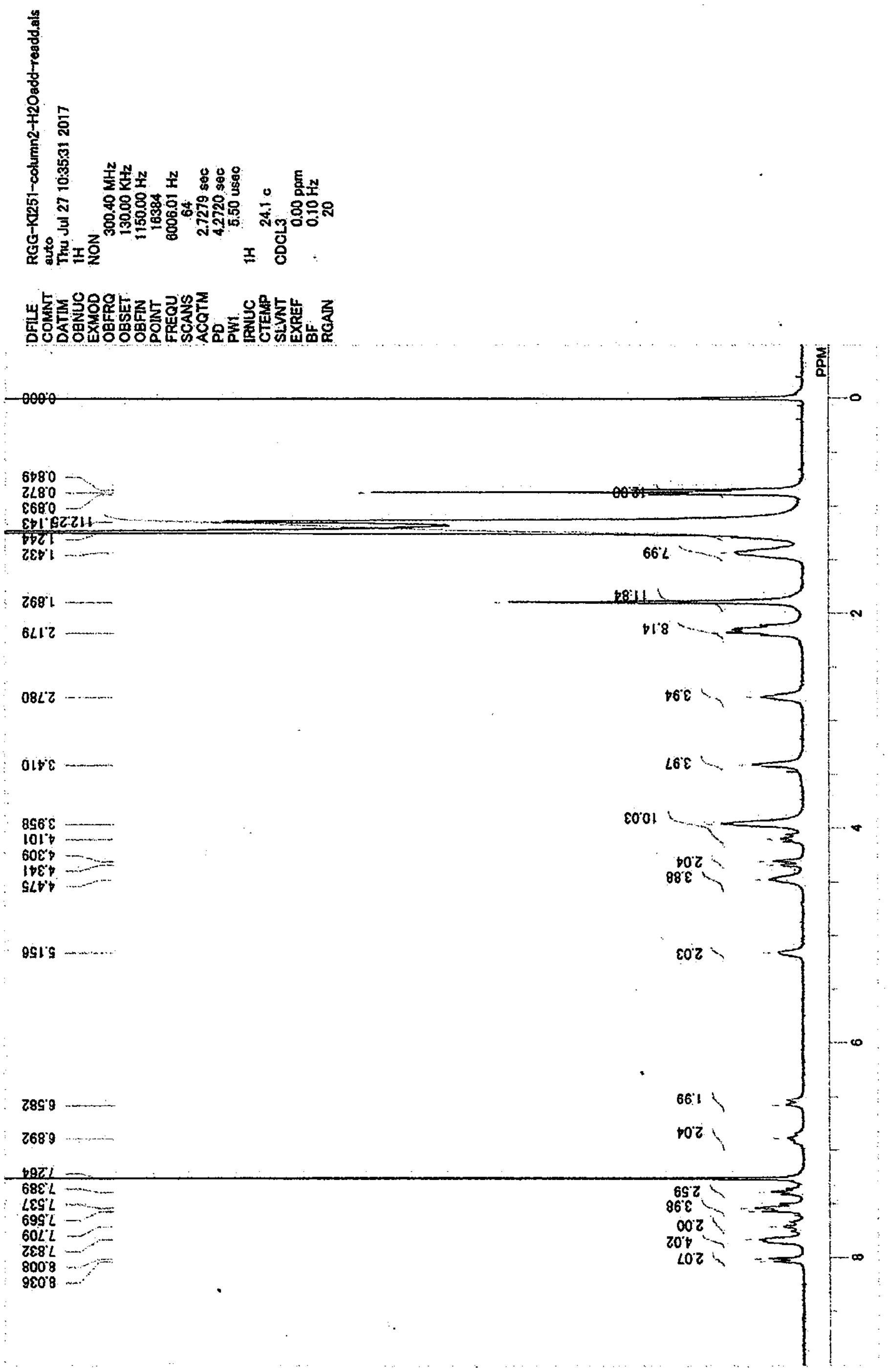
DFILE RGG-KI251-column2-H2Oadd-readd.als
COMNT auto
DATIM Thu Jul 27 10:35:31 2017
OBNUC 1H
EXMOD NON
OBFRQ 300.40 MHz
OBSET 130.00 KHz
OBFIN 1150.00 Hz
POINT 16384
FREQU 6006.01 Hz
SCANS 64
ACQTM 2.7279 sec
PD 4.2720 sec
PW1 5.50 usec
IRNUC 1H
CTEMP 24.1 c
SLVNT CDCL3
EXREF 0.00 ppm
BF 0.10 Hz
RGAIN 20
PPM

[Fig. 4]

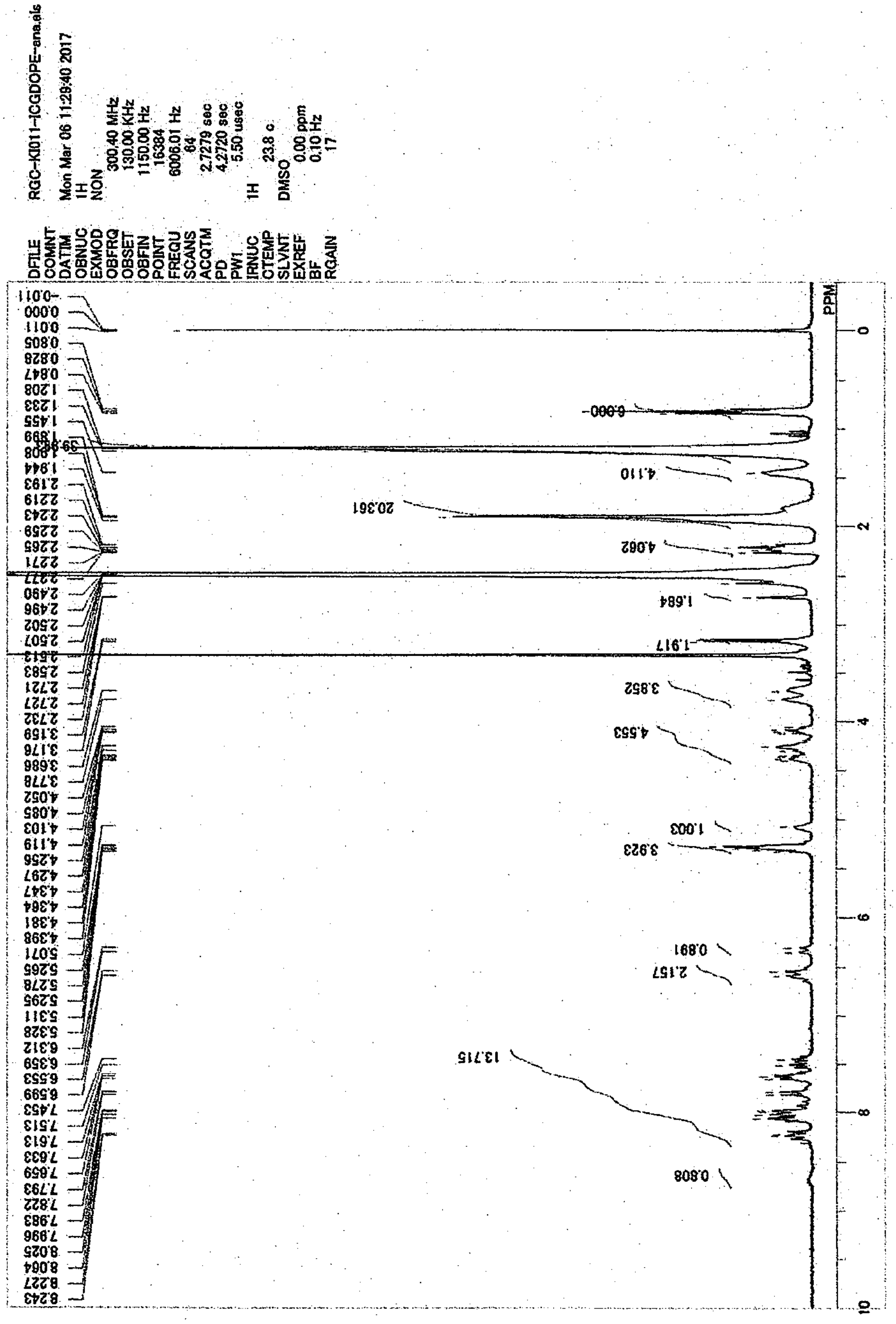

[Fig. 5]

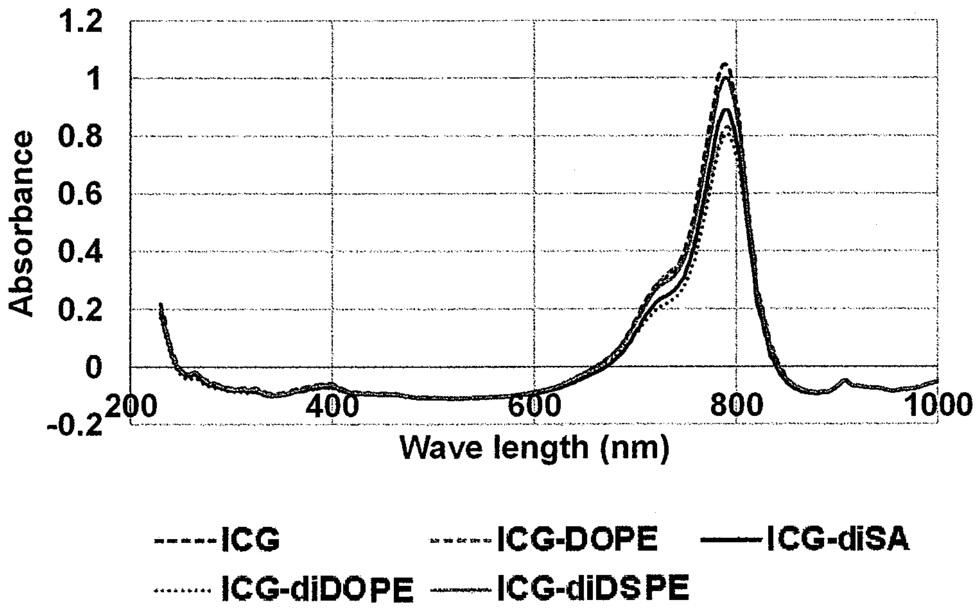
1.2
1
0.8
0.6
0.4
0.2
0
-0.2
Absorbance
200
400
600
800
1000
Wave length (nm)
ICG
ICG-DOPE
ICG-diSA
ICG-diDOPE
ICG-diDSPE

[Fig. 6]

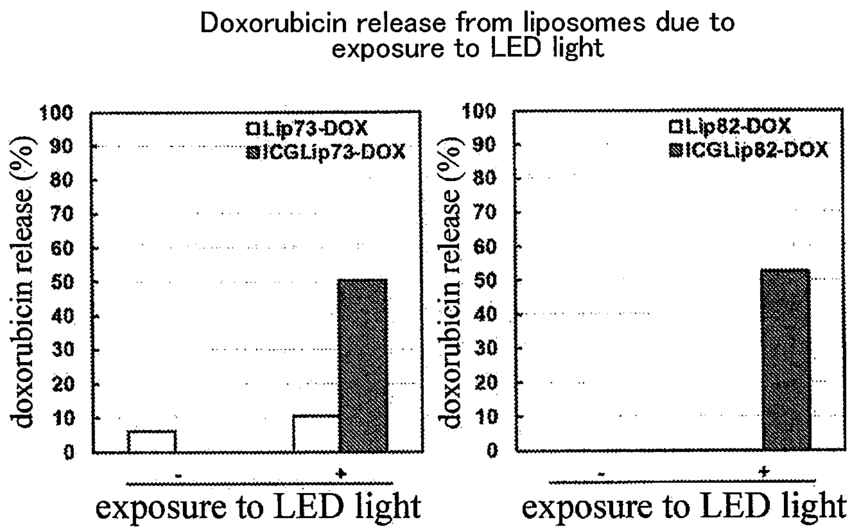
Doxorubicin release from liposomes due to exposure to LED light
doxorubicin release (%)
100
90
80
70
60
50
40
30
20
10
0
Lip73-DOX
ICGLip73-DOX
Lip82-DOX
ICGLip82-DOX
-
+
exposure to LED light
exposure to LED light

[Fig. 7A]

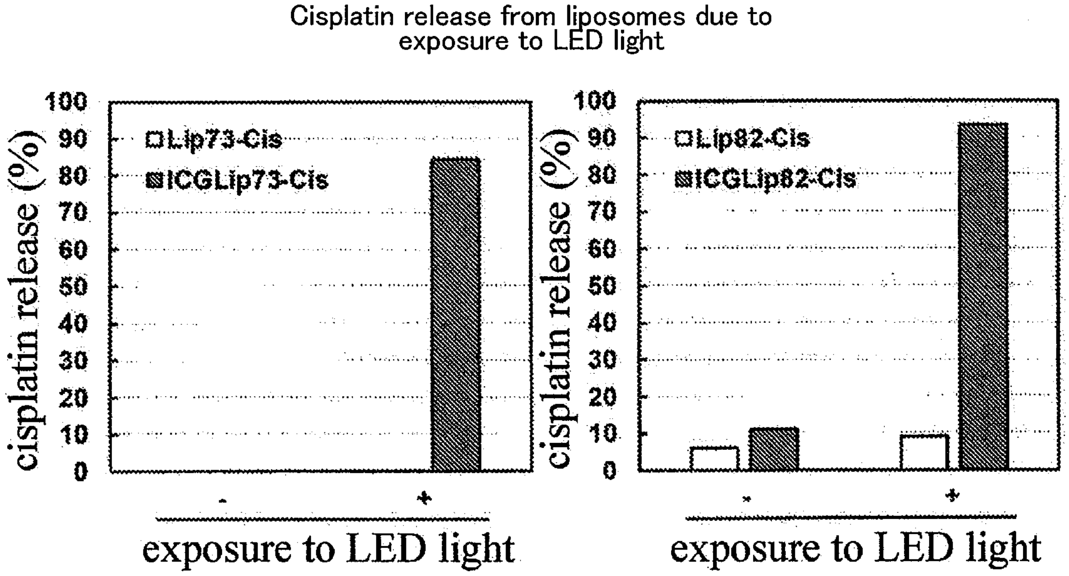
Cisplatin release from liposomes due to exposure to LED light
cisplatin release (%)
Lip73-Cis
ICGLip73-Cis
Lip82-Cis
ICGLip82-Cis
exposure to LED light
-
+

[Fig. 7B]

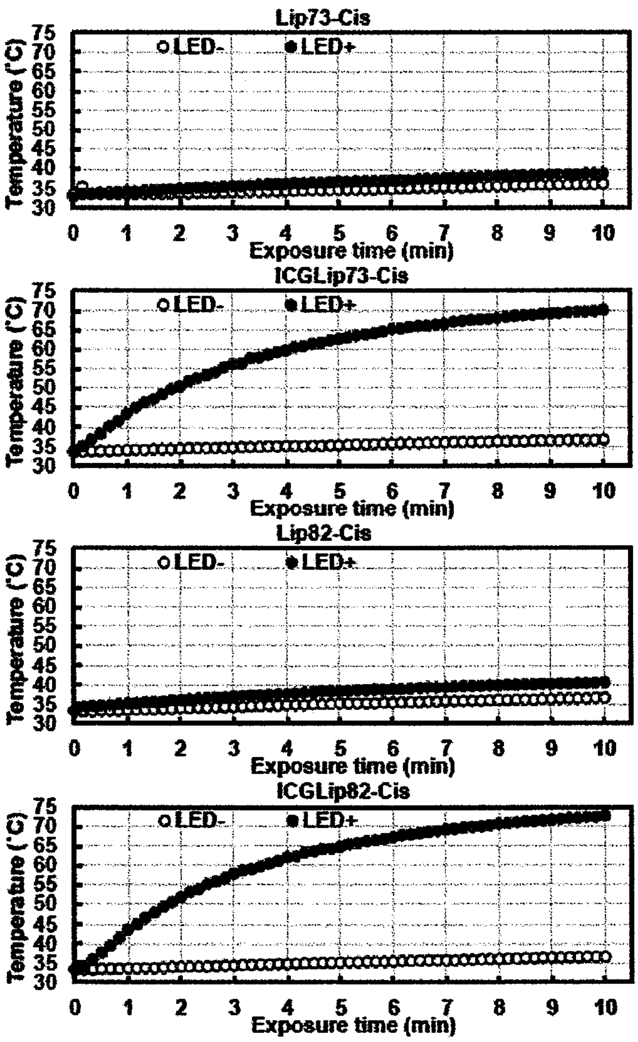
Lip73-Cis
ICGLip73-Cis
Lip82-Cis
ICGLip82-Cis
LED-
LED+
Temperature (°C)
Exposure time (min)

[Fig. 8]

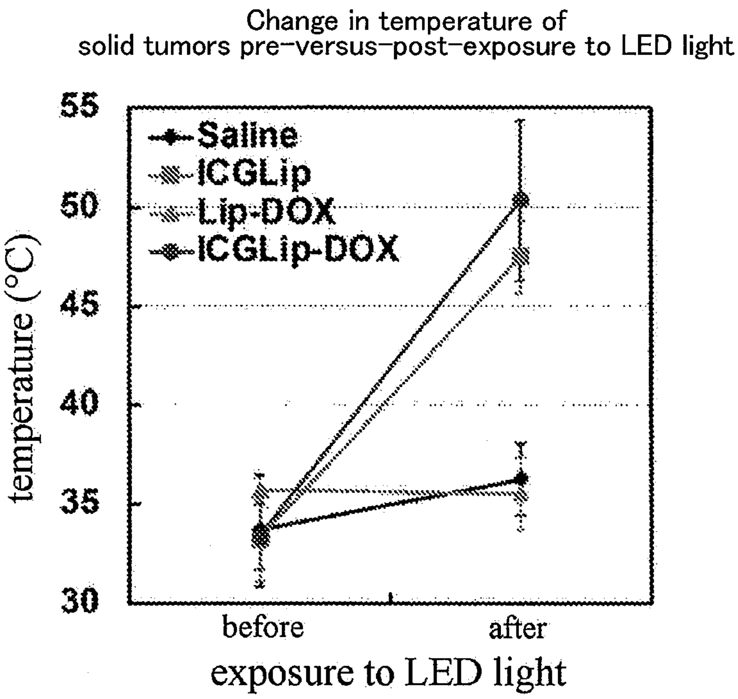
Change in temperature of
solid tumors pre-versus-post-exposure to LED light
Saline
ICGLip
Lip-DOX
ICGLip-DOX
temperature (°C)
55
50
45
40
35
30
before
after
exposure to LED light

[Fig. 9]

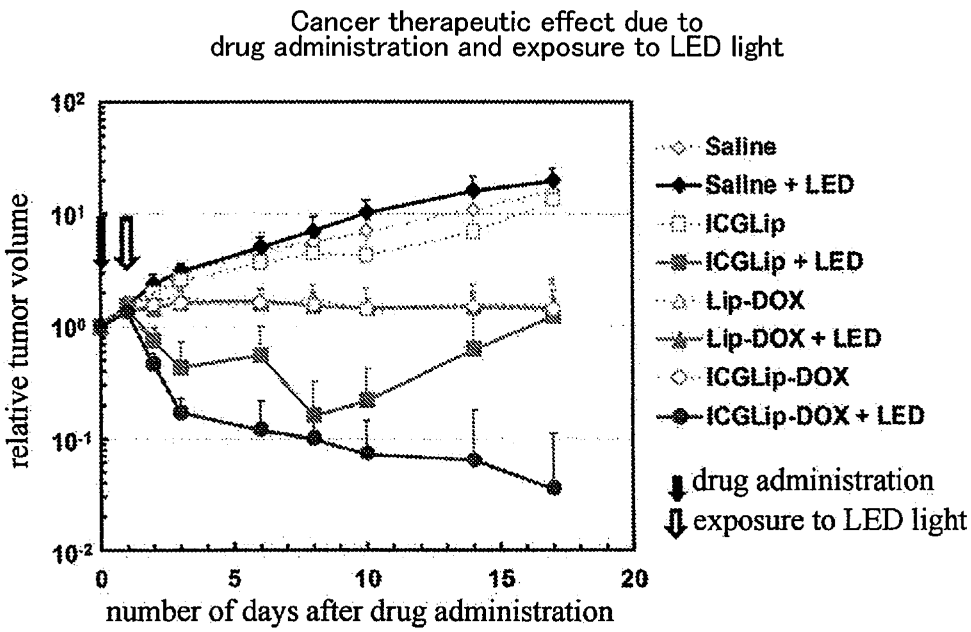
Cancer therapeutic effect due to
drug administration and exposure to LED light
relative tumor volume
$10^2$
$10^1$
$10^0$
$10^{-1}$
$10^{-2}$
0
5
10
15
20
number of days after drug administration
Saline
Saline + LED
ICGLip
ICGLip + LED
Lip-DOX
Lip-DOX + LED
ICGLip-DOX
ICGLip-DOX + LED
drug administration
exposure to LED light

[Fig. 10]

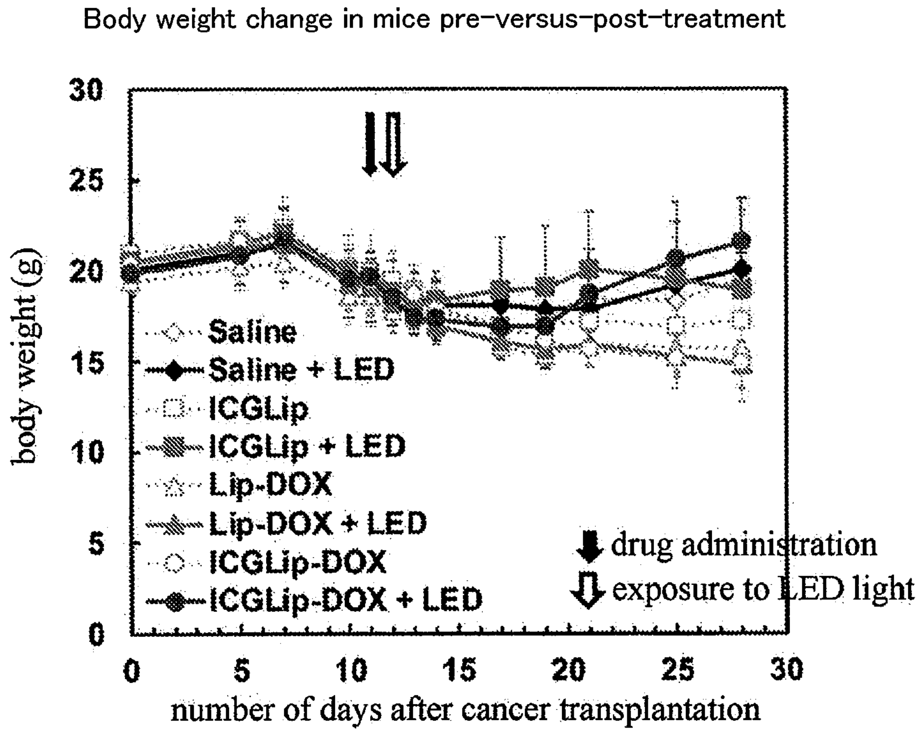
Body weight change in mice pre-versus-post-treatment
body weight (g)
30
25
20
15
10
5
0
Saline
Saline + LED
ICGLip
ICGLip + LED
Lip-DOX
Lip-DOX + LED
ICGLip-DOX
ICGLip-DOX + LED
drug administration
exposure to LED light
0
5
10
15
20
25
30
number of days after cancer transplantation

[Fig. 11]

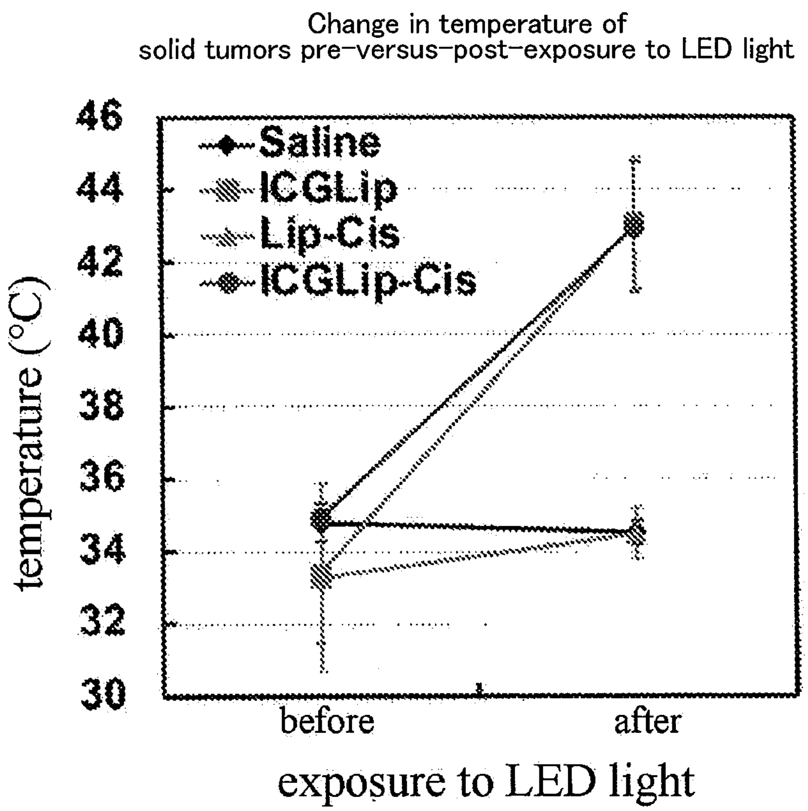
Change in temperature of
solid tumors pre-versus-post-exposure to LED light
temperature (°C)
46
44
42
40
38
36
34
32
30
Saline
ICGLip
Lip-Cis
ICGLip-Cis
before
after
exposure to LED light

[Fig. 12]

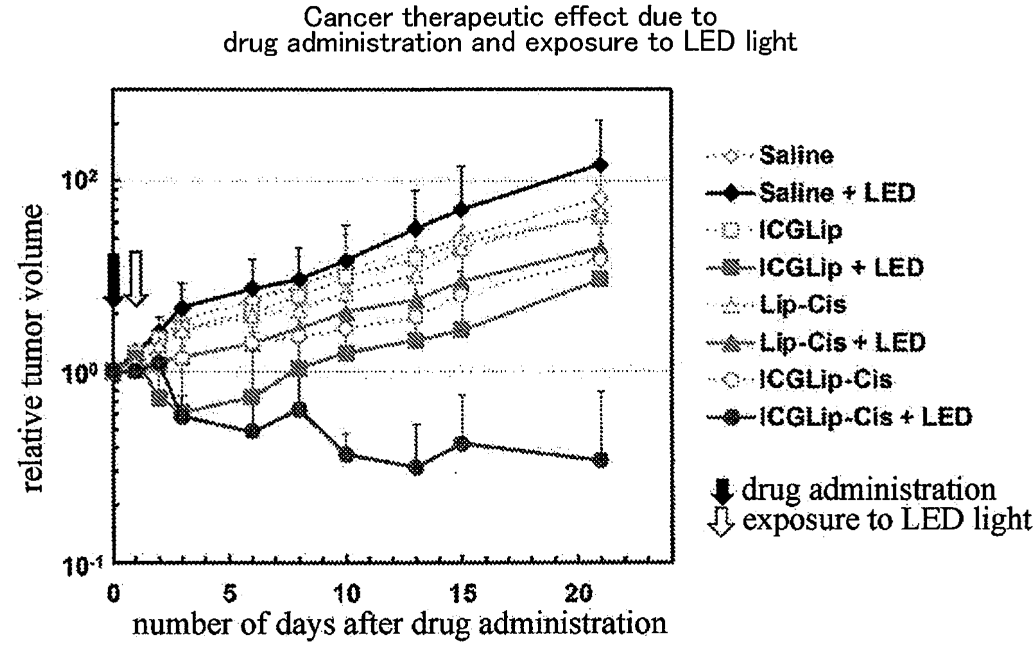
Cancer therapeutic effect due to
drug administration and exposure to LED light
relative tumor volume
10²
10⁰
10⁻¹
0
5
10
15
20
number of days after drug administration
Saline
Saline + LED
ICGLip
ICGLip + LED
Lip-Cis
Lip-Cis + LED
ICGLip-Cis
ICGLip-Cis + LED
drug administration
exposure to LED light

[Fig. 13]

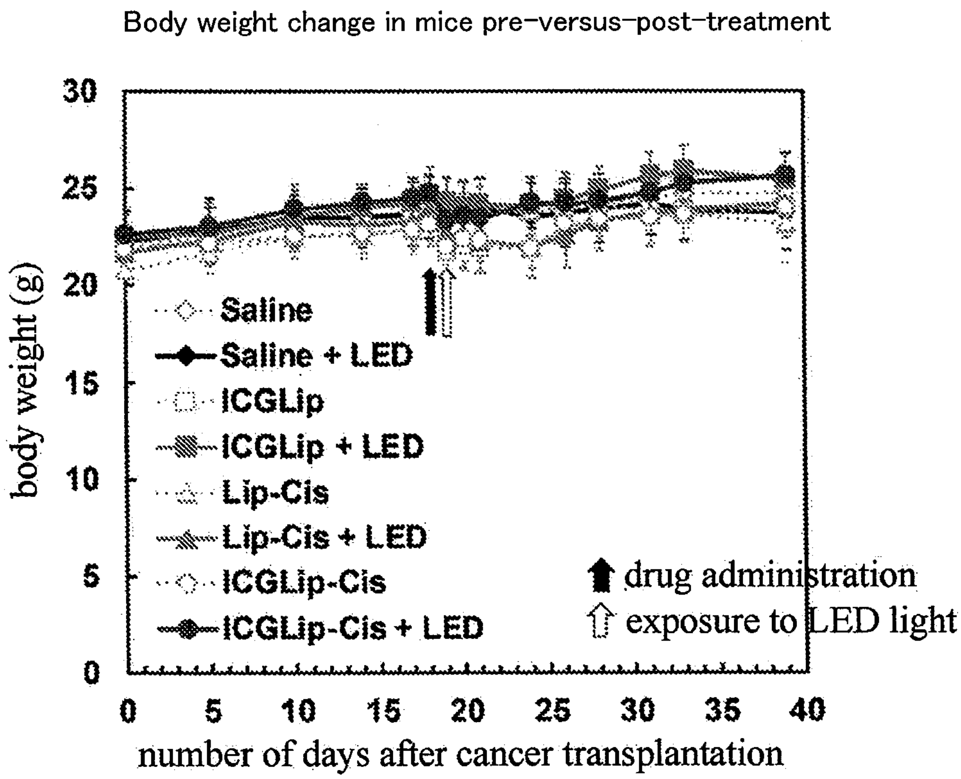
Body weight change in mice pre-versus-post-treatment
body weight (g)
30
25
20
15
10
5
0
0
5
10
15
20
25
30
35
40
number of days after cancer transplantation
Saline
Saline + LED
ICGLip
ICGLip + LED
Lip-Cis
Lip-Cis + LED
ICGLip-Cis
ICGLip-Cis + LED
drug administration
exposure to LED light

## INTERNATIONAL SEARCH REPORT

International application No.
**PCT/JP2023/016320**

**A. CLASSIFICATION OF SUBJECT MATTER**

***C07F 9/572***(2006.01)i; ***A61K 9/127***(2006.01)i; ***A61K 31/403***(2006.01)i; ***A61K 41/00***(2020.01)i; ***A61K 45/00***(2006.01)i; ***A61K 47/14***(2017.01)i; ***A61K 47/28***(2006.01)i; ***A61K 47/44***(2017.01)i; ***A61K 50/00***(2006.01)i; ***A61P 35/00***(2006.01)i; ***A61P 35/04***(2006.01)i; ***A61P 43/00***(2006.01)i; ***C07D 209/60***(2006.01)i; ***C09B 23/08***(2006.01)i
FI: C07F9/572 A; A61P43/00 121; A61K9/127; A61P35/00; A61P35/04; A61K45/00; A61K47/44; A61K47/28; A61K47/14; A61K50/00 200; A61K31/403; A61K41/00; C09B23/08 CSP; C07D209/60

According to International Patent Classification (IPC) or to both national classification and IPC

**B. FIELDS SEARCHED**

Minimum documentation searched (classification system followed by classification symbols)

C07F9/00; A61K9/00; A61K31/00; A61K41/00; A61K45/00; A61K47/00; A61K50/00;C07D209/00; C09B23/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | AKERS, Walter J. et al. Noninvasive Photoacoustic and Fluorescence Sentinel Lymph Node Identification using Dye-Loaded Perfluorocarbon Nanoparticles, ACS Nano, 2011, 5(1), pp. 173-182<br>abstract, fig. 1, table 1 | 1, 4-12 |
| A | | 2-3 |
| X | AKERS, Walter J. et al. Targeting of αvβ3-integrins expressed on tumor tissue and neovasculature using fluorescent small molecules and nanoparticles, Nanomedicine (London, United Kingdom), 2010, 5(5), pp. 715-726<br>fig. 1 | 1, 4-12 |
| A | | 2-3 |
| A | JP 2016-108335 A (CANON INC.) 20 June 2016 (2016-06-20)<br>claims 1-14 | 1-12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 July 2023** | **25 July 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/016320**

**C. DOCUMENTS CONSIDERED TO BE RELEVANT**

| Category* | Citation of document, with indication, where appropriate, of the relevant passages | Relevant to claim No. |
|---|---|---|
| A | JP 2003-261464 A (FUJI PHOTO FILM CO., LTD.) 16 September 2003 (2003-09-16) claims 1-11 | 1-12 |

Form PCT/ISA/210 (second sheet) (January 2015)

**INTERNATIONAL SEARCH REPORT**
**Information on patent family members**

International application No.
**PCT/JP2023/016320**

| Patent document cited in search report | Publication date (day/month/year) | Patent family member(s) | Publication date (day/month/year) |
|---|---|---|---|
| JP 2016-108335 A | 20 June 2016 | US 2017/0335182 A1<br>claims 1-14<br>WO 2016/092799 A1 | |
| JP 2003-261464 A | 16 September 2003 | US 2005/0226815 A1<br>claims 1-11<br>WO 2003/074091 A2<br>EP 1480683 A1<br>CN 1638810 A<br>KR 10-2004-0099301 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001299676 A
- WO 2011152046 A
- JP 2010266295 A
- JP 2010069001 A
- WO 200041726 A
- WO 2013051732 A


### Non-patent literature cited in the description


- **AKERS WJ et al.** *ACS Nano*, 25 January 2011, vol. 5 (1), 173-182
- **S. M. BERGE et al.** *J. Pharmaceutical Sciences*, 1977, vol. 66, 1-19
- *J. Am. Chem. Soc.*, 2003, vol. 125, 7766